# EUROPEAN PATENT SPECIFICATION

(11) **EP 2 154 248 B1**
(45) Date of publication and mention of the grant of the patent: **26.10.2011**
(21) Application number: 09174756.8
(22) Date of filing: 30.08.1996
(51) Int. Cl.: C12Q 1/68

(54) **Compounds and methods for diagnosis of tuberculosis**
Verbindungen und Verfahren zur Diagnose von Tuberkulose
Composés et procédés pour le diagnostic de la tuberculose

(30) Priority: 01.09.1995 US 523435; 22.09.1995 US 532136; 22.03.1996 US 620280; 05.06.1996 US 658800; 12.07.1996 US 680573
(43) Date of publication of application: 17.02.2010
(62) Divisional of application: 06270020.8
(73) Proprietor: CORIXA CORPORATION, Wilmington, DE 19808 (US)
(72) Inventor: Reed, Steven, Bellevue, WA 98005 (US); Skeiky, Yasir, Silver Spring, MD 20904 (US); Dillon, Davin, Issaquah, WA 98027 (US); Campos-Neto, Antonio, Boston, MA 02115 (US); Houghton, Raymond, Bothell, WA 98021 (US); Vedvick, Thomas, Federal Way, WA 98003 (US); Twardzik, Daniel, Bainbridge Island, WA 98110 (US)
(74) Representative: Goodall, Scott

(56) References cited:
- WO-A-95/01441
- DATABASE EMBL [Online] 4 November 1995 (1995-11-04), "Mycobacterium bovis deletion region 1, 6kDa early secretory antigenic target (esat6) gene, complete cds." XP002560304 Database accession no. U34848 -& MAHAIRAS G.G. ET AL.: "Molecular analysis of genetic differences between Mycobacterium bovis BCG and virulent M. bovis" THE JOURNAL OF BACTERIOLOGY, vol. 178, no. 5, March 1996 (1996-03), pages 1274-1282, XP000647583
- SORENSEN A.L. ET AL.: "Purification and characterisation of a low molecular mass T-cell antigen secreted by Mycobacterium tuberculosis" INFECTION AND IMMUNITY, AMERICAN SOCIETY FOR MICROBIOLOGY, WASHINGTON, US, vol. 63, no. 5, 1 May 1995 (1995-05-01), pages 1710-1717, XP002176837 ISSN: 0019-9567
- ANDERSEN A.B. ET AL.: "Structure and mapping of antigenic domains of protein antigen B, a 38,000-molecular-weight protein of Mycobacterium tuberculosis" INFECTION AND IMMUNITY, AMERICAN SOCIETY FOR MICROBIOLOGY, WASHINGTON, US, vol. 57, no. 8, 1 August 1989 (1989-08-01), pages 2481-2488, XP002026677 ISSN: 0019-9567

## Description

### Technical Field

The present invention relates generally to the detection of *Mycobacterium tuberculosis* infection.

### Background of the Invention

Tuberculosis is a chronic, infectious disease, that is generally caused by infection with *Mycobacterium tuberculosis.* It is a major disease in developing countries, as well as an increasing problem in developed areas of the world, with about 8 million new cases and 3 million deaths each year. Although the infection may be asymptomatic for a considerable period of time, the disease is most commonly manifested as an acute inflammation of the lungs, resulting in fever and a nonproductive cough. If left untreated, serious complications and death typically result.

Although tuberculosis can generally be controlled using extended antibiotic therapy, such treatment is not sufficient to prevent the spread of the disease. Infected individuals may be asymptomatic, but contagious, for some time. In addition, although compliance with the treatment regimen is critical, patient behavior is difficult to monitor. Some patients do not complete the course of treatment, which can lead to ineffective treatment and the development of drug resistance.

Inhibiting the spread of tuberculosis will require effective vaccination and accurate, early diagnosis of the disease. Currently, vaccination with live bacteria is the most efficient method for inducing protective immunity. The most common Mycobacterium for this purpose is Bacillus Calmette-Guerin (BCG), an avirulent strain *of Mycobacterium bovis.* However, the safety and efficacy of BCG is a source of controversy and some countries, such as the United States, do not vaccinate the general public. Diagnosis is commonly achieved using a skin test, which involves intradermal exposure to tuberculin PPD (protein-purified derivative). Antigen-specific T cell responses result in measurable incubation at the injection site by 48-72 hours after injection, which indicates exposure to Mycobacterial antigens. Sensitivity and specificity have, however, been a problem with this test, and individuals vaccinated with BCG cannot be distinguished from infected individuals.

While macrophages have been shown to act as the principal effectors of *M. tuberculosis* immunity, T cells are the predominant inducers of such immunity. The essential role of T cells in protection against *M. tuberculosis* infection is illustrated by the frequent occurrence of *M. tuberculosis* in AIDS patients, due to the depletion of CD4 T cells associated with human immunodeficiency virus (HIV) infection. Mycobacterium-reactive CD4 T cells have been shown to be potent producers of gamma-interferon (IFN-γ), which, in turn, has been shown to trigger the anti-mycobacterial effects of macrophages in mice. While the role of IFN-γ in humans is less clear, studies have shown that 1,25-dihydroxy-vitamin D3, either alone or in combination with IFN-γ or tumor necrosis factor-alpha, activates human macrophages to inhibit *M. tuberculosis* infection. Furthermore, it is known that IFN-γ stimulates human macrophages to make 1,25-dihydroxy-vitamin D3. Similarly, IL-12 has been shown to play a role in stimulating resistance to *M. tuberculosis* infection. For a review of the immunology of *M. tuberculosis* infection *see* Chan and Kaufmann, in Tuberculosis: Pathogenesis, Protection and Control, Bloom (ed.), ASM Press, Washington, DC, 1994.

Accordingly, there is a need in the art for improved diagnostic methods for detecting tuberculosis. The present invention fulfills this need and further provides other related advantages.

### Summary of the Invention

Briefly stated, the present invention provides methods for diagnosing tuberculosis. Polypeptides are disclosed comprising an antigenic portion of a soluble *M. tuberculosis* antigen, or a variant of such an antigen that differs only in conservative substitutions and/or modifications.

The soluble antigen may have one of the following N-terminal sequences:
(a) Asp-Pro-Val-Asp-Ala-Val-Ile-Asn-Thr-Thr-Cys-Asn-Tyr-Gly-Gln-Val-Val-Ala-Ala-Leu (SEQ ID No. 115);
(b) Ala-Val-Glu-Ser-Gly-Met-Leu-Ala-Leu-Gly-Thr-Pro-Ala-Pro-Ser (SEQ ID No. 116);
(c) Ala-Ala-Met-Lys-Pro-Arg-Thr-Gly-Asp-Gly-Pro-Leu-Glu-Ala-Ala-Lys-Glu-Gly-Arg (SEQ ID No. 117);
(d) Tyr-Tyr-Trp-Cys-Pro-Gly-Gln-Pro-Phe-Asp-Pro-Ala-Trp-Gly-Pro (SEQ ID No. 118);
(e) Asp-Ile-GIy-Ser-Glu-Ser-Thr-Glu-Asp-Gln-Gln-Xaa-Ala-Val (SEQ ID No. 119);
(f) Ala-Glu-Glu-Ser-Ile-Ser-Thr-Xaa-Glu-Xaa-Ile-Val-Pro (SEQ ID No. 120);
(g) Asp-Pro-Glu-Pro-Ala-Pro-Pro-Val-Pro-Thr-Thr-Ala-Ala-Ser-Pro-Pro-Ser (SEQ ID No. 121);
(h) Ala-Pro-Lys-Thr-Tyr-Xaa-Glu-Glu-Leu-Lys-Gly-Thr-Asp-Thr-Gly (SEQ ID No. 122);
(i) Asp-Pro-Ala-Ser-Ala-Pro-Asp- Val-Pro- Thr-Ala-Ala-GIn-Leu-Thr-Ser-Leu-Leu-Asn-Ser-Leu-Ala-Asp-Pro-Asn-Val-Ser-Phe-Ala-Asn (SEQ ID No. 123);
(j) Xaa-Asp-Ser-Glu-Lys-Ser-Ala-Thr-Ile-Lys-Val-Thr-Asp-Ala-Ser; (SEQ ID No. 129)
(k) Ala-Gly-Asp-Thr-Xaa-Ile-Tyr-Ile-Val-Gly-Asn-Leu-Thr-Ala-Asp; (SEQ ID No. 130) or
(l) Ala-Pro-Glu-Ser-Gly-Ala-Gly-Leu-Gly-Gly-Thr-Val-Gln-Ala-Gly; (SEQ ID No. 131)
wherein Xaa may be any amino acid.

Polypeptides are also disclosed comprising an immunogenic portion of an *M. tuberculosis* antigen, or a variant of such an antigen that differs only in conservative substitutions and/or modifications, the antigen having one of the following N-tenninal sequences:
(m) Xaa-Tyr-Ile-Ala-Tyr-Xaa-Thr-Thr-Ala-Gly-Ile-Val-Pro-Gly-Lys-Ile-Asn-Val-His-Leu-Val; (SEQ ID No. 132) or
(n) Asp-Pro-Pro-Asp-Pro-His-Gln-Xaa-Asp-Met-Thr-Lys-Gly-Tyr-Tyr-Pro-Gly-Gly-Arg-Arg-Xaa-Phe; (SEQ ID No. 124)
wherein Xaa may be any amino acid.

The antigens disclosed may comprise an amino acid sequence encoded by a DNA sequence selected from the group consisting, of the sequences recited in SEQ ID Nos. 1, 2, 4-10, 13-25, 52, 94 and 96, the complements of said sequences, and DNA sequences that hybridize to a sequence recited in SEQ ID Nos. 1, 2, 4-10, 13-25, 52, 94 and 96 or a complement thereof under moderately stringent conditions.

The disclosed polypeptides may comprise an antigenic portion of a *M. tuberculosis* antigen, or a variant of such an antigen that differs only in conservative substitutions and/or modifications, wherein the antigen comprises an amino acid sequence encoded by a DNA sequence selected from the group consisting of the sequences recited in SEQ ID Nos. 26-51, the complements of said sequences, and DNA sequences that hybridize to a sequence recited in SEQ ID Nos. 26-51 or a complement thereof under moderately stringent conditions.

DNA sequences encoding the above polypeptides, recombinant expression vectors comprising these DNA sequences and host cells transformed or transfected with such expression vectors are also disclosed.

According to the present invention is provided a method for detecting sensitisation to a mycobacterial antigen in a subject comprising: (a) contacting a biological sample from said subject with a polypeptide comprising an antigenic portion of SEQ ID No: 89; and (b) detecting in the biological sample the presence of antibodies that bind to the polypeptide.

Methods and diagnostic kits are provided for detecting tuberculosis in a patient. The methods comprise (a) contacting a biological sample with at least one polypeptide comprising an antigenic portion of SEQ ID No: 89 and (b) detecting in the sample the presence of antibodies that bind to the polypeptide or polypeptides, thereby detecting *M. tuberculosis* infection in the biological sample. Suitable biological samples include whole blood, sputum, serum, plasma, saliva, cerebrospinal fluid and urine. The diagnostic kits comprise one or more of the above polypeptides in combination with a detection reagent.

### Brief Description of the Drawings and Sequence Identifiers

Figure 1A and B illustrate the stimulation of proliferation and interferon-γ production in T cells derived from a first and a second *M. tuberculosis*-immune donor, respectively, by the 14 Kd, 20 Kd and 26 Kd antigens described in Example 1.
Figure 2 illustrates the reactivity of two representative polypeptides with sera from *M. tuberculosis*-infected and uninfected individuals, as compared to the reactivity of bacterial lysate.
Figure 3 shows the reactivity of four representative polypeptides with sera from *M. tuberculosis*-infected and uninfected individuals, as compared to the reactivity of the 38 kD antigen.
Figure 4 shows the reactivity of recombinant 38 kD and TbRa11 antigens with sera from *M. tuberculosis* patients, PPD positive donors and normal donors.
Figure 5 shows the reactivity of the antigen TbRa2A with 38 kD negative sera.
Figure 6 shows the reactivity of the antigen of SEQ ID No. 60 with sera from *M. tuberculosis* patients and normal donors.

SEQ. ID NO. 1 is the DNA sequence of TbRa1.
SEQ. ID NO. 2 is the DNA sequence of TbRa10.
SEQ. ID NO. 3 is the DNA sequence of TbRa11.
SEQ. ID NO. 4 is the DNA sequence of TbRa12.
SEQ. ID NO. 5 is the DNA sequence of TbRa13.
SEQ. ID NO. 6 is the DNA sequence of TbRa16.
SEQ. ID NO. 7 is the DNA sequence of TbRa17.
SEQ. ID NO. 8 is the DNA sequence of TbRa18.
SEQ. ID NO. 9 is the DNA sequence of TbRa19.
SEQ. ID NO. 10 is the DNA sequence of TbRa24.
SEQ. ID NO. 11 is the DNA sequence of TbRa26.
SEQ. ID NO. 12 is the DNA sequence of TbRa28.
SEQ. ID NO. 13 is the DNA sequence of TbRa29.
SEQ. ID NO. 14 is the DNA sequence of TbRa2A.
SEQ. ID NO. 15 is the DNA sequence of TbRa3.
SEQ. ID NO. 16 is the DNA sequence of TbRa32.
SEQ. ID NO. 17 is the DNA sequence of TbRa35.
SEQ. ID NO. 18 is the DNA sequence of TbRa36.
SEQ. ID NO. 19 is the DNA sequence of TbRa4.
SEQ. ID NO. 20 is the DNA sequence of TbRa9.
SEQ. ID NO. 21 is the DNA sequence of TbRaB.
SEQ. ID NO. 22 is the DNA sequence of TbRaC.
SEQ. ID NO. 23 is the DNA sequence of TbRaD.
SEQ. ID NO. 24 is the DNA sequence of YYWCPG.
SEQ. ID NO. 25 is the DNA sequence of AAMK.
SEQ. ID NO. 26 is the DNA sequence of TbL-23.
SEQ. ID NO. 27 is the DNA sequence of TbL-24.
SEQ. ID NO. 28 is the DNA sequence of TbL-25.
SEQ: ID NO. 29 is the DNA sequence of TbL-28.
SEQ. ID NO. 30 is the DNA sequence of TbL-29.
SEQ. ID NO. 31 is the DNA sequence of TbH-5.
SEQ. ID NO. 32 is the DNA sequence of TbH-8.
SEQ. ID NO. 33 is the DNA sequence of TbH-9.
SEQ. ID NO. 34 is the DNA sequence of TbM-1.
SEQ. ID NO. 35 is the DNA sequence of TbM-3.
SEQ. ID NO. 36 is the DNA sequence of TbM-6.
SEQ. ID NO. 37 is the DNA sequence of TbM-7.
SEQ. ID NO. 38 is the DNA sequence of TbM-9.
SEQ. ID NO. 39 is the DNA sequence of TbM-12.
SEQ. ID NO. 40 is the DNA sequence ofTbM-13.
SEQ. ID NO. 41 is the DNA sequence of TbM-14.
SEQ. ID NO. 42 is the DNA sequence of TbM-15.
SEQ. ID NO. 43 is the DNA sequence of TbH-4.
SEQ. ID NO. 44 is the DNA sequence of TbH-4-FWD.
SEQ. ID NO. 45 is the DNA sequence of TbH-12.
SEQ. ID NO. 46 is the DNA sequence of Tb38-1.
SEQ. ID NO. 47 is the DNA sequence of Tb38-4.
SEQ. ID NO. 48 is the DNA sequence of TbL-17.
SEQ. ID NO. 49 is the DNA sequence of TbL-20.
SEQ. ID NO. 50 is the DNA sequence of TbL-21.
SEQ. ID NO. 51 is the DNA sequence of TbH-16.
SEQ. ID NO. 52 is the DNA sequence of DPEP.
SEQ. ID NO. 53 is the deduced amino acid sequence of DPEP.
SEQ. ID NO. 54 is the protein sequence of DPV N-terminal Antigen.
SEQ. ID NO. 55 is the protein sequence of AVGS N-terminal Antigen.
SEQ. ID NO. 56 is the protein sequence of AAMK N-terminal Antigen.
SEQ. ID NO. 57 is the protein sequence of YYWC N-terminal Antigen.
SEQ. ID NO. 58 is the protein sequence of DIGS N-terminal Antigen.
SEQ. ID NO. 59 is the protein sequence of AEES N-terminal Antigen.
SEQ. ID NO. 60 is the protein sequence of DPEP N-terminal Antigen.
SEQ. ID NO. 61 is the protein sequence of APKT N-terminal Antigen.
SEQ. ID NO. 62 is the protein sequence of DPAS N-terminal Antigen.
SEQ. ID NO. 63 is the deduced amino acid sequence of TbM-1 Peptide.
SEQ. ID NO. 64 is the deduced amino acid sequence of TbRa1.
SEQ. ID NO. 65 is the deduced amino acid sequence of ThRa10
SEQ. ID NO. 66 is the deduced amino acid sequence of TbRa11.
SEQ. ID NO. 67 is the deduced amino acid sequence of TbRa12.
SEQ. ID NO. 68 is the deduced amino acid sequence of TbRa13.
SEQ. ID NO. 69 is the deduced amino acid sequence of TbRa16.
SEQ. ID NO. 70 is the deduced amino acid sequence of TbRa17.
SEQ. ID NO. 71 is the deduced amino acid sequence of TbRa18.
SEQ. ID NO. 72 is the deduced amino acid sequence of TbRa19.
SEQ. ID NO. 73 is the deduced amino acid sequence of TbRa24.
SEQ. ID NO. 74 is the deduced amino acid sequence of TbRa26.
SEQ. ID NO. 75 is the deduced amino acid sequence of TbRa28.
SEQ. ID NO. 76 is the deduced amino acid sequence of TbRa29.
SEQ. ID NO. 77 is the deduced amino acid sequence of TbRa2A.
SEQ. ID NO. 78 is the deduced amino acid sequence of TbRa3.
SEQ. ID NO. 79 is the deduced amino acid sequence of TbRa32.
SEQ. ID NO. 80 is the deduced amino acid sequence of TbRa35.
SEQ. ID NO. 81 is the deduced amino acid sequence of TbRa36.
SEQ. ID NO. 82 is the deduced amino acid sequence of TbRa4.
SEQ. ID NO. 83 is the deduced amino acid sequence of TbRa9.
SEQ. ID NO. 84 is the deduced amino acid sequence of TbRaB.
SEQ. ID NO. 85 is the deduced amino acid sequence of TbRaC.
SEQ. ID NO. 86 is the deduced amino acid sequence of TbRaD.
SEQ. ID NO. 87 is the deduced amino acid sequence of YYWCPG.
SEQ. ID NO. 88 is the deduced amino acid sequence of TbAAMK.
SEQ. ID NO. 89 is the deduced amino acid sequence of Tb38-1.
SEQ. ID NO. 90 is the deduced amino acid sequence of TbH-4.
SEQ. ID NO. 91 is the deduced amino acid sequence of TbH-8.
SEQ. ID NO. 92 is the deduced amino acid sequence of TbH-9.
SEQ. ID NO. 93 is the deduced amino acid sequence of TbH-12.
SEQ. ID NO. 94 is the DNA sequence of DPAS.
SEQ. ID NO. 95 is the deduced amino acid sequence of DPAS.
SEQ. ID NO. 96 is the DNA sequence of DPV.
SEQ. ID NO. 97 is the deduced amino acid sequence of DPV.
SEQ. ID NO. 98 is the DNA sequence of ESAT-6.
SEQ. ID NO. 99 is the deduced amino acid sequence of ESAT-6.
SEQ. ID NO. 100 is the DNA sequence of TbH-8-2.
SEQ. ID NO. 101 is the DNA sequence of TbH-9FL.
SEQ. ID NO. 102 is the deduced amino acid sequence of TbH-9FL.
SEQ ID NO. 103 is the DNA sequence of TbH-9-1.
SEQ. ID NO. 104 is the deduced amino acid sequence of TbH-9-1.
SEQ. ID NO. 105 is the DNA sequence of TbH-9-4.
SEQ. ID NO. 106 is the deduced amino acid sequence of TbH-9-4.
(SEQ. ID NO. 107 is the DNA sequence of Tb38-1F2 IN.
SEQ. ID NO. 108 is the DNA sequence of Tb38-1F2 RP.
SEQ. ID NO. 109 is the deduced amino acid sequence of Tb37-FL.
SEQ. ID NO. 110 is the deduced amino acid sequence of Tb38-IN.
SEQ. ID NO. 111 is the DNA sequence of Tb38-1F3.
SEQ. ID NO. 112 is the deduced amino acid sequence of Tb38-1F3.
SEQ. ID NO. 113 is the DNA sequence of Tb38-1F5.
SEQ. ID NO. 114 is the DNA sequence of Tb38-1F6.
SEQ. ID NO. 115 is the deduced N-terminal amino acid sequence of DPV.
SEQ. ID NO. 116 is the deduced N-terminal amino acid sequence of AVGS.
SEQ. ID NO. 117 is the deduced N-terminal amino acid sequence of AAMK.
SEQ. ID NO. 118 is the deduced N-terminal amino acid sequence of YYWC.
SEQ. ID NO. 119 is the deduced N-terminal amino acid sequence of DIGS.
SEQ. ID NO. 120 is the deduced N-terminal amino acid sequence of AAES.
SEQ. ID NO. 121 is the deduced N-terminal amino acid sequence of DPEP.
SEQ. ID NO. 122 is the deduced N-terminal amino acid sequence of APKT.
SEQ. ID NO. 123 is the deduced N-terminal amino acid sequence of DPAS.
SEQ. ID NO. 124 is the protein sequence of DPPD N-terminal Antigen.
SEQ ID NO. 125-128 are the protein sequences of four DPPD cyanogen bromide fragments.
SEQ ID NO. 129 is the N-terminal protein sequence of XDS antigen.
SEQ ID NO. 130 is the N-terminal protein sequence of AGD antigen.
SEQ ID NO. 131 is the N-terminal protein sequence of APE antigen.
SEQ ID NO. 132 is the N-tenninal protein sequence of XYI antigen.

### Detailed Description of the Invention

As noted above, the present invention is generally directed to methods for diagnosing tuberculosis.

As used herein, the term "polypeptide" encompasses amino acid chains of any length, including full length proteins (*i.e.,* antigens), wherein the amino acid residues are linked by covalent peptide bonds. Thus, a polypeptide comprising an antigenic portion of one of the above antigens may consist entirely of the antigenic portion, or may contain additional sequences. The additional sequences may be derived from the native *M. tuberculosis* antigen or may be heterologous, and such sequences may (but need not) be antigenic.

An "antigenic portion" of an antigen (which may or may not be soluble) is a portion that is capable of reacting with sera obtained from an *M. tuberculosis-*infected individual (*i.e.,* generates an absorbance reading with sera from infected individuals that is at least three standard deviations above the absorbance obtained with sera from uninfected individuals, in a representative ELISA assay described herein). An *"M. tuberculosis*-infected individual" is a human who has been infected with *M. tuberculosis* (*e.g.,* has an intradermal skin test response to PPD that is at least 0.5 cm in diameter). Infected individuals may display symptoms of tuberculosis or may be free of disease symptoms. Polypeptides comprising at least an antigenic portion of one or more *M. tuberculosis* antigens as described herein may generally be used, alone or in combination, to detect tuberculosis in a patient.

A "variant," as used herein, is a polypeptide that differs from the native antigen only in conservative substitutions and/or modifications, such that the antigenic properties of the polypeptide are retained. Such variants may generally be identified by modifying one of the above polypeptide sequences, and evaluating the antigenic properties of the modified polypeptide using, for example, the representative procedures described herein.

A "conservative substitution" is one in which an amino acid is substituted for another amino acid that has similar properties, such that one skilled in the art of peptide chemistry would expect the secondary structure and hydropathic nature of the polypeptide to be substantially unchanged. In general, the following groups of amino acids represent conservative changes: (1) ala, pro, gly, glu, asp, gin, asn, ser, thr; (2) cys, ser, tyr, thr; (3) val, ile, leu, met, ala, phe; (4) lys, arg, his; and (5) phe, tyr, trp, his.

Variants may also (or alternatively) be modified by, for example, the deletion or addition of amino acids that have minimal influence on the antigenic properties, secondary structure and hydropathic nature of the polypeptide. For example, a polypeptide may be conjugated to a signal (or leader) sequence at the N-terminal end of the protein which co-translationally or post-translationally directs transfer of the protein. The polypeptide may also be conjugated to a linker or other sequence for ease of synthesis, purification or identification of the polypeptide (*e.g.,* poly-His), or to enhance binding of the polypeptide to a solid support. For example, a polypeptide may be conjugated to an immunoglobulin Fc region.

Combination polypeptides are disclosed. A "combination polypeptide" is a polypeptide comprising at least one of the above antigenic portions and one or more additional antigenic *M. tuberculosis* sequences, which are joined via a peptide linkage into a single amino acid chain. The sequences may be joined directly (*i.e.,* with no intervening amino acids) or may be joined by way of a linker sequence (*e.g.*, Gly-Cys-Gly) that does not significantly diminish the antigenic properties of the component polypeptides.

In general, *M. tuberculosis* antigens, and DNA sequences encoding such antigens, may be prepared using any of a variety of procedures. For example, soluble antigens may be isolated from *M. tuberculosis* culture filtrate by procedures known to those of ordinary skill in the art, including anion-exchange and reverse phase chromatography. Purified antigens may then be evaluated for a desired property, such as the ability to react with sera obtained from an *M. tuberculosis*-infected individual. Such screens may be performed using the representative methods described herein. Antigens may then be partially sequenced using, for example, traditional Edman chemistry. *See* Edman and Berg, Eur. J. Biochem. 80:116-132, 1967.

Antigens may also be produced recombinantly using a DNA sequence that encodes the antigen, which has been inserted into an expression vector and expressed in an appropriate host. DNA molecules encoding soluble antigens may be isolated by screening an appropriate *M. tuberculosis* expression library with anti-sera *(e.g.,* rabbit) raised specifically against soluble *M. tuberculosis* antigens. DNA sequences encoding antigens that may or may not be soluble may be identified by screening an appropriate *M. tuberculosis* genomic or cDNA expression library with sera obtained from patients infected with *M. tuberculosis.* Such screens may generally be performed using techniques well known in the art, such as those described in Sambrook et al., Molecular Cloning: A Laboratory Manual, Cold Spring Harbor Laboratories, Cold Spring Harbor, NY, 1989.

DNA sequences encoding soluble antigens may also be obtained by screening an appropriate *M. tuberculosis* cDNA or genomic DNA library for DNA sequences that hybridize to degenerate oligonucleotides derived from partial amino acid sequences of isolated soluble antigens. Degenerate oligonucleotide sequences for use in such a screen may be designed and synthesized, and the screen may be performed, as described (for example) in Sambrook et al., Molecular Cloning: A Laboratory Manual, Cold Spring Harbor Laboratories, Cold Spring Harbor, NY (and references cited therein). Polymerase chain reaction (PCR) may also be employed, using the above oligonucleotides in methods well known in the art, to isolate a nucleic acid probe from a cDNA or genomic library. The library screen may then be performed using the isolated probe.

Regardless of the method of preparation, the antigens described herein are "antigenic." More specifically, the antigens have the ability to react with sera obtained from an *M. tuberculosis*-infected individual. Reactivity may be evaluated using, for example, the representative ELISA assays described herein, where an absorbance reading with sera from infected individuals that is at least three standard deviations above the absorbance obtained with sera from uninfected individuals is considered positive.

Antigenic portions of *M. tuberculosis* antigens may be prepared and identified using well known techniques, such as those summarized in Paul, Fundamental Immunology, 3d ed., Raven Press, 1993, pp. 243-247 and references cited therein. Such techniques include screening polypeptide portions of the native antigen for antigenic properties. The representative ELISAs described herein may generally be employed in these screens. An antigenic portion of a polypeptide is a portion that, within such representative assays, generates a signal in such assays that is substantially similar to that generated by the full length antigen. In other words, an antigenic portion of a *M. tuberculosis* antigen generates at least about 20%, and preferably about 100%, of the signal induced by the full length antigen in a model ELISA as described herein.

Portions and other variants of *M. tuberculosis* antigens may be generated by synthetic or recombinant means. Synthetic polypeptides having fewer than about 100 amino acids, and generally fewer than about 50 amino acids, may be generated using techniques well known in the art. For example, such polypeptides may be synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Applied BioSystems, Inc., Foster City, CA, and may be operated according to the manufacturer's instructions. Variants of a native antigen may generally be prepared using standard mutagenesis techniques, such as oligonucleotide-directed site-specific mutagenesis. Sections of the DNA sequence may also be removed using standard techniques to permit preparation of truncated polypeptides.

Recombinant polypeptides containing portions and/or variants of a native antigen may be readily prepared from a DNA sequence encoding the polypeptide using a variety of techniques well known to those of ordinary skill in the art. For example, supernatants from suitable host/vector systems which secrete recombinant protein into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant protein.

Any of a variety of expression vectors known to those of ordinary skill in the art may be employed to express recombinant polypeptides as described herein. Expression may be achieved in any appropriate host cell that has been transformed or transfected with an expression vector containing a DNA molecule that encodes a recombinant polypeptide. Suitable host cells include prokaryotes, yeast and higher eukaryotic cells. Preferably, the host cells employed are *E. coli,* yeast or a mammalian cell line, such as COS or CHO. The DNA sequences expressed in this manner may encode naturally occurring antigens, portions of naturally occurring antigens, or other variants thereof.

In general, regardless of the method of preparation, the polypeptides disclosed herein are prepared in substantially pure form. Preferably, the polypeptides are at least about 80% pure, more preferably at least about 90% pure and most preferably at least about 99% pure. For use in the methods described herein, however, such substantially pure polypeptides may be combined.

Disclosed herein are polypeptides comprising at least an antigenic portion of a soluble *M. tuberculosis* antigen (or a variant of such an antigen), where the antigen has one of the following N-terminal sequences:
(a) Asp-Pro-Val-Asp-Ala-Val-Ile-Asn-Thr-Thr-Cys-Asn-Tyr-Gly-Gln-Val-Val-Ala-Ala-Leu (SEQ ID No. 115);
(b) Ala-Val-Glu-Ser-Gly-Met-Leu-Ala-Leu-Gly-Thr-Pro-Ala-Pro-Ser (SEQ ID No. 116);
(c) Ala-Ala-Met-Lys-Pro-Arg-Thr-Gly-Asp-Gly-Pro-Leu-Glu-Ala-Ala-Lys-Glu-Gly-Arg (SEQ ID No. 117);
(d) Tyr-Tyr-Trp-Cys-Pro-Gly-Gln-Pro-Phe-Asp-Pro-Ala-Trp-Gly-Pro (SEQ ID No. 118);
(e) Asp-Ile-Gly-Ser-Glu-Ser-Thr-Glu-Asp-Gln-Gln-Xaa-Ala-Val (SEQ ID No. 119);
(f) Ala-Glu-Glu-Ser-Ile-Ser-Thr-Xaa-Glu-Xaa-Ile-Val-Pro (SEQ ID No. 120);
(g) Asp-Pro-Glu-Pro-Ala-Pro-Pro-Val-Pro-Thr-Thr-Ala-Ala-Ser-Pro-Pro-Ser (SEQ ID No. 121);
(h) Ala-Pro-Lys-Thr-Tyr-Xaa-Glu-Glu-Leu-Lys-Gly-Thr-Asp-T'hr-Gly (SEQ ID No. 122);
(i) Asp-Pro-Ala-Ser-Ala-Pro-Asp-Val-Pro-Thr-Ala-Ala-Gln-Gln-Thr-Ser-Leu-Leu-Asn-Ser-Leu-Ala-Asp-Pro-Asn-Val-Ser-Phe-Ala-Asn (SEQ ID No. 123);
(j) Xaa-Asp-Ser-Glu-Lys-Ser-Ala-Thr-Ile-Lys-Val-Thr-Asp-Ala-Ser; (SEQ ID No. 129)
(k) Ala-Gly-Asp-Thr-Xaa-Ile-Tyr-Ile-Val-Gly-Asn-Leu-Thr-Ala-Asp; (SEQ ID No. 130) or
(l) Ala-Pro-Glu-Ser-Gly-Ala-Gly-Leu-Gly-Gly-Thr-Val-Gln-Ala-Gly; (SEQ ID No. 131)
wherein Xaa may be any amino acid, preferably a cysteine residue. A DNA sequence encoding the antigen identified as (g) above is provided in SEQ ID No. 52, the deduced amino acid sequence of which is provided in SEQ ID No. 53. A DNA sequence encoding the antigen identified as (a) above is provided in SEQ ID No. 96; its deduced amino acid sequence is provided in SEQ ID No. 97. A DNA sequence corresponding to antigen (d) above is provided in SEQ ID No. 24, a DNA sequence corresponding to antigen (c) is provided in SEQ ID No. 25 and a DNA sequence corresponding to antigen (I) is disclosed in SEQ ID No. 94 and its deduced amino acid sequence is provided in SEQ ID No. 95.

Also disclosed herein are polypeptides comprising at least an immunogenic portion of an *M. tuberculosis* antigen having one of the following N-terminal sequences, or a variant thereof that differs only in conservative substitutions and/or modifications:
(m) Xaa-Tyr-Ile-Ala-Tyr-Xaa-Thr-Thr-Ala-Gly-Ile-Val-Pro-Gly-Lys-Ile-Asn-Val-His-Leu-Val; (SEQ ID No. 132) or
(n) Asp-Pro-Pro-Asp-Pro-His-Gln-Xaa-Asp-Met-Thr-Lys-Gly-Tyr-Tyr-Pro-Gly-Gly-Arg-Arg-Xaa-Phe; (SEQ ID No. 124)
wherein Xaa may be any amino acid, preferably a cysteine residue.

Also disclosed herein are polypeptides comprising at least an antigenic portion of a soluble *M. tuberculosis* antigen (or a variant of such an antigen) that comprises one or more of the amino acid sequences encoded by (a) the DNA sequences of SEQ. ID Nos. 1, 2, 4-10, 13-25, 52, 94 and 96, (b) the complements of such DNA sequences, or (c) DNA sequences substantially homologous to a sequence in (a) or (b).

Also disclosed herein are polypeptides comprising at least an antigenic portion of a *M. tuberculosis* antigen (or a variant of such an antigen), which may or may not be soluble, that comprises one or more of the amino acid sequences encoded by (a) the DNA sequences of SEQ ID Noes. 26-51, (b) the complements of such DNA sequences or (c) DNA sequences substantially homologous to a sequence in (a) or (b).

*M. tuberculosis* antigens include variants that are encoded DNA sequences which are substantially homologous to one or more of DNA sequences specifically recited herein. "Substantial homology," as used herein, refers to DNA sequences that are capable of hybridizing under moderately stringent conditions. Suitable moderately stringent conditions include prewashing in a solution of 5X SSC, 0.5% SDS, 1.0 mM EDTA (pH 8.0); hybridizing at 50°C-65°C, 5X SSC, overnight or, in the event of cross-species homology, at 45°C with 0.5X SSC; followed by washing twice at 65°C for 20 minutes with each of 2X, 0.5X and 0.2X SSC containing 0.1 % SDS).

Also disclosed herein are fusion proteins comprising a first and a second polypeptide as disclosed herein, or alternatively a polypeptide as disclosed herein and a known *M. tuberculosis* antigen, such as the 38 kD antigen described above or ESAT-6 (SEQ ID Nos. 98 and 99), together with variants of such fusion proteins. The fusion proteins may also include a linker peptide between the first and second polypeptides.

A DNA sequence encoding a fusion protein is constructed using known recombinant DNA techniques to assemble separate DNA sequences encoding the first and second polypeptides into an appropriate expression vector. The 3' end of a DNA sequence encoding the first polypeptide is ligated, with or without a peptide linker, to the 5' end of a DNA sequence encoding the second polypeptide so that the reading frames of the sequences are in phase to permit mRNA translation of the two DNA sequences into a single fusion protein that retains the biological activity of both the first and the second polypeptides.

A peptide linker sequence may be employed to separate the first and the second polypeptides by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad Sci. USA 83:8258-8562, 1986; U.S. Patent No. 4,935,233 and U.S. Patent No. 4,715,180. The linker sequence may be from I to about 50 amino acids in length. Peptide linker sequences are not required when the first and second polypeptides have non-essential N-terminal amino acid regions that can be used to separate the functional domains and prevent steric hindrance.

In another aspect, methods are provided for detecting *M. tuberculosis* infection in a biological sample

In embodiments in which multiple polypeptides are employed, polypeptides other than those specifically described herein, such as the 38 kD antigen described in Andersen and Hansen, Infect. Immun. 57:2481-2488, 1989, may be included. As used herein, a "biological sample" is any antibody-containing sample obtained from a patient. Preferably, the sample is whole blood, sputum, serum, plasma, saliva, cerebrospinal fluid or urine. More preferably, the sample is a blood, serum or plasma sample obtained from a patient or a blood supply. The polypeptide(s) are used in an assay, as described below, to determine the presence or absence of antibodies to the polypeptide(s) in the sample, relative to a predetermined cut-off value. The presence of such antibodies indicates previous sensitization to mycobacteria antigens which may be indicative of tuberculosis.

In embodiments in which more than one polypeptide is employed, the polypeptides used are preferably complementary (*i.e.,* one component polypeptide will tend to detect infection in samples where the infection would not be detected by another component polypeptide). Complementary polypeptides may generally be identified by using each polypeptide individually to evaluate serum samples obtained from a series of patients known to be infected with *M. tuberculosis.* After determining which samples test positive (as described below) with each polypeptide, combinations of two or more polypeptides may be formulated that are capable of detecting infection in most, or all, of the samples tested. Such polypeptides are complementary. For example, approximately 25-30% of sera from tuberculosis-infected individuals are negative for antibodies to any single protein, such as the 38 kD antigen mentioned above. Complementary polypeptides may, therefore, be used in combination with the 38 kD antigen to improve sensitivity of a diagnostic test.

There are a variety of assay formats known to those of ordinary skill in the art for using one or more polypeptides to detect antibodies in a sample. *See, e.g.,* Harlow and Lane, Antibodies: A Laboratory Manual, Cold Spring Harbor Laboratory, 1988. In a preferred embodiment, the assays involves the use of polypeptide immobilized on a solid support to bind to and remove the antibody from the sample. The bound antibody may then be detected using a detection reagent that contains a reporter group. Suitable detection reagents include antibodies that bind to the antibody/polypeptide complex and free polypeptide labeled with a reporter group (*e.g.*, in a semi-competitive assay). Alternatively, a competitive assay may be utilized, in which an antibody that binds to the polypeptide is labeled with a reporter group and allowed to bind to the immobilized antigen after incubation of the antigen with the sample. The extent to which components of the sample inhibit the binding of the labeled antibody to the polypeptide is indicative of the reactivity of the sample with the immobilized polypeptide.

The solid support may be any solid material known to those of ordinary skill in the art to which the antigen may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681.

The polypeptides may be bound to the solid support using a variety of techniques known to those of ordinary skill in the art, which are amply described in the patent and scientific literature. In the context of the present invention, the term "bound" refers to both noncovalent association, such as adsorption, and covalent attachment (which may be a direct linkage between the antigen and functional groups on the support or may be a linkage by way of a cross-linking agent). Binding by adsorption to a well in a microtiter plate or to a membrane is preferred. In such cases, adsorption may be achieved by contacting the polypeptide, in a suitable buffer, with the solid support for a suitable amount of time. The contact time varies with temperature, but is typically between about 1 hour and 1 day. In general, contacting a well of a plastic microtiter plate (such as polystyrene or polyvinylchloride) with an amount of polypeptide ranging from about 10 ng to about 1 µg, and preferably about 100 ng, is sufficient to bind an adequate amount of antigen.

Covalent attachment of polypeptide to a solid support may generally be achieved by first reacting the support with a bifunctional reagent that will react with both the support and a functional group, such as a hydroxyl or amino group, on the polypeptide. For example, the polypeptide may be bound to supports having an appropriate polymer coating using benzoquinone or by condensation of an aldehyde group on the support with an amine and an active hydrogen on the polypeptide (*see, e.g.*, Pierce Immunotechnology Catalog and Handbook, 1991, at A12-A13).

In certain embodiments, the assay is an enzyme linked immunosorbent assay (ELISA). This assay may be performed by first contacting a polypeptide antigen that has been immobilized on a solid support, commonly the well of a microtiter plate, with the sample, such that antibodies to the polypeptide within the sample are allowed to bind to the immobilized polypeptide. Unbound sample is then removed from the immobilized polypeptide and a detection reagent capable of binding to the immobilized antibody-polypeptide complex is added. The amount of detection reagent that remains bound to the solid support is then determined using a method appropriate for the specific detection reagent.

More specifically, once the polypeptide is immobilized on the support as described above, the remaining protein binding sites on the support are typically blocked. Any suitable blocking agent known to those of ordinary skill in the art, such as bovine serum albumin or Tween 20™ (Sigma Chemical Co., St. Louis, MO) may be employed. The immobilized polypeptide is then incubated with the sample, and antibody is allowed to bind to the antigen. The sample may be diluted with a suitable diluent, such as phosphate-buffered saline (PBS) prior to incubation. In general, an appropriate contact time (*i.e.,* incubation time) is that period of time that is sufficient to detect the presence of antibody within a *M. tuberculosis*-infected sample. Preferably, the contact time is sufficient to achieve a level of binding that is at least 95% of that achieved at equilibrium between bound and unbound antibody. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20™. Detection reagent may then be added to the solid support. An appropriate detection reagent is any compound that binds to the immobilized antibody-polypeptide complex and that can be detected by any of a variety of means known to those in the art. Preferably, the detection reagent contains a binding agent (such as, for example, Protein A, Protein G, immunoglobulin, lectin or free antigen) conjugated to a reporter group. Preferred reporter groups include enzymes (such as horseradish peroxidase), substrates, cofactors, inhibitors, dyes, radionuclides, luminescent groups, fluorescent groups and biotin. The conjugation of binding agent to reporter group may be achieved using standard methods known to those of ordinary skill in the art. Common binding agents may also be purchased conjugated to a variety of reporter groups from many commercial sources (*e.g.*, Zymed Laboratories, San Francisco, CA, and Pierce, Rockford, IL).

The detection reagent is then incubated with the immobilized antibody-polypeptide complex for an amount of time sufficient to detect the bound antibody. An appropriate amount of time may generally be determined from the manufacturer's instructions or by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of anti-*M*. *tuberculosis* antibodies in the sample, the signal detected from the reporter group that remains bound to the solid support is generally compared to a signal that corresponds to a predetermined cut-off value. In one preferred embodiment, the cut-off value is the average mean signal obtained when the immobilized antigen is incubated with samples from an uninfected patient. In general, a sample generating a signal that is three standard deviations above the predetermined cut-off value is considered positive for tuberculosis. In an alternate preferred embodiment, the cut-off value is determined using a Receiver Operator Curve, according to the method of Sackett et al., Clinical Epidemiology: A Basic Science for Clinical Medicine, Little Brown and Co., 1985, pp. 106-107. Briefly, in this embodiment, the cut-off value may be determined from a plot of pairs of true positive rates (*i.e.,* sensitivity) and false positive rates (100%-specificity) that correspond to each possible cut-off value for the diagnostic test result. The cut-off value on the plot that is the closest to the upper left-hand corner (*i.e.,* the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for tuberculosis.

In a related embodiment, the assay is performed in a rapid flow-through or strip test format, wherein the antigen is immobilized on a membrane, such as nitrocellulose. In the flow-through test, antibodies within the sample bind to the immobilized polypeptide as the sample passes through the membrane. A detection reagent (*e.g.*, protein A-colloidal gold) then binds to the antibody-polypeptide complex as the solution containing the detection reagent flows through the membrane. The detection of bound detection reagent may then be performed as described above. In the strip test format, one end of the membrane to which polypeptide is bound is immersed in a solution containing the sample. The sample migrates along the membrane through a region containing detection reagent and to the area of immobilized polypeptide. Concentration of detection reagent at the polypeptide indicates the presence of anti-*M. tuberculosis* antibodies in the sample. Typically, the concentration of detection reagent at that site generates a pattern, such as a line, that can be read visually. The absence of such a pattern indicates a negative result. In general, the amount of polypeptide immobilized on the membrane is selected to generate a visually discernible pattern when the biological sample contains a level of antibodies that would be sufficient to generate a positive signal in an ELISA, as discussed above. Preferably, the amount of polypeptide immobilized on the membrane ranges from about 25 ng to about 1 µg, and more preferably from about 50 ng to about 500 ng. Such tests can typically be performed with a very small amount (*e.g.*, one drop) of patient serum or blood.

Of course, numerous other assay protocols exist that are suitable for use with the polypeptides of the present invention. The above descriptions are intended to be exemplary only.

The following Examples are offered by way of illustration and not by way of limitation.

### EXAMPLES

### EXAMPLE 1

### PURIFICATION AND CHARACTERIZATION OF POLYPEPTDES FROM M. TUBERCULOSIS CULTURE FILTRATE

This example illustrates the preparation of *M. tuberculosis* soluble polypeptides from culture filtrate. Unless otherwise noted, all percentages in the following example are weight per volume.

*M. tuberculosis* (either H37Ra, ATCC No. 25177, or H37Rv, ATCC No. 25618) was cultured in sterile GAS media at 37°C for fourteen days. The media was then vacuum filtered (leaving the bulk of the cells) through a 0.45 µ filter into a sterile 2.5 L bottle. The media was then filtered through a 0.2 µ filter into a sterile 4 L bottle. NaN₃ was then added to the culture filtrate to a concentration of 0.04%. The bottles were then placed in a 4°C cold room.

The culture filtrate was concentrated by placing the filtrate in a 12 L reservoir that had been autoclaved and feeding the filtrate into a 400 m1 Amicon stir cell which had been rinsed with ethanol and contained a 10,000 kDa MWCO membrane. The pressure was maintained at 60 psi using nitrogen gas. This procedure reduced the 12 L volume to approximately 50 ml.

The culture filtrate was then dialyzed into 0.1 % ammonium bicarbonate using a 8,000 kDa MWCO cellulose ester membrane, with two changes of ammonium bicarbonate solution. Protein concentration was then determined by a commercially available BCA assay (Pierce, Rockford, IL).

The dialyzed culture filtrate was then lyophilized, and the polypeptides resuspended in distilled water. The polypeptides were then dialyzed against 0.01 mM 1,3 bis[tris(hydroxymethyl)-methylamino]propane, pH 7.5 (Bis-Tris propane buffer), the initial conditions for anion exchange chromatography. Fractionation was performed using gel profusion chromatography on a POROS 146 II Q/M anion exchange column 4.6 mm x 100 mm (Perseptive BioSystems, Framingham, MA) equilibrated in 0.01 mM Bis-Tris propane buffer pH 7.5. Polypeptides were eluted with a linear 0-0.5 M NaCI gradient in the above buffer system. The column eluent was monitored at a wavelength of 220 nm.

The pools of polypeptides eluting from the ion exchange column were dialyzed against distilled water and lyophilized. The resulting material was dissolved in 0.1 % trifluoroacetic acid (TFA) pH 1.9 in water, and the polypeptides were purified on a Delta-Pak C18 column (Waters, Milford, MA) 300 Angstrom pore size, 5 micron particle size (3.9 x 150 mm). The polypeptides were eluted from the column with a linear gradient from 0-60% dilution buffer (0.1% TFA in acetonitrile). The flow rate was 0.75 ml/minute and the HPLC eluent was monitored at 214 nm. Fractions containing the eluted polypeptides were collected to maximize the purity of the individual samples. Approximately 200 purified polypeptides were obtained.

The purified polypeptides were then screened for the ability to induce T-cell proliferation in PBMC preparations. The PBMCs from donors known to be PPD skin test positive and whose T cells were shown to proliferate in response to PPD and crude soluble proteins from MTB were cultured in medium comprising RPMI 1640 supplemented with 10% pooled human serum and 50 µg/ml gentamicin. Purified polypeptides were added in duplicate at concentrations of 0.5 to 10 µg/mL. After six days of culture in 96-well round-bottom plates in a volume of 200 µl, 50 µl of medium was removed from each well for determination of IFN-γ levels, as described below. The plates were then pulsed with 1 µCi/well of tritiated thymidine for a further 18 hours, harvested and tritium uptake determined using a gas scintillation counter. Fractions that resulted in proliferation in both replicates three fold greater than the proliferation observed in cells cultured in medium alone were considered positive.

IFN-γ was measured using an enzyme-linked immunosorbent assay (ELISA). ELISA plates were coated with a mouse monoclonal antibody directed to human IFN-γ (Chemicon) in PBS for four hours at room temperature. Wells were then blocked with PBS containing 5% (W**/**V) non-fat dried milk for 1 hour at room temperature. The plates were then washed six times in PBS/0.2% TWEEN-20 and samples diluted 1:2 in culture medium in the ELISA plates were incubated overnight at room temperature. The plates were again washed and a polyclonal rabbit anti-human IFN-γ serum diluted 1:3000 in PBS/10% normal goat serum was added to each well. The plates were then incubated for two hours at room temperature, washed and horseradish peroxidase-coupled anti-rabbit IgG (Jackson Labs.) was added at a 1:2000 dilution in PBS/5% non-fat dried milk. After a further two hour incubation at room temperature, the plates were washed and TMB substrate added. The reaction was stopped after 20 min with 1 N sulfuric acid. Optical density was determined at 450 nm using 570 nm as a reference wavelength. Fractions that resulted in both replicates giving an OD two fold greater than the mean OD from cells cultured in medium alone, plus 3 standard deviations, were considered positive.

For sequencing, the polypeptides were individually dried onto Biobrene™ (Perkin Elmer/Applied BioSystems Division, Foster City, CA) treated glass fiber filters. The filters with polypeptide were loaded onto a Perkin Elmer/Applied BioSystems Division Procise 492 protein sequencer. The polypeptides were sequenced from the amino terminal and using traditional Edman chemistry. The amino acid sequence was determined for each polypeptide by comparing the retention time of the PTH amino acid derivative to the appropriate PTH derivative standards.

Using the procedure described above, antigens having the following N-terminal sequences were isolated:
(a) Asp-Pro-Val-Asp-Ala-Val-Ile-Asn-Thr-Thr-Xaa-Asn-Tyr-Gly-Gln-Val-Val-Ala-Ala-Leu (SEQ ID No. 54);
(b) Ala-Val-Glu-Ser-Gly-Met-Leu-Ala-Leu-Gly-Thr-Pro-Ala-Pro-Ser (SEQ ID No. 55);
(c) Ala-Ala-Met-Lys-Pro-Arg-Thr-Gly-Asp-Gly-Pro-Leu-Glu-Ala-Ala-Lys-Glu-Gly-Arg (SEQ ID No. 56);
(d) Tyr-Tyr-Trp-Cys-Pro-Gly-Gln-Pro-Phe-Asp-Pro-Ala-Trp-Gly-Pro (SEQ ID No. 57);
(e) Asp-Ile-Gly-Ser-Glu-Ser-Thr-Glu-Asp-Gln-Gln-Xaa-Ala-Val (SEQ ID No. 58);
(f) Ala-Glu-Glu-Ser-Ile-Ser-Thr-Xaa-Glu-Xaa-Ile-Val-Pro (SEQ ID No. 59);
(g) Asp-Pro-Glu-Pro-Ala-Pro-Pro-Val-Pro-Thr-Ala-Ala-Ala-Ala-Pro-Pro-Ala (SEQ ID No. 60); and
(h) Ala-Pro-Lys-Thr-Tyr-Xaa-Glu-Glu-Leu-Lys-Gly-Thr-Asp-Thr-Gly (SEQ ID No. 61);
wherein Xaa may be any amino acid.

An additional antigen was isolated employing a microbore HPLC purification step in addition to the procedure described above. Specifically, 20 µl of a fraction comprising a mixture of antigens from the chromatographic purification step previously described, was purified on an Aquapore C18 column (Perkin Elmer/Applied Biosystems Division, Foster City, CA) with a 7 micron pore size, column size 1 mm x 100 mm, in a Perkin Elmer/Applied Biosystems Division Model 172 HPLC. Fractions were eluted from the column with a linear gradient of l%/minute of acetonitrile (containing 0.05% TFA) in water (0.05% TFA) at a flow rate of 80 µl/minute. The eluent was monitored at 250 nm. The original fraction was separated into 4 major peaks plus other smaller components and a polypeptide was obtained which was shown to have a molecular weight of 12.054 Kd (by mass spectrometry) and the following N-terminal sequence:
(i) Asp-Pro-Ala-Ser-Ala-Pro-Asp-Val-Pro-Thr-Ala-Ala-Gln-Gln-Thr-Ser-Leu-Leu-Asn-Asn-Leu-Ala-Asp-Pro-Asp-Val-Ser-Phe-Ala-Asp (SEQ ID No. 62).
This polypeptide was shown to induce proliferation and IFN-γ production in PBMC preparations using the assays described above.

Additional soluble antigens were isolated from *M. tuberculosis* culture filtrate as follows. *M. tuberculosis* culture filtrate was prepared as described above. Following dialysis against Bis-Tris propane buffer, at pH 5.5, fractionation was performed using anion exchange chromatography on a Poros QE column 4.6 x 100 mm (Perseptive Biosystems) equilibrated in Bis-Tris propane buffer pH 5.5. Polypeptides were eluted with a linear 0-1.5 M NaCl gradient in the above buffer system at a flow rate of 10 ml/min. The column eluent was monitored at a wavelength of 214 nm.

The fractions eluting from the ion exchange column were pooled and subjected to reverse phase chromatography using a Poros R2 column 4.6 x 100 mm (Perseptive Biosystems). Polypeptides were eluted from the column with a linear gradient from 0-100% acetonitrile (0.1% TFA) at a flow rate of 5 ml/mi. The eluent was monitored at 214 nm.

Fractions containing the eluted polypeptides were lyophilized and resuspended in 80 µl of aqueous 0.1 % TFA and further subjected to reverse phase chromatography on a Vydac C4 column 4.6 x 150 mm (Western Analytical, Temecula, CA) with a linear gradient of 0-100% acetonitrile (0.1% TFA) at a flow rate of 2 ml/min. Eluent was monitored at 214 nm.

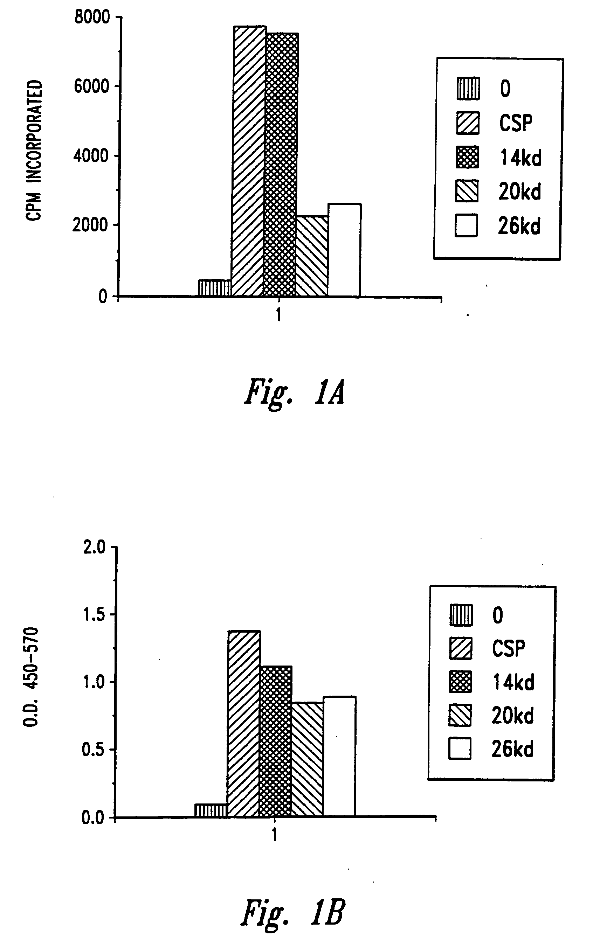

The fraction with biological activity was separated into one major peak plus other smaller components. Western blot of this peak onto PVDF membrane revealed three major bands of molecular weights 14 Kd, 20 Kd and 26 Kd. These polypeptides were determined to have the following N-terminal sequences, respectively:
(j) Xaa-Asp-Ser-Glu-Lys-Ser-Ala-Thr-Ile-Lys-Val-Thr-Asp-Ala-Ser; (SEQ ID No. 129)
(k) Ala-Gly-Asp-Thr-Xaa-Ile-Tyr-Ile-Val-Gly-Asn-Leu-Thr-Ala-Asp: (SEQ ID No. 130) and
(l) Ala-Pro-Glu-Ser-Gly-Ala-Gly-Leu-Gly-Gly-Thr-Val-Gln-Ala-Gly; (SEQ ID No. 131), wherein Xaa may be any amino acid.
Using the assays described above, these polypeptides were shown to induce proliferation and IFN-γ production in PBMC preparations. Figs. 1A and B show the results of such assays using PBMC preparations from a first and a second donor, respectively.

DNA sequences that encode the antigens designated as (a), (c), (d) and (g) above were obtained by screening a *M. tuberculosis* genomic library using ³²p end labeled degenerate oligonucleotides corresponding to the N-terminal sequence and containing *M. tuberculosis* codon bias. The screen performed using a probe corresponding to antigen (a) above identified a clone having the sequence provided in SEQ ID No. 96. The polypeptide encoded by SEQ ID No. 96 is provided in SEQ ID No. 97. The screen performed using a probe corresponding to antigen (g) above identified a clone having the sequence provided in SEQ ID No. 52. The polypeptide encoded by SEQ ID No. 52 is provided in SEQ ID No. 53. The screen performed using a probe corresponding to antigen (d) above identified a clone having the sequence provided in SEQ ID No. 24, and the screen performed with a probe corresponding to antigen (c) identified a clone having the sequence provided in SEQ ID No. 25.

The above amino acid sequences were compared to known amino acid sequences in the gene bank using the DNA STAR system. The database searched contains some 173,000 proteins and is a combination of the Swiss, PIR databases along with translated protein sequences (Version 87). No significant homologies to the amino acid sequences for antigens (a)-(h) and (1) were detected.

The amino acid sequence for antigen (i) was found to be homologous to a sequence from *M. leprae.* The full length *M. leprae* sequence was amplified from genomic DNA using the sequence obtained from GENBANK. This sequence was then used to screen an *M. tuberculosis* library and a full length copy of the *M. tuberculosis* homologue was obtained (SEQ ID No. 94).

The amino acid sequence for antigen (j) was found to be homologous to a known *M. tuberculosis* protein translated from a DNA sequence. To the best of the inventors' knowledge, this protein has not been previously shown to possess T-cell stimulatory activity. The amino acid sequence for antigen (k) was found to be related to a sequence from *M. leprae.*

In the proliferation and IFN-γ assays described above, using three PPD positive donors, the results for representative antigens provided above are presented in Table 1:

**TABLE 1**

| RESULTS OF PBMC PROLIFERATION AND IFN-γ ASSAYS | | |
|---|---|---|
| Sequence | Proliferation | IFN-γ |
| (a) | + | - |
| (c) | +++ | +++ |
| (d) | ++ | ++ |
| (g) | +++ | +++ |
| (h) | +++ | +++ |

In Table 1, responses that gave a stimulation index (SI) of between 2 and 4 (compared to cells cultured in medium alone) were scored as +, as SI of 4-8 or 2-4 at a concentration of 1 µg or less was scored as ++ and an SI of greater than 8 was scored as +++. The antigen of sequence (i) was found to have a high SI (+++) for one donor and lower SI (++ and +) for the two other donors in both proliferation and IFN-γ assays. These results indicate that these antigens are capable of inducing proliferation and/or interferon-γ production.

### EXAMPLE 2

### USE OF PATIENT SERA TO ISOLATE M. TUBERCULOSIS ANTIGENS

This example illustrates the isolation of antigens from *M. tuberculosis* lysate by screening with serum from *M. tuberculosis*-infected individuals.

Dessicated *M. tuberculosis* H37Ra (Difco Laboratories) was added to a 2% NP40 solution, and alternately homogenized and sonicated three times. The resulting suspension was centrifuged at 13,000 rpm in microfuge tubes and the supernatant put through a 0.2 micron syringe filter. The filtrate was bound to Macro Prep DEAE beads (BioRad, Hercules, CA). The beads were extensively washed with 20 mM Tris pH 7.5 and bound proteins eluted with 1M NaCl. The NaCl elute was dialyzed overnight against 10 mM Tris, pH 7.5. Dialyzed solution was treated with DNase and RNase at 0.05 mg/ml for 30 min. at room temperature and then with a-D-mannosidase, 0.5 U/mg at pH 4.5 for 3-4 hours at room temperature. After returning to pH 7.5, the material was fractionated via FPLC over a Bio Scale-Q-20 column (BioRad). Fractions were combined into nine pools, concentrated in a Centriprep 10 (Amicon, Beverley, MA) and screened by Western blot for serological activity using a serum pool from *M. tuberculosis*-infected patients which was not immunoreactive with other antigens of the present invention.

The most reactive fraction was run in SDS-PAGE and transferred to PVDF. A band at approximately 85 Kd was cut out yielding the sequence:
(m) Xaa-Tyr-Ile-Ala-Tyr-Xaa-Thr-Thr-Ala-Gly-Ile-Val-Pro-Gly-Lys-Ile-Asn-Val-His-Leu-Val; (SEQ ID No. 132), wherein Xaa may be any amino acid.

Comparison of this sequence with those in the gene bank as described above, revealed no significant homologies to known sequences.

### EXAMPLE 3

### PREPARATION OF DNA SEQUENCES ENCODING M. TUBERCULOSIS ANTIGENS

This example illustrates the preparation of DNA sequences encoding *M. tuberculosis* antigens by screening a *M. tuberculosis* expression library with sera obtained from patients infected with *M. tuberculosis,* or with anti-sera raised against *M. tuberculosis* antigens.

### A. PREPARATION OF M. TUBERCULOSIS SOLUBLE ANTIGENS USING RaBBIT ANTI-SERA

Genomic DNA was isolated from the *M. tuberculosis* strain H37Ra. The DNA was randomly sheared and used to construct an expression library using the Lambda ZAP expression system (Stratagene, La Jolla, CA). Rabbit anti-sera was generated against secretory proteins of the *M. tuberculosis* strains H37Ra, H37Rv and Erdman by immunizing a rabbit with concentrated supernatant of the *M. tuberculosis* cultures. Specifically, the rabbit was first immunized subcutaneously with 200 µg of protein antigen in a total volume of 2 ml containing 100 µg muramyl dipeptide (Calbiochem, La Jolla, CA) and 1 ml of incomplete Freund's adjuvant. Four weeks later the rabbit was boosted subcutaneously with 100 µg antigen in incomplete Freund's adjuvant. Finally, the rabbit was immunized intravenously four weeks later with 50 µg protein antigen. The anti-sera were used to screen the expression library as described in Sambrook et al., Molecular Cloning: A Laboratory Manual, Cold Spring Harbor Laboratories, Cold Spring Harbor, NY, 1989. Bacteriophage plaques expressing immunoreactive antigens were purified. Phagemid from the plaques was rescued and the nucleotide sequences of the *M. tuberculosis* clones deduced.

Thirty two clones were purified. Of these, 25 represent sequences that have not been previously identified in *M. tuberculosis.* Proteins were induced by IPTG and purified by gel elution, as described in Skeiky et al., J. Exp. Med 181:1527-1537, 1995. Representative partial sequences of DNA molecules identified in this screen are provided in SEQ ID Nos. 1-25. The corresponding predicted amino acid sequences are shown in SEQ ID Nos. 64-88.

On comparison of these sequences with known sequences in the gene bank using the databases described above, it was found that the clones referred to hereinafter as TbRA2A, TbRAl6, TbRA18, and TbRA29 (SEQ ID Nos. 77, 69, 71, 76) show some homology to sequences previously identified in *Mycobacterium leprae* but not in *M. tuberculosis.* TbRA11, TbRA26, TbRA28 and TbDPEP (SEQ ID Nos. 66, 74, 75, 53) have been previously identified in *M. tuberculosis.* No significant homologies were found to TbRA1, TbRA3, TbRA4, TbRA9, TbRA10, TbRA13, TbRA17, TbRAl9, TbRA29, TbRA32, TbRA36 and the overlapping clones TbRA35 and TbRA12 (SEQ ID Nos. 64, 78, 82, 83, 65, 68, 76, 72, 76, 79, 81, 80, 67, respectively). The clone TbRa24 is overlapping with clone TbRa29.

### B. USE OF PATIENT SERA TO IDENTIFY DNA SEOUENCES ENCODING M. TUBERCULOSIS ANTIGENS

The genomic DNA library described above, and an additional H37Rv library, were screened using pools of sera obtained from patients with active tuberculosis. To prepare the H37Rv library, *M. tuberculosis* strain H37Rv genomic DNA was isolated, subjected to partial Sau3A digestion and used to construct an expression library using the Lambda Zap expression system (Stratagene, La Jolla, Ca). Three different pools of sera, each containing sera obtained from three individuals with active pulmonary or pleural disease, were used in the expression screening. The pools were designated TbL, TbM and TbH, referring to relative reactivity with H37Ra lysate (*i.e.,* TbL = low reactivity, TbM = medium reactivity and TbH = high reactivity) in both ELISA and immunoblot format. A fourth pool of sera from seven patients with active pulmonary tuberculosis was also employed. All of the sera lacked increased reactivity with the recombinant 38 kD *M. tuberculosis* H37Ra phosphate-binding protein.

All pools were pre-adsorbed with *E. coli* lysate and used to screen the H37Ra and H37Rv expression libraries, as described in Sambrook et al., Molecular Cloning: A Laboratory Manual, Cold Spring Harbor Laboratories, Cold Spring Harbor, NY, 1989. Bacteriophage plaques expressing immunoreactive antigens were purified. Phagemid from the plaques was rescued and the nucleotide sequences of the *M. tuberculosis* clones deduced.

Thirty two clones were purified. Of these, 31 represented sequences that had not been previously identified in human *M. tuberculosis.* Representative sequences of the DNA molecules identified are provided in SEQ ID NOS.: 26-51 and 100. Of these, TbH-8 and TbH-8-2 (SEQ. ID NO. 100) are non-contiguous DNA sequences from the same clone, and TbH-4 (SEQ. ID NO. 43) and TbH-4-FWD (SEQ. ID NO. 44) are non-contiguous sequences from the same clone. Amino acid sequences for the antigens hereinafter identified as Tb38-l, TbH-4, TbH-8, TbH-9, and TbH-12 are shown in SEQ ID NOS.: 89-93. Comparison of these sequences with known sequences in the gene bank using the databases identified above revealed no significant homologies to TbH-4, TbH-8, TbH-9 and TbM-3, although weak homologies were found to TbH-9. TbH-12 was found to be homologous to a 34 kD antigenic protein previously identified in *M. paratuberculosis* (Acc. No. S28515). Tb38-1 was found to be located 34 base pairs upstream of the open reading frame for the antigen ESAT-6 previously identified in *M. bovis* (Acc. No. U34848) and in *M. tuberculosis* (Sorensen et al., Infec. Immun 63:1710-1717, 1995).

Probes derived from Tb38-1 and TbH-9, both isolated from an H37Ra library, were used to identify clones in an H37Rv library. Tb38-1 hybridized to Tb38-1F2, Tb38-1F3, Tb38-1F5 and Tb38-1F6 (SEQ. ID NOS. 107, 108, 111, 113, and 114). (SEQ ID NOS. 107 and 108 are non-contiguous sequences from clone Tb38-1F2.) Two open reading frames were deduced in Tb38-IF2; one corresponds to Tb37FL (SEQ. ID. NO. 109), the second, a partial sequence, may be the homologue of Tb38-1 and is called Tb38-IN (SEQ. ID NO. 110). The deduced amino acid sequence of Tb38-IF3 is presented in SEQ. ID. NO. 112. A TbH-9 probe identified three clones in the H37Rv library: TbH-9-FL (SEQ. ID NO. 101), which may be the homologue of TbH-9 (R37Ra), TbH-9-1 (SEQ. ID NO. 103), and TbH-9-4 (SEQ. ID NO. 105), all of which are highly related sequences to TbH-9. The deduced amino acid sequences for these three clones are presented in SEQ ID NOS. 102, 104 and 106.

### EXAMPLE 4

### PURIFICATION AND CHARACTERIZATION OF A POLYPEPTIDE FROM TUBERCULIN PURIFIED PROTEIN DERIVATIVE

An *M. tuberculosis* polypeptide was isolated from tuberculin purified protein derivative (PPD) as follows.

PPD was prepared as published with some modification (Seibert, F. et al., Tuberculin purified protein derivative. Preparation and analyses of a large quantity for standard. The American Review of Tuberculosis 44:9-25, 1941). *M. tuberculosis* Rv strain was grown for 6 weeks in synthetic medium in roller bottles at 37 °C. Bottles containing the bacterial growth were then heated to 100°C in water vapor for 3 hours. Cultures were sterile filtered using a 0.22 µ filter and the liquid phase was concentrated 20 times using a 3 kD cut-off membrane. Proteins were precipitated once with 50% ammonium sulfate solution and eight times with 25% ammonium sulfate solution. The resulting proteins (PPD) were fractionated by reverse phase liquid chromatography (RP-HPLC) using a C18 column (7.8 x 300 mM; Waters, Milford, MA) in a Biocad HPLC system (Perseptive Biosystems, Framingham, MA). Fractions were eluted from the column with a linear gradient from 0-100% buffer (0.1% TFA in acetonitrile). The flow rate was 10 ml/minute and eluent was monitored at 214 nm and 280 nm.

Six fractions were collected, dried, suspended in PBS and tested individually in *M. tuberculosis*-infected guinea pigs for induction of delayed type hypersensitivity (DTH) reaction. One fraction was found to induce a strong DTH reaction and was subsequently fractionated furtherby RP-HPLC on a microbore Vydac C18 column (Cat. No. 218TP5115) in a Perkin Elmer/Applied Biosystems Division Model 172 HPLC. Fractions were eluted with a linear gradient from 5-100% buffer (0.05% TFA in acetonitrile) with a flow rate of 80 µl/minute. Eluent was monitored at 215 nm. Eight fractions were collected and tested for induction of DTH in *M. tuberculosis*-infected guinea pigs. One fraction was found to induce strong DTH of about 16 mm induration. The other fractions did not induce detectable DTH. The positive fraction was submitted to SDS-PAGE gel electrophoresis and found to contain a single protein band of approximately 12 kD molecular weight.

This polypeptide, herein after referred to as DPPD, was sequenced from the amino terminal using a Perkin Elmer/Applied Biosystems Division Procise 492 protein sequencer as described above and found to have the N-terminal sequence shown in SEQ ID No.: 124. Comparison of this sequence with known sequences in the gene bank as described above revealed no known homologies. Four cyanogen bromide fragments of DPPD were isolated and found to have the sequences shown in SEQ ID Nos.: 125-128.

### EXAMPLE 5

### SYNTHESIS OF SYNTHETIC POLYPEPTIDES

Polypeptides may be synthesized on a Millipore 9050 peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugation or labeling of the peptide. Cleavage of the peptides from the solid support may be carried out using the following cleavage mixture: trifluoroacetic acid:ethanedithiolahioanisole:water:phenol (40:1:2:2:3). After cleaving for 2 hours, the peptides may be precipitated in cold methyl-t-butyl-ether. The peptide pellets may then be dissolved in water containing 0.1% trifluoroacetic acid (TFA) and lyophilized prior to purification by C 18 reverse phase HPLC. A gradient of 0-60% acetonitrile (containing 0.1% TFA) in water (containing 0.1% TFA) may be used to elute the peptides. Following lyophilization of the pure fractions, the peptides may be characterized using electrospray mass spectrometry and by amino acid analysis.

This procedure was used to synthesize a TbM-1 peptide that contains one and a half repeats of a TbM-1 sequence. The TbM-1 peptide has the sequence GCGDRSGGNLDQIRLRRDRSGGNL (SEQ ID No. 63).

### EXAMPLE 6

### USE OF REPRESENTATIVE ANTIGENS FOR SERODIAGNOSIS OF TUBERCULOSIS

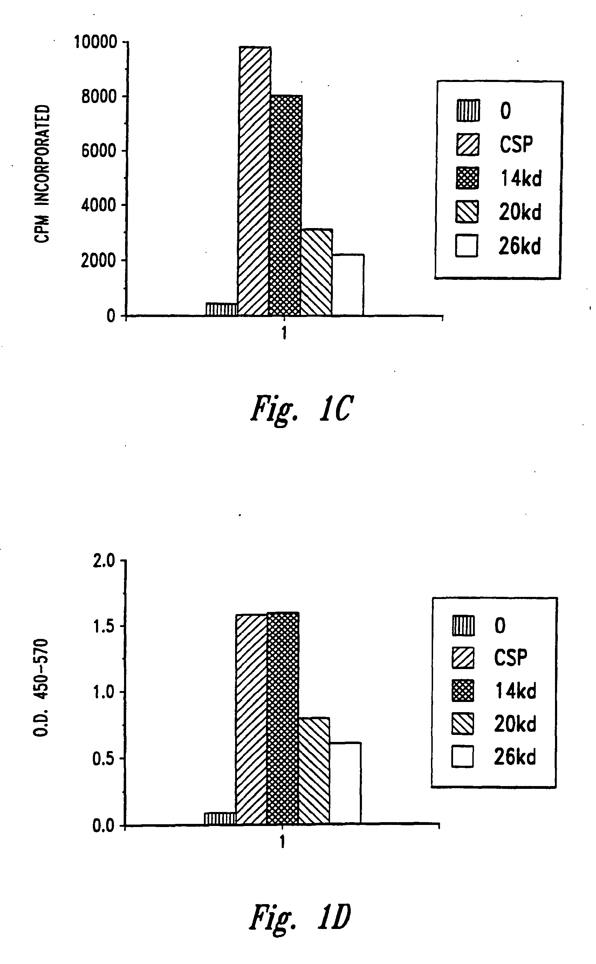

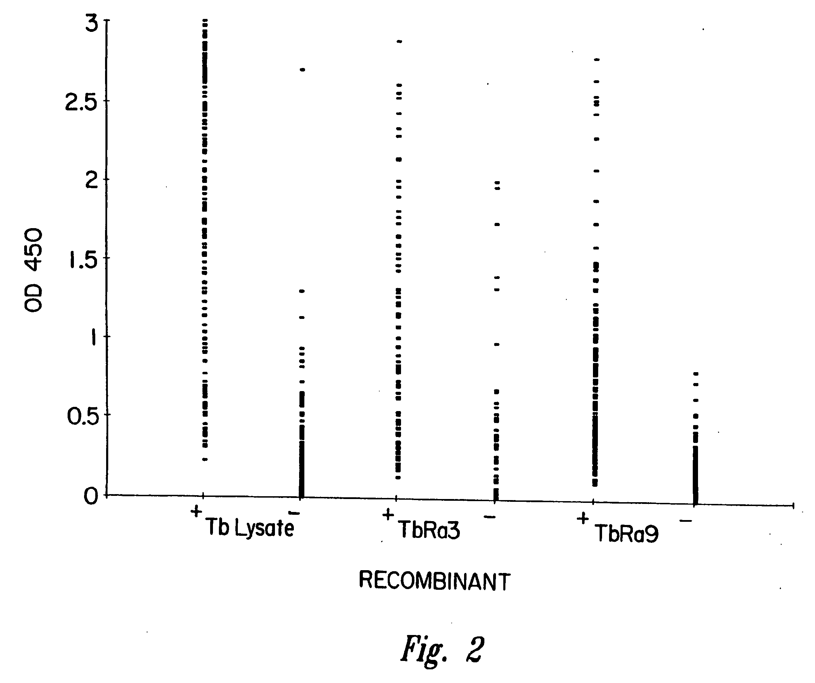

This Example illustrates the diagnostic properties of several representative antigens. Figures 1 and 2 present the reactivity of representative antigens with sera from *M. tuberculosis*-infected and uninfected individuals, as compared to the reactivity of bacterial lysate and the 38 kD antigen.

Assays were performed in 96-well plates were coated with 200 ng antigen diluted to 50 µL in carbonate coating buffer, pH 9.6. The wells were coated overnight at 4°C (or 2 hours at 37°C). The plate contents were then removed and the wells were blocked for 2 hours with 200 µL of PBS/1% BSA. After the blocking step, the wells were washed five times with PBS/0.1% Tween 20™. 50 µL sera, diluted 1:100 in PBS/0.1% Tween 20™/0.1% BSA, was then added to each well and incubated for 30 minutes at room temperature. The plates were then washed again five times with PBS/0.1% Tween 20™.

The enzyme conjugate (horseradish peroxidase - Protein A, Zymed, San Francisco, CA) was then diluted 1:10,000 in PBS/0.1% Tween 20™/0.1% BSA, and 50 µL of the diluted conjugate was added to each well and incubated for 30 minutes at room temperature. Following incubation, the wells were washed five times with PBS/0.1% Tween 20™. 100 µL of tetramethylbenzidine peroxidase (TMB) substrate (Kirkegaard and Perry Laboratories, Gaithersburg, MD) was added, undiluted, and incubated for about 15 minutes. The reaction was stopped with the addition of 100 µL of 1 N H₂SO₄ to each well, and the plates were read at 450 nm.

Figure 2 shows the ELISA reactivity of two recombinant antigens isolated using method A in Example 3 (TbRa3 and TbRa9) with sera from *M. tuberculosis* positive and negative patients. The reactivity of these antigens is compared to that of bacterial lysate isolated from *M. tuberculosis* strain H37Ra (Difco, Detroit MI). In both cases, the recombinant antigens differentiated positive from negative sera. Based on cut-off values obtained from receiver-operator curves, TbRa3 detected 56 out of 87 positive sera, and TbRa9 detected 111 out of 165 positive sera.

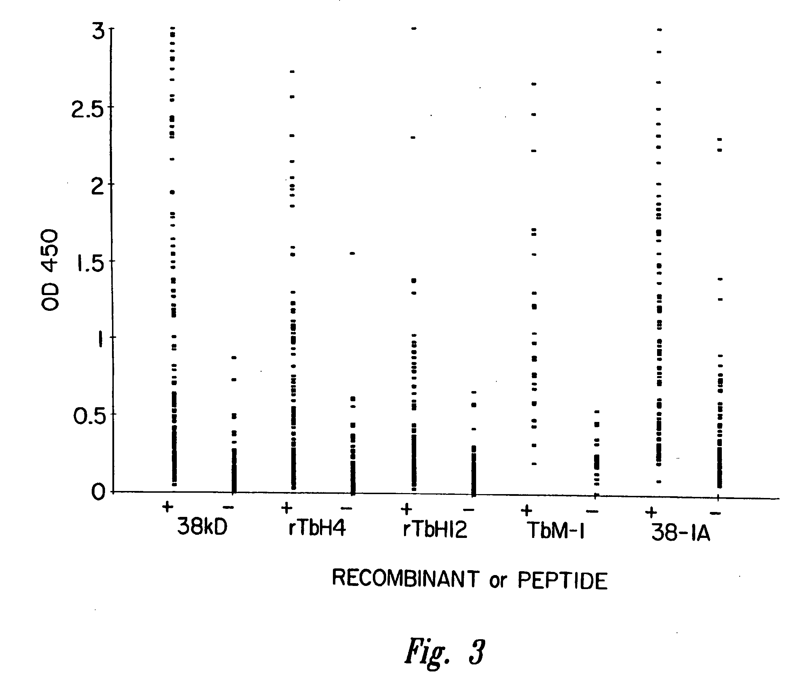

Figure 3 illustrates the ELISA reactivity of representative antigens isolated using method B of Example 3. The reactivity of the recombinant antigens TbH4, TbH12, Tb38-1 and the peptide TbM-1 (as described in Example 4) is compared to that of the 38 kD antigen described by Andersen and Hansen, Infect. Immun. 57:2481-2488, 1989. Again, all of the polypeptides tested differentiated positive from negative sera. Based on cut-off values obtained from receiver-operator curves, TbH4 detected 67 out of 126 positive sera, TbH12 detected 50 out of 125 positive sera, 38-1 detected 61 out of 101 positive sera and the TbM-1 peptide detected 25 out of 30 positive sera.

The reactivity of four antigens (TbRa3, TbRa9, TbH4 and TbH12) with sera from a group of *M. tuberculosis* infected patients with differing reactivity in the acid fast stain of sputum (Smithwick and David, Tubercle 52:226, 1971) was also examined, and compared to the reactivity of *M. tuberculosis* lysate and the 38 kD antigen. The results are presented in Table 2, below:

**TABLE 2**

| REACTIVITY OF ANTIGENS WITH SERA FROM *M. TUBERCULOSIS* PATIENTS | | | | | | | |
|---|---|---|---|---|---|---|---|
| Patient | Acid Fast Sputum | ELISA Values | | | | | |
| | | Lysate | 38kD | TbRa9 | TbH12 | TbH4 | TbRa3 |
| Tb01B93I-2 | ++++ | 1.853 | 0.634 | 0.998 | 1.022 | 1.030 | 1.314 |
| Tb01B93I-19 | ++++ | 2.657 | 2.322 | 0.608 | 0.837 | 1.857 | 2.335 |
| Tb01B93I-8 | +++ | 2.703 | 0.527 | 0.492 | 0.281 | 0.501 | 2.002 |
| Tb01B93I-10 | +++ | 1.665 | 1.301 | 0.685 | 0.216 | 0.448 | 0.458 |
| Tb01B93I-11 | +++ | 2.817 | 0.697 | 0.509 | 0.301 | 0.173 | 2.608 |
| Tb01B93I-15 | +++ | 1.28 | 0.283 | 0.808 | 0.218 | 1.537 | 0.811 |
| Tb01B93I-16 | +++ | 2.908 | >3 | 0.899 | 0.441 | 0.593 | 1.080 |
| Tb01B93I-25 | +++ | 0.395 | 0.131 | 0.335 | 0.211 | 0.107 | 0.948 |
| Tb01B93I-87 | +++ | 2.653 | 2.432 | 2.282 | 0.977 | 1.221 | 0.857 |
| Tb01B93I-89 | +++ | 1.912 | 2.370 | 2.436 | 0.876 | 0.520 | 0.952 |
| Tb01B94I-108 | +++ | 1.639 | 0.341 | 0.797 | 0.368 | 0.654 | 0.798 |
| Tb01B94I-201 | +++ | 1.721 | 0.419 | 0.661 | 0.137 | 0.064 | 0.692 |
| Tb01B93I-88 | ++ | 1.939 | 1.269 | 2.519 | 1.381 | 0.214 | 0.530 |
| Tb01B93I-92 | ++ | 2.355 | 2.329 | 2.78 | 0.685 | 0.997 | 2.527 |
| Tb01B94I-109 | ++ | 0.993 | 0.620 | 0.574 | 0.441 | 0.5 | 2.558 |
| Tb01B94I-210 | ++ | 2.777 | >3 | 0.393 | 0.367 | 1.004 | 1.315 |
| Tb01B94I-224 | ++ | 2.913 | 0.476 | 0.251 | 1.297 | 1.990 | 0.256 |
| Tb01B93I-9 | + | 2.649 | 0.278 | 0.210 | 0.140 | 0.181 | 1.586 |
| TbO1B93I-14 | + | >3 | 1.538 | 0.282 | 0.291 | 0.549 | 2.880 |
| Tb01B93I-21 | + | 2.645 | 0.739 | 2.499 | 0.783 | 0.536 | 1.770 |
| Tb01B93I-22 | + | 0.714 | 0.451 | 2.082 | 0.285 | 0.269 | 1.159 |
| Tb01B93I-31 | + | 0.956 | 0.490 | 1.019 | 0.812 | 0.176 | 1.293 |
| TbO1B93I-32 | - | 2.261 | 0.786 | 0.668 | 0.273 | 0.535 | 0.405 |
| Tb01B93I-52 | - | 0.658 | 0.114 | 0.434 | 0.330 | 0.273 | 1.140 |
| Tb01B93I-99 | - | 2.118 | 0.584 | 1.62 | 0.119 | 0.977 | 0.729 |
| Tb01B94I-130 | - | 1.349 | 0.224 | 0.86 | 0.282 | 0.383 | 2.146 |
| Th01B94I-131 | - | 0.685 | 0.324 | 1.173 | 0.059 | 0.118 | 1.431 |
| AT4-0070 | Normal | 0.072 | 0.043 | 0.092 | 0.071 | 0.040 | 0.039 |
| AT4-0105 | Normal | 0.397 | 0.121 | 0.118 | 0.103 | 0.078 | 0.390 |
| 3/15/94-1 | Normal | 0.227 | 0.064 | 0.098 | 0.026 | 0.001 | 0.228 |
| 4/15/93-2 | Normal | 0.114 | 0.240 | 0.071 | 0.034 | 0.041 | 0.264 |
| 5/26/94-4 | Normal | 0.089 | 0.259 | 0.096 | 0.046 | 0.008 | 0.053 |
| 5/26/94-3 | Normal | 0.139 | 0.093 | 0.085 | 0.019 | 0.067 | 0.01 |

Based on cut-off values obtained from receiver-operator curves, TbRa3 detected 23 out of 27 positive sera, TbRa9 detected 22 out of 27, TbH4 detected 18 out of 27 and TbH12 detected 15 out of 27. If used in combination, these four antigens would have a theoretical sensitivity of 27 out of 27, indicating that these antigens should complement each other in the serological detection of *M. tuberculosis* infection. In addition, several of the recombinant antigens detected positive sera that were not detected using the 38 kD antigen, indicating that these antigens may be complementary to the 38 kD antigen.

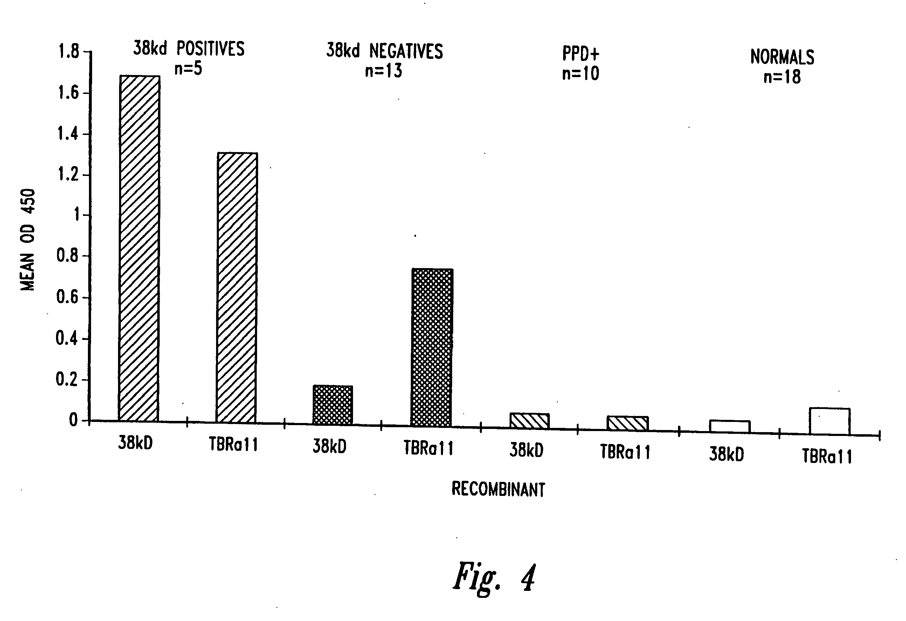

The reactivity of the recombinant antigen TbRa11 with sera from *M. tuberculosis* patients shown to be negative for the 38 kD antigen, as well as with sera from PPD positive and normal donors, was determined by ELISA as described above. The results are shown in Figure 4 which indicates that ThRa11, while being negative with sera from PPD positive and normal donors, detected sera that were negative with the 38 kD antigen. Of the thirteen 38 kD negative sera tested, nine were positive with TbRa11, indicating that this antigen may be reacting with a sub-group of 38 kD antigen negative sera. In contrast, in a group of 38 kD positive sera where TbRa11 was reactive, the mean OD 450 for TbRa11 was lower than that for the 38 kD antigen. The data indicate an inverse relationship between the presence of TbRa11 activity and 38 kD positivity.

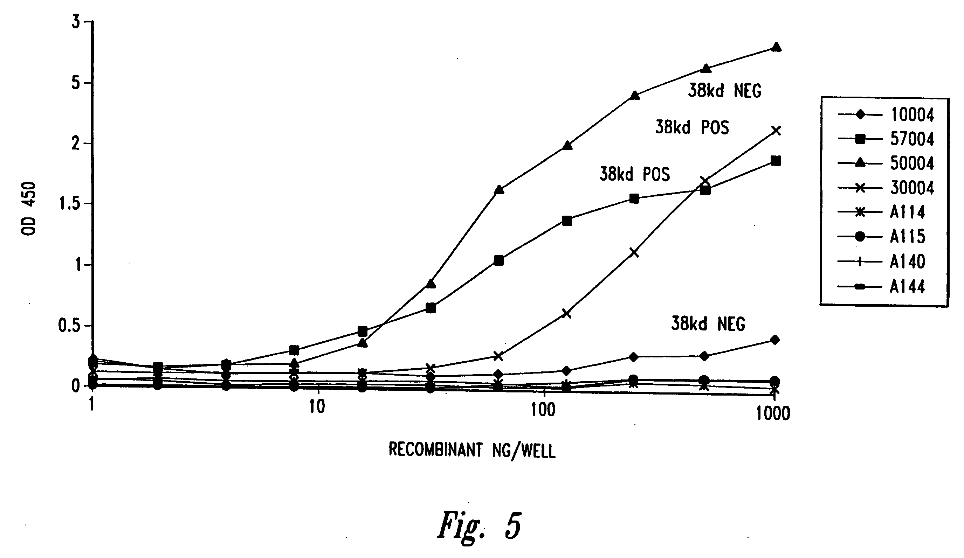

The antigen TbRa2A was tested in an indirect ELISA using initially 50 µl of serum at 1:100 dilution for 30 minutes at room temperature followed by washing in PBS Tween and incubating for 30 minutes with biotinylated Protein A (Zymed, San Francisco, CA) at a 1:10,000 dilution. Following washing, 50 µl of streptavidin-horseradish peroxidase (Zymed) at 1:10,000 dilution was added and the mixture incubated for 30 minutes. After washing, the assay was developed with TMB substrate as described above. The reactivity of TbRa2A with sera from *M. tuberculosis* patients and normal donors in shown in Table 3. The mean value for reactivity of TbRa2A with sera from *M. tuberculosis* patients was 0.444 with a standard deviation of 0.309. The mean for reactivity with sera from normal donors was 0.109 with a standard deviation of 0.029. Testing of 38 kD negative sera (Figure 5) also indicated that the TbRa2A antigen was capable of detecting sera in this category.

**TABLE 3**

| REACTIVITY OF TBRA2A WITH SERA FROM *M. TUBERCULOSIS* PATIENTS AND FROM NORMAL DONORS | | |
|---|---|---|
| Serum ID | Status | OD 450 |
| Tb85 | TB | 0.680 |
| Tb86 | TB | 0.450 |
| Tb87 | TB | 0.263 |
| Tb88 | TB | 0.275 |
| Tb89 | TB | 0.403 |
| Tb91 | TB | 0.393 |
| Tb92 | TB | 0.401 |
| Tb93 | TB | 0.232 |
| Tb94 | TB | 0.333 |
| Tb95 | TB | 0.435 |
| Tb96 | TB | 0.284 |
| Tb97 | TB | 0.320 |
| Tb99 | TB | 0.328 |
| Tb100 | TB | 0.817 |
| Tb101 | TB | 0.607 |
| Tb102 | TB | 0.191 |
| Tb103 | TB | 0.228 |
| Tb107 | TB | 0.324 |
| Tb109 | TB | 1.572 |
| Tb112 | TB | 0.338 |
| DL4-0176 | Normal | 0.036 |
| AT4-0043 | Normal | 0.126 |
| AT4-0044 | Normal | 0.130 |
| AT4-0052 | Normal | 0.135 |
| AT4-0053 | Normal | 0.133 |
| AT4-0062 | Normal | 0.128 |
| AT4-0070 | Normal | 0.088 |
| AT4-0091 | Normal | 0.108 |
| AT4-0100 | Normal | 0.106 |
| AT4-0105 | Normal | 0.108 |
| AT4-0109 | Normal | 0.105 |

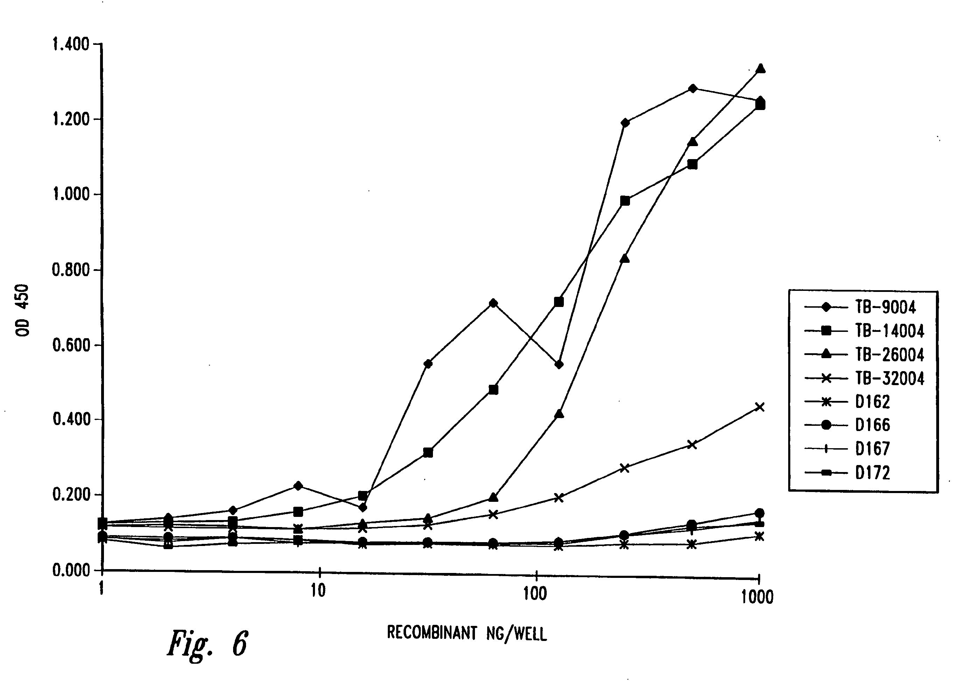

The reactivity of the recombinant antigen (g) (SEQ ID No. 60) with sera from *M. tuberculosis* patients and normal donors was determined by ELISA as described above. Figure 6 shows the results of the titration of antigen (g) with four *M. tuberculosis* positive sera that were all reactive with the 38 kD antigen and with four donor sera. All four positive sera were reactive with antigen (g).

## Claims

1. A method for detecting sensitisation to a mycobacterial antigen in a subject comprising:
(a) contacting a biological sample from said subject with a polypeptide comprising an antigenic portion of SEQ ID No: 89; and
(b) detecting in the biological sample the presence of antibodies that bind to the polypeptide.

2. The method according to claim 1, for detecting *M. tuberculosis* infection in a subject comprising:
(a) contacting a biological sample from said subject with a polypeptide comprising an antigenic portion of SEQ ID No: 89; and
(b) detecting in the biological sample the presence of antibodies that bind to the polypeptide.

3. The method of claim 2 wherein step (a) additionally comprises contacting the biological sample with a 38 kD *M. tuberculosis* antigen and step (b) additionally comprises detecting in the sample the presence of antibodies that bind to the 38 kD *M. tuberculosis* antigen.

4. The method of either claim 1or 2 wherein the polypeptide is bound to a solid support.

5. The method of claim 4 wherein the solid support comprises nitrocellulose, latex or a plastic material.

6. The method of claim 2 wherein the biological sample is selected from the group consisting of whole blood, serum, plasma, saliva, cerebrospinal fluid and urine.

7. The method of claim 6 wherein the biological sample is whole blood or serum.

8. A diagnostic kit comprising:
(a) a polypeptide comprising an antigenic portion of SEQ ID No: 89; and
(b) a detection reagent.

9. The diagnostic kit of claim 8 wherein the polypeptide is immobilised on a solid support.

10. The kit of claim 9 wherein the solid support comprises nitrocellulose, latex or a plastic material.

11. The kit of claim 8 wherein the detection reagent comprises a reporter group conjugated to a binding agent.

12. The kit of claim 11 wherein the binding agent is selected from the group consisting of anti-immunoglobulins, Protein G, Protein A and lectins.

13. The kit of claim 11 wherein the reporter group is selected from the group consisting of radioisotopes, fluorescent groups, luminescent groups, enzymes, biotin and dye particles.

## Patentansprüche

1. Verfahren zum Detektieren der Sensibilisierung gegenüber einem mycobakteriellen Antigen in einem Subjekt, umfassend:
(a) Inkontaktbringen einer biologischen Probe aus dem Subjekt mit einem Polypeptid, das einen Antigen-Abschnitt der SEQ ID NR: 89 umfasst; und
(b) Detektieren der Anwesenheit von Antikörpern in der biologischen Probe, die an das Polypeptid binden.

2. Verfahren gemäß Anspruch 1, zum Detektieren einer *M. tuberculosis*-Infektion in einem Subjekt, umfassend:
(a) Inkontaktbringen einer biologischen Probe aus dem Subjekt mit einem Polypeptid, das einen Antigen-Abschnitt der SEQ ID NR: 89 umfasst; und
(b) Detektieren der Anwesenheit von Antikörpern in der biologischen Probe, die an das Polypeptid binden.

3. Verfahren gemäß Anspruch 2, worin Schritt (a) weiterhin das Inkontaktbringen der biologischen Probe mit einem 38 kD *M. tuberculosis*-Antigen umfasst, und Schritt (b) weiterhin das Detektieren der Anwesenheit von Antikörpern in der Probe, die an das 38 kD *M. tuberculosis*-Antigen binden, umfasst.

4. Verfahren gemäß Anspruch 1 oder 2, worin das Polypeptid an einen festen Träger gebunden ist.

5. Verfahren gemäß Anspruch 4, worin der feste Träger Nitrocellulose, Latex oder ein Kunststoffmaterial umfasst.

6. Verfahren gemäß Anspruch 2, worin die biologische Probe aus der Gruppe bestehend aus vollem Blut, Serum, Plasma, Speichel, cerebrospinaler Flüssigkeit und Urin ausgewählt ist.

7. Verfahren gemäß Anspruch 6, worin die biologische Probe volles Blut oder Serum ist.

8. Diagnostisches Kit, umfassend:
(a) ein Polypeptid, das einen Antigen-Abschnitt der SEQ ID NR: 89 umfasst; und
(b) ein Detektionsreagenz.

9. Diagnostisches Kit gemäß Anspruch 8, worin das Polypeptid auf einem festen Träger immobilisiert ist.

10. Kit gemäß Anspruch 9, worin der feste Träger Nitrocellulose, Latex oder ein Kunststoffmaterial umfasst.

11. Kit gemäß Anspruch 8, worin das Detektionsreagenz eine Reportergruppe, die mit einem bindenden Mittel konjugiert ist, umfasst.

12. Kit gemäß Anspruch 11, worin das bindende Mittel aus der Gruppe bestehend aus Antiimmunoglobulinen, Protein G, Protein A und Lektinen ausgewählt ist.

13. Kit gemäß Anspruch 11, worin die Reportergruppe aus der Gruppe bestehend aus Radioisotopen, fluoreszierenden Gruppen, lumineszierenden Gruppen, Enzymen, Biotin und Farbstoffteilchen ausgewählt ist.

## Revendications

1. Procédé de détection d'une sensibilisation à un antigène mycobactérien chez un sujet comprenant les étapes consistant à :
(a) mettre en contact un échantillon biologique provenant dudit sujet avec un polypeptide comprenant une partie de l'antigène ayant la SEQ ID No. : 89 ; et
(b) détecter dans l'échantillon biologique la présence d'anticorps se liant au polypeptide.

2. Procédé selon la revendication 1, destiné à détecter une infection par *M. tuberculosis* chez un sujet comprenant les étapes consistant à :
(a) mettre en contact un échantillon biologique provenant dudit sujet avec un polypeptide comprenant une partie de l'antigène ayant la SEQ ID No. : 89 ; et
(b) détecter dans l'échantillon biologique la présence d'anticorps se liant au polypeptide.

3. Procédé selon la revendication 2, dans lequel l'étape (a) comprend en outre l'étape consistant à mettre en contact l'échantillon biologique avec un antigène de 38 kD de *M. tuberculosis,* et l'étape (b) comprend en outre l'étape consistant à détecter dans l'échantillon la présence d'anticorps se liant à l'antigène de 38 kD de *M. tuberculosis.*

4. Procédé selon l'une quelconque des revendications 1 ou 2, dans lequel le polypeptide est lié à un support solide.

5. Procédé selon la revendication 4, dans lequel le support solide comprend de la nitrocellulose, du latex ou une matière plastique.

6. Procédé selon la revendication 2, dans lequel l'échantillon biologique est choisi dans le groupe comprenant du sang total, du sérum, du plasma, de la salive, du liquide céphalorachidien et de l'urine.

7. Procédé selon la revendication 6, dans lequel l'échantillon biologique est du sang total ou du sérum.

8. Kit de diagnostic comprenant :
(a) un polypeptide comprenant une partie de l'antigène ayant la SEQ ID No. : 89 ; et
(b) un réactif de détection.

9. Kit de diagnostic selon la revendication 8, dans lequel le polypeptide est immobilisé sur un support solide.

10. Kit selon la revendication 9, dans lequel le support solide comprend de la nitrocellulose, du latex ou une matière plastique.

11. Kit selon la revendication 8, dans lequel le réactif de détection comprend un groupe rapporteur conjugué à un agent de liaison.

12. Kit selon la revendication 11, dans lequel l'agent de liaison est choisi dans le groupe comprenant des anti-immunoglobines, la protéine G, la protéine A et des lectines.

13. Kit selon la revendication 11, dans lequel le groupe rapporteur est choisi dans le groupe comprenant des radio-isotopes, des groupes fluorescents, des groupes luminescents, des enzymes, de la biotine et des particules colorantes.


### SEQUENCE LISTING

<110> Corixa Corporation
<120> Compounds and methods for diagnosis of tuberculosis
<130> CRX-P417
<150> 08/523,435
   <151> 1995-09-01
<150> 08/532,136
   <151> 1995-09-22
<150> 08/620,280
   <151> 1996-03-22
<150> 08/658,800
   <151> 1996-06-05
<150> 08/680,573
   <151> 1996-07-12
<160> 132
<170> PatentIn version 3.5
<210> 1
   <211> 766
   <212> DNA
   <213> Mycobacterium tuberculosis
<220>
   <221> misc_feature <222> (565)..(565)
   <223> Unknown
<220>
   <221> misc_feature
   <222> (584)..(584)
   <223> Unknown
<220>
   <221> misc_feature <222> (663)..(663)
   <223> Unknown
<220>
   <221> misc_feature
   <222> (673)..(673)
   <223> Unknown
<220>
   <221> misc_feature
   <222> (717)..(717)
   <223> Unknown
<220>
   <221> misc_feature
   <222> (722)..(722)
   <223> Unknown
<220>
   <221> misc_feature
   <222> (729)..(729)
   <223> Unknown
<220>
   <221> misc_feature
   <222> (732)..(732)
   <223> Unknown
<220>
   <221> misc_feature
   <222> (740)..(742)
   <223> Unknown
<220>
   <221> misc_feature
   <222> (746)..(746)
   <223> Unknown
<220>
   <221> misc_feature
   <222> (748)..(749)
   <223> Unknown
<220>
   <221> misc_feature
   <222> (756)..(756)
   <223> Unknown
<220>
   <221> misc_feature
   <222> (759)..(759)
   <223> Unknown
<220>
   <221> misc_feature
   <222> (762)..(762)
   <223> Unknown
<400> 1
   cgaggcaccg gtagtttgaa ccaaacgcac aatcgacggg caaacgaacg gaagaacaca     60
   accatgaaga tggtgaaatc gatcgccgca ggtctgaccg ccgcggctgc aatcggcgcc    120
   gctgcggccg gtgtgacttc gatcatggct ggcggcccgg tcgtatacca gatgcagccg    180
   gtcgtcttcg gcgcgccact gccgttggac ccggcatccg cccctgacgt cccgaccgcc    240
   gcccagttga ccagcctgct caacagcctc gccgatccca acgtgtcgtt tgcgaacaag    300
   ggcagtctgg tcgagggcgg catcgggggc accgaggcgc gcatcgccga ccacaagctg    360
   aagaaggccg ccgagcacgg ggatctgccg ctgtcgttca gcgtgacgaa catccagccg    420
   gcggccgccg gttcggccac cgccgacgtt tccgtctcgg gtccgaagct ctcgtcgccg    480
   gtcacgcaga acgtcacgtt cgtgaatcaa ggcggctgga tgctgtcacg cgcatcggcg    540
   atggagttgc tgcaggccgc agggnaactg attggcgggc cggnttcagc ccgctgttca    600
   gctacgccgc ccgcctggtg acgcgtccat gtcgaacact cgcgcgtgta gcacggtgcg    660
   gtntgcgcag ggncgcacgc accgcccggt gcaagccgtc ctcgagatag gtggtgnctc    720
   gncaccagng ancacccccn nntcgncnnt tctcgntgnt gnatga                   766
<210> 2
   <211> 752
   <212> DNA
   <213> Mycobacterium tuberculosis
<220>
   <221> misc_feature
   <222> (591)..(591)
   <223> Unknown
<220>
   <221> misc_feature
   <222> (618)..(618)
   <223> Unknown
<220>
   <221> misc_feature
   <222> (624)..(624)
   <223> Unknown
<220>
   <221> misc_feature
   <222> (630)..(630)
   <223> Unknown
<220>
   <221> misc_feature
   <222> (642)..(642)
   <223> Unknown
<220>
   <221> misc_feature
   <222> (647)..(647)
   <223> Unknown
<220>
   <221> misc_feature
   <222> (654)..(654)
   <223> Unknown
<220>
   <221> misc_feature
   <222> (660)..(660)
   <223> Unknown
<220>
   <221> misc_feature
   <222> (665)..(665)
   <223> Unknown
<220>
   <221> misc_feature
   <222> (671)..(671)
   <223> Unknown
<220>
   <221> misc_feature
   <222> (678)..(678)
   <223> Unknown
<220>
   <221> misc_feature
   <222> (685)..(685)
   <223> Unknown
<220>
   <221> misc_feature
   <222> (686)..(686)
   <223> Unknown
<220>
   <221> misc_feature
   <222> (696)..(696)
   <223> Unknown
<220>
   <221> misc_feature
   <222> (700)..(700)
   <223> Unknown
<220>
   <221> misc_feature
   <222> (710)..(710)
   <223> Unknown
<220>
   <221> misc_feature
   <222> (715)..(715)
   <223> Unknown
<220>
   <221> misc_feature
   <222> (719)..(719) <223> Unknown
<220>
   <221> misc_feature
   <222> (724)..(724)
   <223> Unknown
<220>
   <221> misc_feature
   <222> (731)..(731)
   <223> Unknown
<220>
   <221> misc_feature
   <222> (736)..(736)
   <223> Unknown
<220>
   <221> misc_feature
   <222> (740)..(740)
   <223> Unknown
<220>
   <221> mist_feature
   <222> (745)..(745)
   <223> Unknown
<400> 2
   atgcatcacc atcaccatca cgatgaagtc acggtagaga cgacctccgt cttccgcgca 60
   gacttcctca gcgagctgga cgctcctgcg caagcgggta cggagagcgc ggtctccggg 120
   gtggaagggc tcccgccggg ctcggcgttg ctggtagtca aacgaggccc caacgccggg 180
   tcccggttcc tactcgacca agccatcacg tcggctggtc ggcatcccga cagcgacata 240
   tttctcgacg acgtgaccgt gagccgtcgc catgctgaat tccggttgga aaacaacgaa 300
   ttcaatgtcg tcgatgtcgg gagtctcaac ggcacctacg tcaaccgcga gcccgtggat 360
   tcggcggtgc tggcgaacgg cgacgaggtc cagatcggca agctccggtt ggtgttcttg 420
   accggaccca agcaaggcga ggatgacggg agtaccgggg gcccgtgagc gcacccgata 480
   gccccgcgct ggccgggatg tcgatcgggg cggtcctccg acctgctacg accggatttt 540
   ccctgatgtc caccatctcc aagattcgat tcttgggagg cttgagggtc ngggtgaccc 600
   ccccgcgggc ctcattcngg ggtntcggcn ggtttcaccc cntaccnact gccncccggn 660
   ttgcnaattc nttcttcnct gcccnnaaag ggaccnttan cttgccgctn gaaanggtna 720
   tccngggccc ntcctngaan ccccntcccc ct 752
<210> 3
   <211> 813
   <212> DNA
   <213> Mycobacterium tuberculosis
<220>
   <221> misc_feature
   <222> (760)..(760)
   <223> Unknown
<220>
   <221> misc_feature
   <222> (779)..(779)
   <223> Unknown
<220>
   <221> misc_feature
   <222> (807)..(807)
   <223> Unknown
<220>
   <221> misc_feature
   <222> (811)..(811)
   <223> Unknown
<400> 3
   catatgcatc accatcacca tcacacttct aaccgcccag cgcgtcgggg gcgtcgagca    60
   ccacgcgaca ccgggcccga tcgatctgct agcttgagtc tggtcaggca tcgtcgtcag   120
   cagcgcgatg ccctatgttt gtcgtcgact cagatatcgc ggcaatccaa tctcccgcct   180
   gcggccggcg gtgctgcaaa ctactcccgg aggaatttcg acgtgcgcat caagatcttc   240
   atgctggtca cggctgtcgt tttgctctgt tgttcgggtg tggccacggc cgcgcccaag   300
   acctactgcg aggagttgaa aggcaccgat accggccagg cgtgccagat tcaaatgtcc   360
   gacccggcct acaacatcaa catcagcctg cccagttact accccgacca gaagtcgctg   420
   gaaaattaca tcgcccagac gcgcgacaag ttcctcagcg cggccacatc gtccactcca   480
   cgcgaagccc cctacgaatt gaatatcacc tcggccacat accagtccgc gataccgccg   540
   cgtggtacgc aggccgtggt gctcamggtc taccacaacg ccggcggcac gcacccaacg   600
   accacgtaca aggccttcga ttgggaccag gcctatcgca agccaatcac ctatgacacg   660
   ctgtggcagg ctgacaccga tccgctgcca gtcgtcttcc ccattgttgc aaggtgaact   720
   gagcaacgca gaccgggaca acwggtatcg atagccgccn aatgccggct tggaacccng   780
   tgaaattatc acaacttcgc agtcacnaaa naa                                813
<210> 4
   <211> 447
   <212> DNA
   <213> Mycobacterium tuberculosis
<400> 4
   cggtatgaac acggccgcgt ccgataactt ccagctgtcc cagggtgggc agggattcgc    60
   cattccgatc gggcaggcga tggcgatcgc gggccagatc cgatcgggtg gggggtcacc   120
   caccgttcat atcgggccta ccgccttcct cggcttgggt gttgtcgaca acaacggcaa      180
   cggcgcacga gtccaacgcg tggtcgggag cgctccggcg gcaagtctcg gcatctccac      240
   cggcgacgtg atcaccgcgg tcgacggcgc tccgatcaac tcggccaccg cgatggcgga      300
   cgcgcttaac gggcatcatc ccggtgacgt catctcggtg aactggcaaa ccaagtcggg      360
   cggcacgcgt acagggaacg tgacattggc cgagggaccc ccggcctgat ttcgtcgygg      420
   ataccacccg ccggccggcc aattgga                                          447
<210> 5
   <211> 604
   <212> DNA
   <213> Mycobacterium tuberculosis
<220>
   <221> misc_feature
   <222> (191)..(191)
   <223> Unknown
<220>
   <221> misc_feature
   <222> (223)..(223)
   <223> Unknown
<220>
   <221> misc_feature
   <222> (237)..(237)
   <223> Unknown
<220>
   <221> misc_feature
   <222> (255)..(255)
   <223> Unknown
<220>
   <221> misc_feature
   <222> (262)..(262)
   <223> Unknown
<220>
   <221> misc_feature
   <222> (266)..(266)
   <223> Unknown
<220>
   <221> misc_feature
   <222> (270)..(270)
   <223> Unknown
<220>
   <221> misc_feature
   <222> (319)..(319)
   <223> Unknown
<220>
   <221> misc_feature
   <222> (322)..(322)
   <223> Unknown
<220>
   <221> misc_feature
   <222> (331)..(331)
   <223> Unknown
<220>
   <221> misc_feature
   <222> (345)..(345)
   <223> Unknown
<220>
   <221> misc_feature
   <222> (352)..(352)
   <223> Unknown
<220>
   <221> misc_feature
   <222> (356)..(356)
   <223> Unknown
<220>
   <221> misc_feature
   <222> (361)..(361)
   <223> Unknown
<220>
   <221> misc_feature
   <222> (364)..(364)
   <223> Unknown
<220>
   <221> misc_feature
   <222> (366)..(366)
   <223> Unknown
<220>
   <221> misc_feature
   <222> (369)..(369)
   <223> Unknown
<220>
   <221> misc_feature
   <222> (387)..(387)
   <223> Unknown
<220>
   <221> misc_feature
   <222> (393)..(393)
   <223> Unknown
<220>
   <221> misc_feature
   <222> (400)..(400)
   <223> Unknown
<220>
   <221> misc_feature
   <222> (416)..(416)
   <223> Unknown
<220>
   <221> misc_feature
   <222> (421)..(422)
   <223> Unknown
<220>
   <221> misc_feature
   <222> (424)..(425)
   <223> Unknown
<220>
   <221> misc_feature
   <222> (428)..(428) <223> Unknown
<220>
   <221> misc_feature
   <222> (430)..(430)
   <223> Unknown
<220>
   <221> misc_feature
   <222> (433)..(433)
   <223> Unknown
<220>
   <221> misc_feature
   <222> (438)..(438)
   <223> Unknown
<220>
   <221> misc_feature
   <222> (440)..(440)
   <223> Unknown
<220>
   <221> misc_feature
   <222> (444)..(447)
   <223> Unknown
<220>
   <221> misc_feature
   <222> (454)..(454)
   <223> Unknown
<220>
   <221> misc_feature
   <222> (458)..(458)
   <223> Unknown
<220>
   <221> misc_feature
   <222> (463)..(463)
   <223> Unknown
<220>
   <221> misc_feature
   <222> (465)..(465)
   <223> Unknown
<220>
   <221> misc_feature
   <222> (468)..(468)
   <223> Unknown
<220>
   <221> misc_feature
   <222> (471)..(471)
   <223> Unknown
<220>
   <221> misc_feature
   <222> (478)..(478)
   <223> Unknown
<220>
   <221> misc_feature
   <222> (481)..(481)
   <223> Unknown
<220>
   <221> misc_feature
   <222> (493)..(493)
   <223> Unknown
<220>
   <221> misc_feature
   <222> (497)..(500)
   <223> Unknown
<220>
   <221> misc_feature
   <222> (503)..(503)
   <223> Unknown
<220>
   <221> misc_feature
   <222> (505)..(505)
   <223> Unknown
<220>
   <221> misc_feature
   <222> (507)..(507)
   <223> Unknown
<220>
   <221> misc_feature
   <222> (510)..(510)
   <223> Unknown
<220>
   <221> misc_feature
   <222> (513)..(513)
   <223> Unknown
<220>
   <221> misc_feature
   <222> (516)..(516)
   <223> Unknown
<220>
   <221> misc_feature
   <222> (519)..(519)
   <223> Unknown
<220>
   <221> misc_feature
   <222> (521)..(523)
   <223> Unknown
<220>
   <221> misc_feature
   <222> (525)..(526)
   <223> Unknown
<220>
   <221> misc_feature
   <222> (528)..(529)
   <223> Unknown
<220>
   <221> misc_feature
   <222> (531)..(532)
   <223> Unknown
<220>
   <221> misc_feature
   <222> (535)..(535)
   <223> Unknown
<220>
   <221> misc_feature
   <222> (537)..(537)
   <223> Unknown
<220>
   <221> misc_feature
   <222> (539)..(539)
   <223> Unknown
<220>
   <221> misc_feature
   <222> (541)..(543)
   <223> Unknown
<220>
   <221> misc_feature
   <222> (545)..(545)
   <223> Unknown
<220>
   <221> misc_feature
   <222> (548)..(549)
   <223> Unknown
<220>
   <221> misc_feature
   <222> (552)..(558)
   <223> Unknown
<220>
   <221> misc_feature
   <222> (561)..(562)
   <223> Unknown
<220>
   <221> misc_feature
   <222> (564)..(565)
   <223> Unknown
<220>
   <221> misc_feature
   <222> (567)..(568)
   <223> Unknown
<220>
   <221> misc_feature
   <222> (570)..(573)
   <223> Unknown
<220>
   <221> misc_feature
   <222> (577)..(577)

   <223> Unknown
<220>
   <221> misc_feature
   <222> (579)..(581)
   <223> Unknown
<220>
   <221> misc_feature
   <222> (583)..(586)
   <223> Unknown
<220>
   <221> misc_feature
   <222> (588)..(588)
   <223> Unknown
<220>
   <221> misc_feature
   <222> (591)..(592)
   <223> Unknown
<220>
   <221> misc_feature
   <222> (595)..(595)
   <223> Unknown
<220>
   <221> misc_feature
   <222> (598)..(598)
   <223> Unknown
<220>
   <221> misc_feature
   <222> (601)..(601)
   <223> Unknown
<400> 5
   gtcccactgc ggtcgccgag tatgtcgccc agcaaatgtc tggcagccgc ccaacggaat      60
   ccggtgatcc gacgtcgcag gttgtcgaac ccgccgccgc ggaagtatcg gtccatgcct     120
   agcccggcga cggcgagcgc cggaatggcg cgagtgagga ggcgggcaat ttggcggggc     180
   ccggcgacgg ngagcgccgg aatggcgcga gtgaggaggt ggncagtcat gcccagngtg     240
   atccaatcaa cctgnattcg gnctgngggn ccatttgaca atcgaggtag tgagcgcaaa     300
   tgaatgatgg aaaacgggng gngacgtccg ntgttctggt ggtgntaggt gnctgnctgg     360
   ngtngnggnt atcaggatgt tcttcgncga aanctgatgn cgaggaacag ggtgtncccg     420
   nnannccnan ggngtccnan cccnnnntcc tcgncganat cananagncg nttgatgnga     480
   naaaagggtg gancagnnnn aantngnggn ccnaanaanc nnnanngnng nnagntngnt     540
   nnntnttnnc annnnnnntg nngnngnncn nnncaancnn ntnnnngnaa nnggnttntt     600
   naat                                                                  604
<210> 6
   <211> 633
   <212> DNA
   <213> Mycobacterium tuberculosis
<220>
   <221> misc_feature
   <222> (6)..(6)
   <223> Unknown
<220>
   <221> misc_feature
   <222> (39)..(39)
   <223> Unknown
<220>
   <221> misc_feature
   <222> (507)..(507)
   <223> Unknown
<220>
   <221> misc_feature
   <222> (583)..(583)
   <223> Unknown
<400> 6
   ttgcangtcg aaccacctca ctaaagggaa caaaagctng agctccaccg cggtggcggc      60
   cgctctagaa ctagtgkatm yyyckggctg cagsaatycg gyacgagcat taggacagtc     120
   taacggtcct gttacggtga tcgaatgacc gacgacatcc tgctgatcga caccgacgaa     180
   cgggtgcgaa ccctcaccct caaccggccg cagtcccgya acgcgctctc ggcggcgcta     240
   cgggatcggt ttttcgcggy gttggycgac gccgaggycg acgacgacat cgacgtcgtc     300
   atcctcaccg gygccgatcc ggtgttctgc gccggactgg acctcaaggt agctggccgg     360
   gcagaccgcg ctgccggaca tctcaccgcg gtgggcggcc atgaccaagc cggtgatcgg     420
   cgcgatcaac ggcgccgcgg tcaccggcgg gctcgaactg gcgctgtact gcgacatcct     480
   gatcgcctcc gagcacgccc gcttcgncga cacccacgcc cgggtggggc tgctgcccac     540
   ctggggactc agtgtgtgct tgccgcaaaa ggtcggcatc ggnctgggcc ggtggatgag     600
   cctgaccggc gactacctgt ccgtgaccga cgc                                 633
<210> 7
   <211> 1362
   <212> DNA
   <213> Mycobacterium tuberculosis
<400> 7
   cgacgacgac ggcgccggag agcgggcgcg aacggcgatc gacgcggccc tggccagagt      60
   cggcaccacc caggagggag tcgaatcatg aaatttgtca accatattga gcccgtcgcg     120
   ccccgccgag ccggcggcgc ggtcgccgag gtctatgccg aggcccgccg cgagttcggc     180
   cggctgcccg agccgctcgc catgctgtcc ccggacgagg gactgctcac cgccggctgg     240
   gcgacgttgc gcgagacact gctggtgggc caggtgccgc gtggccgcaa ggaagccgtc     300
   gccgccgccg tcgcggccag cctgcgctgc ccctggtgcg tcgacgcaca caccaccatg     360
   ctgtacgcgg caggccaaac cgacaccgcc gcggcgatct tggccggcac agcacctgcc     420
   gccggtgacc cgaacgcgcc gtatgtggcg tgggcggcag gaaccgggac accggcggga     480
   ccgccggcac cgttcggccc ggatgtcgcc gccgaatacc tgggcaccgc ggtgcaattc     540
   cacttcatcg cacgcctggt cctggtgctg ctggacgaaa ccttcctgcc ggggggcccg     600
   cgcgcccaac agctcatgcg ccgcgccggt ggactggtgt tcgcccgcaa ggtgcgcgcg     660
   gagcatcggc cgggccgctc cacccgccgg ctcgagccgc gaacgctgcc cgacgatctg     720
   gcatgggcaa caccgtccga gcccatagca accgcgttcg ccgcgctcag ccaccacctg     780
   gacaccgcgc cgcacctgcc gccaccgact cgtcaggtgg tcaggcgggt cgtggggtcg     840
   tggcacggcg agccaatgcc gatgagcagt cgctggacga acgagcacac cgccgagctg     900
   cccgccgacc tgcacgcgcc cacccgtctt gccctgctga ccggcctggc cccgcatcag     960
   gtgaccgacg acgacgtcgc cgcggcccga tccctgctcg acaccgatgc ggcgctggtt    1020
   ggcgccctgg cctgggccgc cttcaccgcc gcgcggcgca tcggcacctg gatcggcgcc    1080
   gccgccgagg gccaggtgtc gcggcaaaac ccgactgggt gagtgtgcgc gccctgtcgg    1140
   tagggtgtca tcgctggccc gagggatctc gcggcggcga acggaggtgg cgacacaggt    1200
   ggaagctgcg cccactggct tgcgccccaa cgccgtcgtg ggcgttcggt tggccgcact    1260
   ggccgatcag gtcggcgccg gcccttggcc gaaggtccag ctcaacgtgc cgtcaccgaa    1320
   ggaccggacg gtcaccgggg gtcaccctgc gcgcccaagg aa                       1362
<210> 8
   <211> 1458
   <212> DNA
   <213> Mycobacterium tuberculosis
<400> 8
   gcgacgaccc cgatatgccg ggcaccgtag cgaaagccgt cgccgacgca ctcgggcgcg      60
   gtatcgctcc cgttgaggac attcaggact gcgtggaggc ccggctgggg gaagccggtc     120
   tggatgacgt ggcccgtgtt tacatcatct accggcagcg gcgcgccgag ctgcggacgg     180
   ctaaggcctt gctcggcgtg cgggacgagt taaagctgag cttggcggcc gtgacggtac     240
   tgcgcgagcg ctatctgctg cacgacgagc agggccggcc ggccgagtcg accggcgagc     300
   tgatggaccg atcggcgcgc tgtgtcgcgg cggccgagga ccagtatgag ccgggctcgt     360
   cgaggcggtg ggccgagcgg ttcgccacgc tattacgcaa cctggaattc ctgccgaatt     420
   cgcccacgtt gatgaactct ggcaccgacc tgggactgct cgccggctgt tttgttctgc     480
   cgattgagga ttcgctgcaa tcgatctttg cgacgctggg acaggccgcc gagctgcagc     540
   gggctggagg cggcaccgga tatgcgttca gccacctgcg acccgccggg gatcgggtgg     600
   cctccacggg cggcacggcc agcggaccgg tgtcgtttct acggctgtat gacagtgccg     660
   cgggtgtggt ctccatgggc ggtcgccggc gtggcgcctg tatggctgtg cttgatgtgt     720
   cgcacccgga tatctgtgat ttcgtcaccg ccaaggccga atcccccagc gagctcccgc     780
   atttcaacct atcggttggt gtgaccgacg cgttcctgcg ggccgtcgaa cgcaacggcc     840
   tacaccggct ggtcaatccg cgaaccggca agatcgtcgc gcggatgccc gccgccgagc     900
   tgttcgacgc catctgcaaa gccgcgcacg ccggtggcga tcccgggctg gtgtttctcg     960
   acacgatcaa tagggcaaac ccggtgccgg ggagaggccg catcgaggcg accaacccgt    1020
   gcggggaggt cccactgctg ccttacgagt catgtaatct cggctcgatc aacctcgccc    1080
   ggatgctcgc cgacggtcgc gtcgactggg accggctcga ggaggtcgcc ggtgtggcgg    1140
   tgcggttcct tgatgacgtc atcgatgtca gccgctaccc cttccccgaa ctgggtgagg    1200
   cggcccgcgc cacccgcaag atcgggctgg gagtcatggg tttggcggaa ctgcttgccg    1260
   cactgggtat tccgtacgac agtgaagaag ccgtgcggtt agccacccgg ctcatgcgtc    1320
   gcatacagca ggcggcgcac acggcatcgc ggaggctggc cgaagagcgg ggcgcattcc    1380
   cggcgttcac cgatagccgg ttcgcgcggt cgggcccgag gcgcaacgca caggtcacct    1440
   ccgtcgctcc gacgggca                                                 1458
<210> 9
   <211> 862
   <212> DNA
   <213> Mycobacterium tuberculosis
<400> 9
   acggtgtaat cgtgctggat ctggaaccgc gtggcccgct acctaccgag atctactggc 60
   ggcgcagggg gctggccctg ggcatcgcgg tcgtcgtagt cgggatcgcg gtggccatcg 120
   tcatcgcctt cgtcgacagc agcgccggtg ccaaaccggt cagcgccgac aagccggcct 180
   ccgcccagag ccatccgggc tcgccggcac cccaagcacc ccagccggcc gggcaaaccg 240
   aaggtaacgc cgccgcggcc ccgccgcagg gccaaaaccc cgagacaccc acgcccaccg 300
   ccgcggtgca gccgccgccg gtgctcaagg aaggggacga ttgccccgat tcgacgctgg 360
   ccgtcaaagg tttgaccaac gcgccgcagt actacgtcgg cgaccagccg aagttcacca 420
   tggtggtcac caacatcggc ctggtgtcct gtaaacgcga cgttggggcc gcggtgttgg 480
   ccgcctacgt ttactcgctg gacaacaagc ggttgtggtc caacctggac tgcgcgccct 540
   cgaatgagac gctggtcaag acgttttccc ccggtgagca ggtaacgacc gcggtgacct 600
   ggaccgggat gggatcggcg ccgcgctgcc cattgccgcg gccggcgatc gggccgggca 660
   cctacaatct cgtggtacaa ctgggcaatc tgcgctcgct gccggttccg ttcatcctga 720
   atcagccgcc gccgccgccc gggccggtac ccgctccggg tccagcgcag gcgcctccgc 780
   cggagtctcc cgcgcaaggc ggataattat tgatcgctga tggtcgattc cgccagctgt 840
   gacaacccct cgcctcgtgc cg 862
<210> 10
   <211> 622
   <212> DNA
   <213> Mycobacterium tuberculosis
<220>
   <221> misc_feature
   <222> (578)..(578)
   <223> Unknown
<220>
   <221> misc_feature
   <222> (585)..(585)
   <223> Unknown
<220>
   <221> misc_feature
   <222> (614)..(614)
   <223> Unknown
<400> 10
   ttgatcagca ccggcaaggc gtcacatgcc tccctgggtg tgcaggtgac caatgacaaa 60
   gacaccccgg gcgccaagat cgtcgaagta gtggccggtg gtgctgccgc gaacgctgga 120
   gtgccgaagg gcgtcgttgt caccaaggtc gacgaccgcc cgatcaacag cgcggacgcg 180
   ttggttgccg ccgtgcggtc caaagcgccg ggcgccacgg tggcgctaac ctttcaggat 240
   ccctcgggcg gtagccgcac agtgcaagtc accctcggca aggcggagca gtgatgaagg 300
   tcgccgcgca gtgttcaaag ctcggatata cggtggcacc catggaacag cgtgcggagt 360
   tggtggttgg ccgggcactt gtcgtcgtcg ttgacgatcg cacggcgcac ggcgatgaag 420
   accacagcgg gccgcttgtc accgagctgc tcaccgaggc cgggtttgtt gtcgacggcg 480
   tggtggcggt gtcggccgac gaggtcgaga tccgaaatgc gctgaacaca gcggtgatcg 540
   gcggggtgga cctggtggtg tcggtcggcg ggaccggngt gacgnctcgc gatgtcaccc 600
   cggaagccac ccgngacatt ct 622
<210> 11
   <211> 1200
   <212> DNA
   <213> Mycobacterium tuberculosis
<400> 11
   ggcgcagcgg taagcctgtt ggccgccggc acactggtgt tgacagcatg cggcggtggc 60
   accaacagct cgtcgtcagg cgcaggcgga acgtctgggt cggtgcactg cggcggcaag 120
   aaggagctcc actccagcgg ctcgaccgca caagaaaatg ccatggagca gttcgtctat 180
   gcctacgtgc gatcgtgccc gggctacacg ttggactaca acgccaacgg gtccggtgcc 240
   ggggtgaccc agtttctcaa caacgaaacc gatttcgccg gctcggatgt cccgttgaat 300
   ccgtcgaccg gtcaacctga ccggtcggcg gagcggtgcg gttccccggc atgggacctg 360
   ccgacggtgt tcggcccgat cgcgatcacc tacaatatca agggcgtgag cacgctgaat 420
   cttgacggac ccactaccgc caagattttc aacggcacca tcaccgtgtg gaatgatcca 480
   cagatccaag ccctcaactc cggcaccgac ctgccgccaa caccgattag cgttatcttc 540
   cgcagcgaca agtccggtac gtcggacaac ttccagaaat acctcgacgg tgtatccaac 600
   ggggcgtggg gcaaaggcgc cagcgaaacg ttcagcgggg gcgtcggcgt cggcgccagc 660
   gggaacaacg gaacgtcggc cctactgcag acgaccgacg ggtcgatcac ctacaacgag 720
   tggtcgtttg cggtgggtaa gcagttgaac atggcccaga tcatcacgtc ggcgggtccg 780
   gatccagtgg cgatcaccac cgagtcggtc ggtaagacaa tcgccggggc caagatcatg 840
   ggacaaggca acgacctggt attggacacg tcgtcgttct acagacccac ccagcctggc 900
   tcttacccga tcgtgctggc gacctatgag atcgtctgct cgaaataccc ggatgcgacg 960
   accggtactg cggtaagggc gtttatgcaa gccgcgattg gtccaggcca agaaggcctg 1020
   gaccaatacg gctccattcc gttgcccaaa tcgttccaag caaaattggc ggccgcggtg 1080
   aatgctattt cttgacctag tgaagggaat tcgacggtga gcgatgccgt tccgcaggta 1140
   gggtcgcaat ttgggccgta tcagctattg cggctgctgg gccgaggcgg gatgggcgag 1200
<210> 12
   <211> 1155
   <212> DNA
   <213> Mycobacterium tuberculosis
<400> 12
   gcaagcagct gcaggtcgtg ctgttcgacg aactgggcat gccgaagacc aaacgcacca 60
   agaccggcta caccacggat gccgacgcgc tgcagtcgtt gttcgacaag accgggcatc 120
   cgtttctgca acatctgctc gcccaccgcg acgtcacccg gctcaaggtc accgtcgacg 180
   ggttgctcca agcggtggcc gccgacggcc gcatccacac cacgttcaac cagacgatcg      240
   ccgcgaccgg ccggctctcc tcgaccgaac ccaacctgca gaacatcccg atccgcaccg      300
   acgcgggccg gcggatccgg gacgcgttcg tggtcgggga cggttacgcc gagttgatga      360
   cggccgacta cagccagatc gagatgcgga tcatggggca cctgtccggg gacgagggcc      420
   tcatcgaggc gttcaacacc ggggaggacc tgtattcgtt cgtcgcgtcc cgggtgttcg      480
   gtgtgcccat cgacgaggtc accggcgagt tgcggcgccg ggtcaaggcg atgtcctacg      540
   ggctggttta cgggttgagc gcctacggcc tgtcgcagca gttgaaaatc tccaccgagg      600
   aagccaacga gcagatggac gcgtatttcg cccgattcgg cggggtgcgc gactacctgc      660
   gcgccgtagt cgagcgggcc cgcaaggacg gctacacctc gacggtgctg ggccgtcgcc      720
   gctacctgcc cgagctggac agcagcaacc gtcaagtgcg ggaggccgcc gagcgggcgg      780
   cgctgaacgc gccgatccag ggcagcgcgg ccgacatcat caaggtggcc atgatccagg      840
   tcgacaaggc gctcaacgag gcacagctgg cgtcgcgcat gctgctgcag gtccacgacg      900
   agctgctgtt cgaaatcgcc cccggtgaac gcgagcgggt cgaggccctg gtgcgcgaca      960
   agatgggcgg cgcttacccg ctcgacgtcc cgctggaggt gtcggtgggc tacggccgca     1020
   gctgggacgc ggcggcgcac tgagtgccga gcgtgcatct ggggcgggaa ttcggcgatt     1080
   tttccgccct gagttcacgc tcggcgcaat cgggaccgag tttgtccagc gtgtacccgt     1140
   cgagtagcct cgtca                                                      1155
<210> 13
   <211> 1771
   <212> DNA
   <213> Mycobacterium tuberculosis
<400> 13
   gagcgccgtc tggtgtttga acggttttac cggtcggcat cggcacgggc gttgccgggt      60
   tcgggcctcg ggttggcgat cgtcaaacag gtggtgctca accacggcgg attgctgcgc     120
   atcgaagaca ccgacccagg cggccagccc cctggaacgt cgatttacgt gctgctcccc     180
   ggccgtcgga tgccgattcc gcagcttccc ggtgcgacgg ctggcgctcg gagcacggac     240
   atcgagaact ctcggggttc ggcgaacgtt atctcagtgg aatctcagtc cacgcgcgca     300
   acctagttgt gcagttactg ttgaaagcca cacccatgcc agtccacgca tggccaagtt     360
   ggcccgagta gtgggcctag tacaggaaga gcaacctagc gacatgacga atcacccacg     420
   gtattcgcca ccgccgcagc agccgggaac cccaggttat gctcaggggc agcagcaaac     480
   gtacagccag cagttcgact ggcgttaccc accgtccccg cccccgcagc caacccagta     540
   ccgtcaaccc tacgaggcgt tgggtggtac ccggccgggt ctgatacctg gcgtgattcc     600
   gaccatgacg ccccctcctg ggatggttcg ccaacgccct cgtgcaggca tgttggccat     660
   cggcgcggtg acgatagcgg tggtgtccgc cggcatcggc ggcgcggccg catccctggt     720
   cgggttcaac cgggcacccg ccggccccag cggcggccca gtggctgcca gcgcggcgcc     780
   aagcatcccc gcagcaaaca tgccgccggg gtcggtcgaa caggtggcgg ccaaggtggt     840
   gcccagtgtc gtcatgttgg aaaccgatct gggccgccag tcggaggagg gctccggcat     900
   cattctgtct gccgaggggc tgatcttgac caacaaccac gtgatcgcgg cggccgccaa     960
   gcctcccctg ggcagtccgc cgccgaaaac gacggtaacc ttctctgacg ggcggaccgc    1020
   acccttcacg gtggtggggg ctgaccccac cagtgatatc gccgtcgtcc gtgttcaggg    1080
   cgtctccggg ctcaccccga tctccctggg ttcctcctcg gacctgaggg tcggtcagcc    1140
   ggtgctggcg atcgggtcgc cgctcggttt ggagggcacc gtgaccacgg ggatcgtcag    1200
   cgctctcaac cgtccagtgt cgacgaccgg cgaggccggc aaccagaaca ccgtgctgga    1260
   cgccattcag accgacgccg cgatcaaccc cggtaactcc gggggcgcgc tggtgaacat    1320
   gaacgctcaa ctcgtcggag tcaactcggc cattgccacg ctgggcgcgg actcagccga    1380
   tgcgcagagc ggctcgatcg gtctcggttt tgcgattcca gtcgaccagg ccaagcgcat    1440
   cgccgacgag ttgatcagca ccggcaaggc gtcacatgcc tccctgggtg tgcaggtgac    1500
   caatgacaaa gacaccccgg gcgccaagat cgtcgaagta gtggccggtg gtgctgccgc    1560
   gaacgctgga gtgccgaagg gcgtcgttgt caccaaggtc gacgaccgcc cgatcaacag    1620
   cgcggacgcg ttggttgccg ccgtgcggtc caaagcgccg ggcgccacgg tggcgctaac    1680
   ctttcaggat ccctcgggcg gtagccgcac agtgcaagtc accctcggca aggcggagca    1740
   gtgatgaagg tcgccgcgca gtgttcaaag c                                   1771
<210> 14
   <211> 1058
   <212> DNA
   <213> Mycobacterium tuberculosis
<220>
   <221> misc_feature
   <222> (1025)..(1025)
   <223> Unknown
<220>
   <221> misc_feature
   <222> (1056)..(1056)
   <223> Unknown
<400> 14
   ctccaccgcg gtggcggccg ctctagaact agtggatccc ccgggctgca ggaattcggc      60
   acgaggatcc gacgtcgcag gttgtcgaac ccgccgccgc ggaagtatcg gtccatgcct     120
   agcccggcga cggcgagcgc cggaatggcg cgagtgagga ggcgggcaat ttggcggggc     180
   ccggcgacgg cgagcgccgg aatggcgcga gtgaggaggc gggcagtcat gcccagcgtg     240
   atccaatcaa cctgcattcg gcctgcgggc ccatttgaca atcgaggtag tgagcgcaaa     300
   tgaatgatgg aaaacgggcg gtgacgtccg ctgttctggt ggtgctaggt gcctgcctgg     360
   cgttgtggct atcaggatgt tcttcgccga aacctgatgc cgaggaacag ggtgttcccg     420
   tgagcccgac ggcgtccgac cccgcgctcc tcgccgagat caggcagtcg cttgatgcga     480
   caaaagggtt gaccagcgtg cacgtagcgg tccgaacaac cgggaaagtc gacagcttgc     540
   tgggtattac cagtgccgat gtcgacgtcc gggccaatcc gctcgcggca aagggcgtat     600
   gcacctacaa cgacgagcag ggtgtcccgt ttcgggtaca aggcgacaac atctcggtga     660
   aactgttcga cgactggagc aatctcggct cgatttctga actgtcaact tcacgcgtgc     720
   tcgatcctgc cgctggggtg acgcagctgc tgtccggtgt cacgaacctc caagcgcaag     780
   gtaccgaagt gatagacgga atttcgacca ccaaaatcac cgggaccatc cccgcgagct     840
   ctgtcaagat gcttgatcct ggcgccaaga gtgcaaggcc ggcgaccgtg tggattgccc     900
   aggacggctc gcaccacctc gtccgagcga gcatcgacct cggatccggg tcgattcagc     960
   tcacgcagtc gaaatggaac gaacccgtca acgtcgacta ggccgaagtt gcgtcgacgc    1020
   gttgntcgaa acgcccttgt gaacggtgtc aacggnac                            1058
<210> 15
   <211> 542
   <212> DNA
   <213> Mycobacterium tuberculosis
<220>
   <221> misc_feature
   <222> (406)..(406)
   <223> Unknown
<400> 15
   gaattcggca cgagaggtga tcgacatcat cgggaccagc cccacatcct gggaacaggc 60
   ggcggcggag gcggtccagc gggcgcggga tagcgtcgat gacatccgcg tcgctcgggt 120
   cattgagcag gacatggccg tggacagcgc cggcaagatc acctaccgca tcaagctcga 180
   agtgtcgttc aagatgaggc cggcgcaacc gcgctagcac gggccggcga gcaagacgca 240
   aaatcgcacg gtttgcggtt gattcgtgcg attttgtgtc tgctcgccga ggcctaccag 300
   gcgcggccca ggtccgcgtg ctgccgtatc caggcgtgca tcgcgattcc ggcggccacg 360
   ccggagttaa tgcttcgcgt cgacccgaac tgggcgatcc gccggngagc tgatcgatga 420
   ccgtggccag cccgtcgatg cccgagttgc ccgaggaaac gtgctgccag gccggtagga 480
   agcgtccgta ggcggcggtg ctgaccggct ctgcctgcgc cctcagtgcg gccagcgagc 540
   gg 542
<210> 16
   <211> 913
   <212> DNA
   <213> Mycobacterium tuberculosis
<220>
   <221> misc_feature
   <222> (614)..(614)
   <223> Unknown
<220>
   <221> misc_feature
   <222> (773)..(773)
   <223> unknown
<220>
   <221> misc_feature
   <222> (886)..(886)
   <223> Unknown
<400> 16
   cggtgccgcc cgcgcctccg ttgcccccat tgccgccgtc gccgatcagc tgcgcatcgc 60
   caccatcacc gcctttgccg ccggcaccgc cggtggcgcc ggggccgccg atgccaccgc 120
   ttgaccctgg ccgccggcgc cgccattgcc atacagcacc ccgccggggg caccgttacc 180
   gccgtcgcca ccgtcgccgc cgctgccgtt tcaggccggg gaggccgaat gaaccgccgc 240
   caagcccgcc gccggcaccg ttgccgcctt ttccgcccgc cccgccggcg ccgccaattg 300
   ccgaacagcc amgcaccgtt gccgccagcc ccgccgccgt taacggcgct gccgggcgcc 360
   gccgccggac ccgccattac cgccgttccc gttcggtgcc ccgccgttac cggcgccgcc 420
   gtttgccgcc aatattcggc gggcaccgcc agacccgccg gggccaccat tgccgccggg 480
   caccgaaaca acagcccaac ggtgccgccg gccccgccgt ttgccgccat caccggccat 540
   tcaccgccag caccgccgtt aatgtttatg aacccggtac cgccagcgcg gcccctattg 600
   ccgggcgccg gagngcgtgc ccgccggcgc cgccaacgcc caaaagcccg gggttgccac 660
   cggccccgcc ggacccaccg gtcccgccga tccccccgtt gccgccggtg ccgccgccat 720
   tggtgctgct gaagccgtta gcgccggttc cgcsggttcc ggcggtggcg ccntggccgc 780
   cggccccgcc gttgccgtac agccaccccc cggtggcgcc gttgccgcca ttgccgccat 840
   tgccgccgtt gccgccattg ccgccgttcc cgccgccacc gccggnttgg ccgccggcgc 900
   cgccggcggc cgc 913
<210> 17
   <211> 1872
   <212> DNA
   <213> Mycobacterium tuberculosis
<220>
   <221> misc_feature
   <222> (1460)..(1460)
   <223> unknown
<220>
   <221> misc_feature
   <222> (1854)..(1854)
   <223> Unknown
<400> 17
   gactacgttg gtgtagaaaa atcctgccgc ccggaccctt aaggctggga caatttctga 60
   tagctacccc gacacaggag gttacgggat gagcaattcg cgccgccgct cactcaggtg 120
   gtcatggttg ctgagcgtgc tggctgccgt cgggctgggc ctggccacgg cgccggccca 180
   ggcggccccg ccggccttgt cgcaggaccg gttcgccgac ttccccgcgc tgcccctcga 240
   cccgtccgcg atggtcgccc aagtggcgcc acaggtggtc aacatcaaca ccaaactggg 300
   ctacaacaac gccgtgggcg ccgggaccgg catcgtcatc gatcccaacg gtgtcgtgct 360
   gaccaacaac cacgtgatcg cgggcgccac cgacatcaat gcgttcagcg tcggctccgg 420
   ccaaacctac ggcgtcgatg tggtcgggta tgaccgcacc caggatgtcg cggtgctgca 480
   gctgcgcggt gccggtggcc tgccgtcggc ggcgatcggt ggcggcgtcg cggttggtga      540
   gcccgtcgtc gcgatgggca acagcggtgg gcagggcgga acgccccgtg cggtgcctgg      600
   cagggtggtc gcgctcggcc aaaccgtgca ggcgtcggat tcgctgaccg gtgccgaaga      660
   gacattgaac gggttgatcc agttcgatgc cgcaatccag cccggtgatt cgggcgggcc      720
   cgtcgtcaac ggcctaggac aggtggtcgg tatgaacacg gccgcgtccg ataacttcca      780
   gctgtcccag ggtgggcagg gattcgccat tccgatcggg caggcgatgg cgatcgcggg      840
   ccaaatccga tcgggtgggg ggtcacccac cgttcatatc gggcctaccg ccttcctcgg      900
   cttgggtgtt gtcgacaaca acggcaacgg cgcacgagtc caacgcgtgg tcggaagcgc      960
   tccggcggca agtctcggca tctccaccgg cgacgtgatc accgcggtcg acggcgctcc     1020
   gatcaactcg gccaccgcga tggcggacgc gcttaacggg catcatcccg gtgacgtcat     1080
   ctcggtgaac tggcaaacca agtcgggcgg cacgcgtaca gggaacgtga cattggccga     1140
   gggacccccg gcctgatttg tcgcggatac cacccgccgg ccggccaatt ggattggcgc     1200
   cagccgtgat tgccgcgtga gcccccgagt tccgtctccc gtgcgcgtgg cattgtggaa     1260
   gcaatgaacg aggcagaaca cagcgttgag caccctcccg tgcagggcag ttacgtcgaa     1320
   ggcggtgtgg tcgagcatcc ggatgccaag gacttcggca gcgccgccgc cctgcccgcc     1380
   gatccgacct ggtttaagca cgccgtcttc tacgaggtgc tggtccgggc gttcttcgac     1440
   gccagcgcgg acggttccgn cgatctgcgt ggactcatcg atcgcctcga ctacctgcag     1500
   tggcttggca tcgactgcat ctgttgccgc cgttcctacg actcaccgct gcgcgacggc     1560
   ggttacgaca ttcgcgactt ctacaaggtg ctgcccgaat tcggcaccgt cgacgatttc     1620
   gtcgccctgg tcgacaccgc tcaccggcga ggtatccgca tcatcaccga cctggtgatg     1680
   aatcacacct cggagtcgca cccctggttt caggagtccc gccgcgaccc agacggaccg     1740
   tacggtgact attacgtgtg gagcgacacc agcgagcgct acaccgacgc ccggatcatc     1800
   ttcgtcgaca ccgaagagtc gaactggtca ttcgatcctg tccgccgaca gttnctactg     1860
   gcaccgattc tt                                                         1872
<210> 18
   <211> 1482
   <212> DNA
   <213> Mycobacterium tuberculosis
<400> 18
   cttcgccgaa acctgatgcc gaggaacagg gtgttcccgt gagcccgacg gcgtccgacc      60
   ccgcgctcct cgccgagatc aggcagtcgc ttgatgcgac aaaagggttg accagcgtgc     120
   acgtagcggt ccgaacaacc gggaaagtcg acagcttgct gggtattacc agtgccgatg     180
   tcgacgtccg ggccaatccg ctcgcggcaa agggcgtatg cacctacaac gacgagcagg     240
   gtgtcccgtt tcgggtacaa ggcgacaaca tctcggtgaa actgttcgac gactggagca     300
   atctcggctc gatttctgaa ctgtcaactt cacgcgtgct cgatcctgcc gctggggtga     360
   cgcagctgct gtccggtgtc acgaacctcc aagcgcaagg taccgaagtg atagacggaa     420
   tttcgaccac caaaatcacc gggaccatcc ccgcgagctc tgtcaagatg cttgatcctg     480
   gcgccaagag tgcaaggccg gcgaccgtgt ggattgccca ggacggctcg caccacctcg     540
   tccgagcgag catcgacctc ggatccgggt cgattcagct cacgcagtcg aaatggaacg     600
   aacccgtcaa cgtcgactag gccgaagttg cgtcgacgcg ttgctcgaaa cgcccttgtg     660
   aacggtgtca acggcacccg aaaactgacc ccctgacggc atctgaaaat tgacccccta     720
   gaccgggcgg ttggtggtta ttcttcggtg gttccggctg gtgggacgcg gccgaggtcg     780
   cggtctttga gccggtagct gtcgcctttg agggcgacga cttcagcatg gtggacgagg     840
   cggtcgatca tggcggcagc aacgacgtcg tcgccgccga aaacctcgcc ccaccggccg     900
   aaggccttat tggacgtgac gatcaagctg gcccgctcat accgggagga caccagctgg     960
   aagaagaggt tggcggcctc gggctcaaac ggaatgtaac cgacttcgtc aaccaccagg    1020
   agcggatagc ggccaaaccg ggtgagttcg gcgtagatgc gcccggcgtg gtgagcctcg    1080
   gcgaaccgtg ctacccattc ggcggcggtg gcgaacagca cccgatgacc ggcctgacac    1140
   gcgcgtatcg ccaggccgac cgcaagatga gtcttcccgg tgccaggcgg ggcccaaaaa    1200
   cacgacgtta tcgcgggcgg tgatgaaatc cagggtgccc agatgtgcga tggtgtcgcg    1260
   tttgaggcca cgagcatgct caaagtcgaa ctcttccaac gacttccgaa ccgggaagcg    1320
   ggcggcgcgg atgcggccct caccaccatg ggactcccgg gctgacactt cccgctgcag    1380
   gcaggcggcc aggtattctt cgtggctcca gttctcggcg cgggcgcgat cggccagccg    1440
   ggacactgac tcacgcaggg tgggagcttt caatgctctt gt                       1482

<210> 19
   <211> 876
   <212> DNA
   <213> Mycobacterium tuberculosis
<220>
   <221> misc_feature
   <222> (204)..(204)
   <223> Unknown
<220>
   <221> misc_feature
   <222> (343)..(343)
   <223> Unknown
<220>
   <221> misc_feature
   <222> (378)..(378)
   <223> Unknown
<220>
   <221> misc_feature
   <222> (870)..(870)
   <223> Unknown
<400> 19
   gaattcggca cgagccggcg atagcttctg ggccgcggcc gaccagatgg ctcgagggtt 60
   cgtgctcggg gccaccgccg ggcgcaccac cctgaccggt gagggcctgc aacacgccga 120
   cggtcactcg ttgctgctgg acgccaccaa cccggcggtg gttgcctacg acccggcctt 180
   cgcctacgaa atcggctaca tcgnggaaag cggactggcc aggatgtgcg gggagaaccc 240
   ggagaacatc ttcttctaca tcaccgtcta caacgagccg tacgtgcagc cgccggagcc 300
   ggagaacttc gatcccgagg gcgtgctggg gggtatctac cgntatcacg cggccaccga 360
   gcaacgcacc aacaaggngc agatcctggc ctccggggta gcgatgcccg cggcgctgcg 420
   ggcagcacag atgctggccg ccgagtggga tgtcgccgcc gacgtgtggt cggtgaccag 480
   ttggggcgag ctaaaccgcg acggggtggt catcgagacc gagaagctcc gccaccccga 540
   tcggccggcg ggcgtgccct acgtgacgag agcgctggag aatgctcggg gcccggtgat 600
   cgcggtgtcg gactggatgc gcgcggtccc cgagcagatc cgaccgtggg tgccgggcac 660
   atacctcacg ttgggcaccg acgggttcgg tttttccgac actcggcccg ccggtcgtcg 720
   ttacttcaac accgacgccg aatcccaggt tggtcgcggt tttgggaggg gttggccggg 780
   tcgacgggtg aatatcgacc cattcggtgc cggtcgtggg ccgcccgccc agttacccgg 840
   attcgacgaa ggtggggggt tgcgcccgan taagtt 876
<210> 20
   <211> 1021
   <212> DNA
   <213> Mycobacterium tuberculosis
<220>
   <221> misc_feature
   <222> (664)..(664)
   <223> Unknown
<220>
   <221> misc_feature
   <222> (1010)..(1010)
   <223> Unknown
<220>
   <221> misc_feature
   <222> (1014)..(1015)
   <223> Unknown
<400> 20
   atccccccgg gctgcaggaa ttcggcacga gagacaaaat tccacgcgtt aatgcaggaa 60
   cagattcata acgaattcac agcggcacaa caatatgtcg cgatcgcggt ttatttcgac 120
   agcgaagacc tgccgcagtt ggcgaagcat ttttacagcc aagcggtcga ggaacgaaac 180
   catgcaatga tgctcgtgca acacctgctc gaccgcgacc ttcgtgtcga aattcccggc 240
   gtagacacgg tgcgaaacca gttcgacaga ccccgcgagg cactggcgct ggcgctcgat 300
   caggaacgca cagtcaccga ccaggtcggt cggctgacag cggtggcccg cgacgagggc 360
   gatttcctcg gcgagcagtt catgcagtgg ttcttgcagg aacagatcga agaggtggcc 420
   ttgatggcaa ccctggtgcg ggttgccgat cgggccgggg ccaacctgtt cgagctagag 480
   aacttcgtcg cacgtgaagt ggatgtggcg ccggccgcat caggcgcccc gcacgctgcc 540
   gggggccgcc tctagatccc tgggggggat cagcgagtgg tcccgttcgc ccgcccgtct 600
   tccagccagg ccttggtgcg gccggggtgg tgagtaccaa tccaggccac cccgacctcc 660
   cggnaaaagt cgatgtcctc gtactcatcg acgttccagg agtacaccgc ccggccctga 720
   gctgccgagc ggtcaacgag ttgcggatat tcctttaacg caggcagtga gggtcccacg 780
   gcggttggcc cgaccgccgt ggccgcactg ctggtcaggt atcggggggt cttggcgagc 840
   aacaacgtcg gcaggagggg tggagcccgc cggatccgca gaccgggggg gcgaaaacga 900
   catcaacacc gcacgggatc gatctgcgga ggggggtgcg ggaataccga accggtgtag 960
   gagcgccagc agttgttttt ccaccagcga agcgttttcg ggtcatcggn ggcnnttaag 1020
   t 1021
<210> 21
   <211> 321
   <212> DNA
   <213> Mycobacterium tuberculosis
<220>
   <221> misc_feature
   <222> (120)..(120)
   <223> unknown
<220>
   <221> misc_feature
   <222> (179)..(179)
   <223> Unknown
<220>
   <221> misc_feature
   <222> (192)..(192)
   <223> Unknown
<220>
   <221> misc_feature
   <222> (219)..(219)
   <223> Unknown
<220>
   <221> misc_feature
   <222> (231)..(231)
   <223> Unknown
<220>
   <221> mist feature
   <222> (236)..(236)
   <223> Unknown
<220>
   <221> misc_feature
   <222> (257)..(257)
   <223> Unknown
<220>
   <221> misc_feature
   <222> (267)..(267)
   <223> Unknown
<220>
   <221> misc_feature
   <222> (282)..(282)
   <223> unknown
<220>
   <221> misc_feature
   <222> (285)..(285)
   <223> Unknown
<220>
   <221> misc_feature
   <222> (291)..(291)
   <223> Unknown
<220>
   <221> misc_feature
   <222> (294)..(294)
   <223> Unknown
<220>
   <221> misc_feature
   <222> (296)..(296)
   <223> Unknown
<220>
   <221> misc_feature
   <222> (303)..(303)
   <223> Unknown
<220>
   <221> misc_feature
   <222> (305)..(305)
   <223> Unknown
<220>
   <221> misc_feature
   <222> (307)..(307)
   <223> Unknown
<220>
   <221> misc_feature
   <222> (312)..(312)
   <223> unknown
<220>
   <221> misc_feature
   <222> (316)..(316)
   <223> Unknown
<400> 21
   cgtgccgacg aacggaagaa cacaaccatg aagatggtga aatcgatcgc cgcaggtctg      60
   accgccgcgg ctgcaatcgg cgccgctgcg gccggtgtga cttcgatcat ggctggcggn     120
   ccggtcgtat accagatgca gccggtcgtc ttcggcgcgc cactgccgtt ggacccggna     180
   tccgcccctg angtcccgac cgccgcccag tggaccagnc tgctcaacag nctcgncgat     240
   cccaacgtgt cgtttgngaa caagggnagt ctggtcgagg gnggnatcgg nggnancgag     300
   ggngngnatc gncgancaca a                                               321
<210> 22
   <211> 373
   <212> DNA
   <213> Mycobacterium tuberculosis
<220>
   <221> misc_feature
   <222> (34)..(34)
   <223> Unknown
<220>
   <221> misc_feature
   <222> (132)..(132)
   <223> Unknown
<220>
   <221> misc_feature
   <222> (159)..(159)
   <223> Unknown
<400> 22
   tcttatcggt tccggttggc gacgggtttt gggngcgggt ggttaacccg ctcggccagc 60
   cgatcgacgg gcgcggagac gtcgactccg atactcggcg cgcgctggag ctccaggcgc 120
   cctcggtggt gnaccggcaa ggcgtgaagg agccgttgna gaccgggatc aaggcgattg 180
   acgcgatgac cccgatcggc cgcgggcagc gccagctgat catcggggac cgcaagaccg 240
   gcaaaaaccg ccgtctgtgt cggacaccat cctcaaacca gcgggaagaa ctgggagtcc 300
   ggtggatccc aagaagcagg tgcgcttgtg tatacgttgg ccatcgggca agaaggggaa 360
   cttaccatcg ccg 373
<210> 23
   <211> 352
   <212> DNA
   <213> Mycobacterium tuberculosis
<220>
   <221> misc_feature
   <222> (272)..(272)
   <223> Unknown
<220>
   <221> misc_feature
   <222> (296)..(296)
   <223> Unknown
<220>
   <221> misc_feature
   <222> (298)..(298)
   <223> Unknown
<220>
   <221> misc_feature
   <222> (311)..(311)
   <223> Unknown
<220>
   <221> misc_feature
   <222> (321)..(321)
   <223> Unknown
<220>
   <221> misc_feature
   <222> (329)..(329)
   <223> Unknown
<220>
   <221> misc_feature
   <222> (335)..(335)
   <223> Unknown
<220>
   <221> misc_feature
   <222> (347)..(347)
   <223> Unknown
<400> 23
   gtgacgccgt gatgggattc ctgggcgggg ccggtccgct ggcggtggtg gatcagcaac     60
   tggttacccg ggtgccgcaa ggctggtcgt ttgctcaggc agccgctgtg ccggtggtgt    120
   tcttgacggc ctggtacggg ttggccgatt tagccgagat caaggcgggc gaatcggtgc    180
   tgatccatgc cggtaccggc ggtgtgggca tggcggctgt gcagctggct cgccagtggg    240
   gcgtggaggt tttcgtcacc gccagccgtg gnaagtggga cacgctgcgc gccatngngt    300
   ttgacgacga nccatatcgg ngattcccnc acatncgaag ttccgangga ga            352
<210> 24
   <211> 726
   <212> DNA
   <213> Mycobacterium tuberculosis
<400> 24
   gaaatccgcg ttcattccgt tcgaccagcg gctggcgata atcgacgaag tgatcaagcc     60
   gcggttcgcg gcgctcatgg gtcacagcga gtaatcagca agttctctgg tatatcgcac    120
   ctagcgtcca gttgcttgcc agatcgcttt cgtaccgtca tcgcatgtac cggttcgcgt    180
   gccgcacgct catgctggcg gcgtgcatcc tggccacggg tgtggcgggt ctcggggtcg    240
   gcgcgcagtc cgcagcccaa accgcgccgg tgcccgacta ctactggtgc ccggggcagc    300
   ctttcgaccc cgcatggggg cccaactggg atccctacac ctgccatgac gacttccacc    360
   gcgacagcga cggccccgac cacagccgcg actaccccgg acccatcctc gaaggtcccg    420
   tgcttgacga tcccggtgct gcgccgccgc ccccggctgc cggtggcggc gcatagcgct    480
   cgttgaccgg gccgcatcag cgaatacgcg tataaacccg ggcgtgcccc cggcaagcta    540
   cgacccccgg cggggcagat ttacgctccc gtgccgatgg atcgcgccgt ccgatgacag    600
   aaaataggcg acggttttgg caaccgcttg gaggacgctt gaagggaacc tgtcatgaac    660
   ggcgacagcg cctccaccat cgacatcgac aaggttgtta cccgcacacc cgttcgccgg    720
   atcgtg                                                               726
<210> 25
   <211> 580
   <212> DNA
   <213> Mycobacterium tuberculosis
<400> 25
   cgcgacgacg acgaacgtcg ggcccaccac cgcctatgcg ttgatgcagg cgaccgggat 60
   ggtcgccgac catatccaag catgctgggt gcccactgag cgaccttttg accagccggg 120
   ctgcccgatg gcggcccggt gaagtcattg cgccggggct tgtgcacctg atgaacccga 180
   atagggaaca ataggggggt gatttggcag ttcaatgtcg ggtatggctg gaaatccaat 240
   ggcggggcat gctcggcgcc gaccaggctc gcgcaggcgg gccagcccga atctggaggg 300
   agcactcaat ggcggcgatg aagccccgga ccggcgacgg tcctttggaa gcaactaagg 360
   aggggcgcgg cattgtgatg cgagtaccac ttgagggtgg cggtcgcctg gtcgtcgagc 420
   tgacacccga cgaagccgcc gcactgggtg acgaactcaa aggcgttact agctaagacc 480
   agcccaacgg cgaatggtcg gcgttacgcg cacaccttcc ggtagatgtc cagtgtctgc 540
   tcggcgatgt atgcccagga gaactcttgg atacagcgct 580
<210> 26
   <211> 160
   <212> DNA
   <213> Mycobacterium tuberculosis
<400> 26
   aacggaggcg ccgggggttt tggcggggcc ggggcggtcg gcggcaacgg cggggccggc 60
   ggtaccgccg ggttgttcgg tgtcggcggg gccggtgggg ccggaggcaa cggcatcgcc 120
   ggtgtcacgg gtacgtcggc cagcacaccg ggtggatccg 160
<210> 27
   <211> 272
   <212> DNA
   <213> Mycobacterium tuberculosis
<400> 27
   gacaccgata cgatggtgat gtacgccaac gttgtcgaca cgctcgaggc gttcacgatc 60
   cagcgcacac ccgacggcgt gaccatcggc gatgcggccc cgttcgcgga ggcggctgcc 120
   aaggcgatgg gaatcgacaa gctgcgggta attcataccg gaatggaccc cgtcgtcgct 180
   gaacgcgaac agtggacgga cggcaacaac acgttggcgt tggcgcccgg tgtcgttgtc 240
   gcctacgagc gcaacgtaca gaccaacgcc cg 272
<210> 28
   <211> 317
   <212> DNA
   <213> Mycobacterium tuberculosis
<400> 28
   gcagccggtg gttctcggac tatctgcgca cggtgacgca gcgcgacgtg cgcgagctga 60
   agcggatcga gcagacggat cgcctgccgc ggttcatgcg ctacctggcc gctatcaccg 120
   cgcaggagct gaacgtggcc gaagcggcgc gggtcatcgg ggtcgacgcg gggacgatcc 180
   gttcggatct ggcgtggttc gagacggtct atctggtaca tcgcctgccc gcctggtcgc 240
   ggaatctgac cgcgaagatc aagaagcggt caaagatcca cgtcgtcgac agtggcttcg 300
   cggcctggtt gcgcggg 317
<210> 29
   <211> 182
   <212> DNA
   <213> Mycobacterium tuberculosis
<400> 29
   gatcgtggag ctgtcgatga acagcgttgc cggacgcgcg gcggccagca cgtcggtgta 60
   gcagcgccgg accacctcgc cggtgggcag catggtgatg accacgtcgg cctcggccac 120
   cgcttcgggc gcgctacgaa acaccgcgac accgtgcgcg gcggcgccgg acgccgccgt 180
   gg 182
<210> 30
   <211> 308
   <212> DNA
   <213> Mycobacterium tuberculosis
<400> 30
   gatcgcgaag tttggtgagc aggtggtcga cgcgaaagtc tgggcgcctg cgaagcgggt 60
   cggcgttcac gaggcgaaga cacgcctgtc cgagctgctg cggctcgtct acggcgggca 120
   gaggttgaga ttgcccgccg cggcgagccg gtagcaaagc ttgtgccgct gcatcctcat 180
   gagactcggc ggttaggcat tgaccatggc gtgtaccgcg tgcccgacga tttggacgct 240
   ccgttgtcag acgacgtgct cgaacgcttt caccggtgaa gcgctacctc atcgacaccc 300
   acgtttgg 308
<210> 31
   <211> 267
   <212> DNA
   <213> Mycobacterium tuberculosis
<400> 31
   ccgacgacga gcaactcacg tggatgatgg tcggcagcgg cattgaggac ggagagaatc        60
   cggccgaagc tgccgcgcgg caagtgctca tagtgaccgg ccgtagaggg ctcccccgat       120
   ggcaccggac tattctggtg tgccgctggc cggtaagagc gggtaaaaga atgtgagggg       180
   acacgatgag caatcacacc taccgagtga tcgagatcgt cgggacctcg cccgacggcg       240
   tcgacgcggc aatccagggc ggtctgg                                           267
<210> 32
   <211> 189
   <212> DNA
   <213> Mycobacterium tuberculosis
<400> 32
   ctcgtgccga aagaatgtga ggggacacga tgagcaatca cacctaccga gtgatcgaga        60
   tcgtcgggac ctcgcccgac ggcgtcgacg cggcaatcca gggcggtctg gcccgagctg       120
   cgcagaccat gcgcgcgctg gactggttcg aagtacagtc aattcgaggc cacctggtcg       180
   acggagcgg                                                               189
<210> 33
   <211> 851
   <212> DNA
   <213> Mycobacterium tuberculosis
<220>
   <221> misc_feature
   <222> (767)..(767)
   <223> Unknown
<400> 33
   ctgcagggtg gcgtggatga gcgtcaccgc ggggcaggcc gagctgaccg ccgcccaggt        60
   ccgggttgct gcggcggcct acgagacggc gtatgggctg acggtgcccc cgccggtgat       120
   cgccgagaac cgtgctgaac tgatgattct gatagcgacc aacctcttgg ggcaaaacac 180
   cccggcgatc gcggtcaacg aggccgaata cggcgagatg tgggcccaag acgccgccgc 240
   gatgtttggc tacgccgcgg cgacggcgac ggcgacggcg acgttgctgc cgttcgagga 300
   ggcgccggag atgaccagcg cgggtgggct cctcgagcag gccgccgcgg tcgaggaggc 360
   ctccgacacc gccgcggcga accagttgat gaacaatgtg ccccaggcgc tgaaacagtt 420
   ggcccagccc acgcagggca ccacgccttc ttccaagctg ggtggcctgt ggaagacggt 480
   ctcgccgcat cggtcgccga tcagcaacat ggtgtcgatg gccaacaacc acatgtcgat 540
   gaccaactcg ggtgtgtcga tgaccaacac cttgagctcg atgttgaagg gctttgctcc 600
   ggcggcggcc gcccaggccg tgcaaaccgc ggcgcaaaac ggggtccggg cgatgagctc 660
   gctgggcagc tcgctgggtt cttcgggtct gggcggtggg gtggccgcca acttgggtcg 720
   ggcggcctcg gtacggtatg gtcaccggga tggcggaaaa tatgcanagt ctggtcggcg 780
   gaacggtggt ccggcgtaag gtttaccccc gttttctgga tgcggtgaac ttcgtcaacg 840
   gaaacagtta c 851
<210> 34
   <211> 254
   <212> DNA
   <213> Mycobacterium tuberculosis
<400> 34
   gatcgatcgg gcggaaattt ggaccagatt cgcctccggc gataacccaa tcaatcgaac 60
   ctagatttat tccgtccagg ggcccgagta atggctcgca ggagaggaac cttactgctg 120
   cgggcacctg tcgtaggtcc tcgatacggc ggaaggcgtc gacattttcc accgacaccc 180
   ccatccaaac gttcgagggc cactccagct tgtgagcgag gcgacgcagt cgcaggctgc 240
   gcttggtcaa gatc 254
<210> 35
   <211> 408
   <212> DNA
   <213> Mycobacterium tuberculosis
<220>
   <221> misc_feature
   <222> (265)..(265)
   <223> Unknown
<400> 35
   cggcacgagg atcctgaccg aagcggccgc cgccaaggcg aagtcgctgt tggaccagga 60
   gggacgggac gatctggcgc tgcggatcgc ggttcagccg ggggggtgcg ctggattgcg 120
   ctataacctt ttcttcgacg accggacgct ggatggtgac caaaccgcgg agttcggtgg 180
   tgtcaggttg atcgtggacc ggatgagcgc gccgtatgtg gaaggcgcgt cgatcgattt 240
   cgtcgacact attgagaagc aaggnttcac catcgacaat cccaacgcca ccggctcctg 300
   cgcgtgcggg gattcgttca actgataaaa cgctagtacg accccgcggt gcgcaacacg 360
   tacgagcaca ccaagacctg accgcgctgg aaaagcaact gagcgatg 408
<210> 36
   <211> 181
   <212> DNA
   <213> Mycobacterium tuberculosis
<220>
   <221> misc_feature
   <222> (125)..(125)
   <223> Unknown
<220>
   <221> misc_feature
   <222> (143)..(143)
   <223> Unknown
<400> 36
   gcggtgtcgg cggatccggc gggtggttga acggcaacgg cggggccggc ggggccggcg 60
   ggaccggcgc taacggtggt gccggcggca acgcctggtt gttcggggcc ggcgggtccg 120
   gcggngccgg caccaatggt ggngtcggcg ggtccggcgg atttgtctac ggcaacggcg 180
   g 181
<210> 37
   <211> 290
   <212> DNA
   <213> Mycobacterium tuberculosis
<220>
   <221> misc_feature
   <222> (230)..(230)
   <223> Unknown
<220>
   <221> misc_feature
   <222> (266)..(266)
   <223> Unknown
<400> 37
   gcggtgtcgg cggatccggc gggtggttga acggcaacgg cggtgtcggc ggccggggcg 60
   gcgacggcgt ctttgccggt gccggcggcc agggcggcct cggtgggcag ggcggcaatg 120
   gcggcggctc caccggcggc aacggcggtc ttggcggcgc gggcggtggc ggaggcaacg 180
   ccccggacgg cggcttcggt ggcaacggcg gtaagggtgg ccagggcggn attggcggcg 240
   gcactcagag cgcgaccggc ctcggnggtg acggcggtga cggcggtgac 290
<210> 38
   <211> 34
   <212> DNA
   <213> Mycobacterium tuberculosis
<220>
   <221> misc_feature
   <222> (16)..(16)
   <223> Unknown
<400> 38
   gatccagtgg catggngggt gtcagtggaa gcat 34
<210> 39
   <211> 155
   <212> DNA
   <213> Mycobacterium tuberculosis
<220>
   <221> misc_feature
   <222> (141)..(141)
   <223> Unknown
<220>
   <221> misc_feature
   <222> (142)..(142)
   <223> n is a, c, g, or t
<400> 39
   gatcgctgct cgtccccccc ttgccgccga cgccaccggt cccaccgtta ccgaacaagc 60
   tggcgtggtc gccagcaccc ccggcaccgc cgacgccgga gtcgaacaat ggcaccgtcg 120
   tatccccacc attgccgccg gncccaccgg caccg 155
<210> 40
   <211> 53
   <212> DNA
   <213> Mycobacterium tuberculosis
<220>
   <221> misc_feature
   <222> (38)..(38)
   <223> unknown
<400> 40
   atggcgttca cggggcgccg gggaccgggc agcccggngg ggccgggggg tgg 53
<210> 41
   <211> 132
   <212> DNA
   <213> Mycobacterium tuberculosis
<220>
   <221> misc_feature
   <222> (98)..(98)
   <223> Unknown
<400> 41
   gatccaccgc gggtgcagac ggtgcccgcg gcgccacccc gaccagcggc ggcaacggcg 60
   gcaccggcgg caacggcgcg aacgccaccg tcgtcggngg ggccggcggg gccggcggca 120
   agggcggcaa cg 132
<210> 42
   <211> 132
   <212> DNA
   <213> Mycobacterium tuberculosis
<220>
   <221> misc_feature
   <222> (14)..(14)
   <223> Unknown
<220>
   <221> misc_feature
   <222> (19)..(19)
   <223> Unknown
<220>
   <221> misc_feature
   <222> (41)..(41)
   <223> Unknown
<220>
   <221> misc_feature
   <222> (55)..(55)
   <223> Unknown
<220>
   <221> misc_feature
   <222> (63)..(63)
   <223> Unknown
<220>
   <221> misc_feature
   <222> (79)..(79)
   <223> Unknown
<220>
   <221> misc_feature
   <222> (84)..(84)
   <223> Unknown
<220>
   <221> misc_feature
   <222> (124)..(124)
   <223> Unknown
<400> 42
   gatcggcggc cggnacggnc ggggacggcg gcaagggcgg naacgggggc gccgnagcca 60
   ccngccaaga atcctccgng tccnccaatg gcgcgaatgg cggacagggc ggcaacggcg 120
   gcancggcgg ca 132
<210> 43
   <211> 702
   <212> DNA
   <213> Mycobacterium tuberculosis
<220>
   <221> misc_feature
   <222> (325)..(325)
   <223> Unknown
<220>
   <221> misc_feature
   <222> (328)..(328)
   <223> Unknown
<220>
   <221> misc_feature
   <222> (348)..(348)
   <223> Unknown
<220>
   <221> misc_feature
   <222> (353)..(353)
   <223> Unknown
<220>
   <221> misc_feature
   <222> (442)..(442)
   <223> Unknown
<220>
   <221> misc_feature
   <222> (659)..(659)
   <223> Unknown
<220>
   <221> misc_feature
   <222> (679)..(679)
   <223> Unknown
<400> 43
   cggcacgagg atcggtaccc cgcggcatcg gcagctgccg attcgccggg tttccccacc 60
   cgaggaaagc cgctaccaga tggcgctgcc gaagtagggc gatccgttcg cgatgccggc 120
   atgaacgggc ggcatcaaat tagtgcagga acctttcagt ttagcgacga taatggctat 180
   agcactaagg aggatgatcc gatatgacgc agtcgcagac cgtgacggtg gatcagcaag 240
   agattttgaa cagggccaac gaggtggagg ccccgatggc ggacccaccg actgatgtcc 300
   ccatcacacc gtgcgaactc acggnggnta aaaacgccgc ccaacagntg gtnttgtccg 360
   ccgacaacat gcgggaatac ctggcggccg gtgccaaaga gcggcagcgt ctggcgacct 420
   cgctgcgcaa cgcggccaag gngtatggcg aggttgatga ggaggctgcg accgcgctgg 480
   acaacgacgg cgaaggaact gtgcaggcag aatcggccgg ggccgtcgga ggggacagtt 540
   cggccgaact aaccgatacg ccgagggtgg ccacggccgg tgaacccaac ttcatggatc 600
   tcaaagaagc ggcaaggaag ctcgaaacgg gcgaccaagg cgcatcgctc gcgcactgng 660
   gggatgggtg gaacacttnc accctgacgc tgcaaggcga cg 702
<210> 44
   <211> 298
   <212> DNA
   <213> Mycobacterium tuberculosis
<400> 44
   gaagccgcag cgctgtcggg cgacgtggcg gtcaaagcgg catcgctcgg tggcggtgga 60
   ggcggcgggg tgccgtcggc gccgttggga tccgcgatcg ggggcgccga atcggtgcgg 120
   cccgctggcg ctggtgacat tgccggctta ggccagggaa gggccggcgg cggcgccgcg 180
   ctgggcggcg gtggcatggg aatgccgatg ggtgccgcgc atcagggaca agggggcgcc 240
   aagtccaagg gttctcagca ggaagacgag gcgctctaca ccgaggatcc tcgtgccg 298
<210> 45
   <211> 1058
   <212> DNA
   <213> Mycobacterium tuberculosis
<400> 45
   cggcacgagg atcgaatcgc gtcgccggga gcacagcgtc gcactgcacc agtggaggag 60
   ccatgaccta ctcgccgggt aaccccggat acccgcaagc gcagcccgca ggctcctacg 120
   gaggcgtcac accctcgttc gcccacgccg atgagggtgc gagcaagcta ccgatgtacc 180
   tgaacatcgc ggtggcagtg ctcggtctgg ctgcgtactt cgccagcttc ggcccaatgt 240
   tcaccctcag taccgaactc ggggggggtg atggcgcagt gtccggtgac actgggctgc 300
   cggtcggggt ggctctgctg gctgcgctgc ttgccggggt ggttctggtg cctaaggcca 360
   agagccatgt gacggtagtt gcggtgctcg gggtactcgg cgtatttctg atggtctcgg 420
   cgacgtttaa caagcccagc gcctattcga ccggttgggc attgtgggtt gtgttggctt 480
   tcatcgtgtt ccaggcggtt gcggcagtcc tggcgctctt ggtggagacc ggcgctatca 540
   ccgcgccggc gccgcggccc aagttcgacc cgtatggaca gtacgggcgg tacgggcagt 600
   acgggcagta cggggtgcag ccgggtgggt actacggtca gcagggtgct cagcaggccg 660
   cgggactgca gtcgcccggc ccgcagcagt ctccgcagcc tcccggatat gggtcgcagt 720
   acggcggcta ttcgtccagt ccgagccaat cgggcagtgg atacactgct cagcccccgg 780
   cccagccgcc ggcgcagtcc gggtcgcaac aatcgcacca gggcccatcc acgccaccta 840
   ccggctttcc gagcttcagc ccaccaccac cggtcagtgc cgggacgggg tcgcaggctg 900
   gttcggctcc agtcaactat tcaaacccca gcgggggcga gcagtcgtcg tcccccgggg 960
   gggcgccggt ctaaccgggc gttcccgcgt ccggtcgcgc gtgtgcgcga agagtgaaca 1020
   gggtgtcagc aagcgcggac gatcctcgtg ccgaattc 1058
<210> 46
   <211> 327
   <212> DNA
   <213> Mycobacterium tuberculosis
<400> 46
   cggcacgaga gaccgatgcc gctaccctcg cgcaggaggc aggtaatttc gagcggatct 60
   ccggcgacct gaaaacccag atcgaccagg tggagtcgac ggcaggttcg ttgcagggcc 120
   agtggcgcgg cgcggcgggg acggccgccc aggccgcggt ggtgcgcttc caagaagcag 180
   ccaataagca gaagcaggaa ctcgacgaga tctcgacgaa tattcgtcag gccggcgtcc 240
   aatactcgag ggccgacgag gagcagcagc aggcgctgtc ctcgcaaatg ggcttctgac 300
   ccgctaatac gaaaagaaac ggagcaa 327
<210> 47
   <211> 170
   <212> DNA
   <213> Mycobacterium tuberculosis
<220>
   <221> misc_feature
   <222> (93)..(93)
   <223> Unknown
<220>
   <221> misc_feature
   <222> (160)..(160)
   <223> Unknown
<400> 47
   cggtcgcgat gatggcgttg tcgaacgtga ccgattctgt accgccgtcg ttgagatcaa 60
   ccaacaacgt gttggcgtcg gcaaatgtgc cgnacccgtg gatctcggtg atcttgttct 120
   tcttcatcag gaagtgcaca ccggccaccc tgccctcggn tacctttcgg 170
<210> 48
   <211> 127
   <212> DNA
   <213> Mycobacterium tuberculosis
<400> 48
   gatccggcgg cacggggggt gccggcggca gcaccgctgg cgctggcggc aacggcgggg 60
   ccgggggtgg cggcggaacc ggtgggttgc tcttcggcaa cggcggtgcc ggcgggcacg 120
   gggccgt 127
<210> 49
   <211> 81
   <212> DNA
   <213> Mycobacterium tuberculosis
<400> 49
   cggcggcaag ggcggcaccg ccggcaacgg gagcggcgcg gccggcggca acggcggcaa 60
   cggcggctcc ggcctcaacg g 81
<210> 50
   <211> 149
   <212> DNA
   <213> Mycobacterium tuberculosis
<220>
   <221> misc_feature
   <222> (77)..(77)
   <223> Unknown
<220>
   <221> misc_feature
   <222> (113)..(113)
   <223> Unknown
<400> 50
   gatcagggct ggccggctcc ggccagaagg gcggtaacgg aggagctgcc ggattgtttg 60
   gcaacggcgg ggccggnggt gccggcgcgt ccaaccaagc cggtaacggc ggngccggcg 120
   gaaacggtgg tgccggtggg ctgatctgg 149
<210> 51
   <211> 355
   <212> DNA
   <213> Mycobacterium tuberculosis
<220>
   <221> misc_feature
   <222> (67)..(67)
   <223> Unknown
<400> 51
   cggcacgaga tcacacctac cgagtgatcg agatcgtcgg gacctcgccc gacggtgtcg 60
   acgcggnaat ccagggcggt ctggcccgag ctgcgcagac catgcgcgcg ctggactggt 120
   tcgaagtaca gtcaattcga ggccacctgg tcgacggagc ggtcgcgcac ttccaggtga 180
   ctatgaaagt cggcttccgc ctggaggatt cctgaacctt caagcgcggc cgataactga 240
   ggtgcatcat taagcgactt ttccagaaca tcctgacgcg ctcgaaacgc ggttcagccg 300
   acggtggctc cgccgaggcg ctgcctccaa aatccctgcg acaattcgtc ggcgg 355
<210> 52
   <211> 999
   <212> DNA
   <213> Mycobacterium tuberculosis
<400> 52
   atgcatcacc atcaccatca catgcatcag gtggacccca acttgacacg tcgcaaggga      60
   cgattggcgg cactggctat cgcggcgatg gccagcgcca gcctggtgac cgttgcggtg     120
   cccgcgaccg ccaacgccga tccggagcca gcgcccccgg tacccacaac ggccgcctcg     180
   ccgccgtcga ccgctgcagc gccacccgca ccggcgacac ctgttgcccc cccaccaccg     240
   gccgccgcca acacgccgaa tgcccagccg ggcgatccca acgcagcacc tccgccggcc     300
   gacccgaacg caccgccgcc acctgtcatt gccccaaacg caccccaacc tgtccggatc     360
   gacaacccgg ttggaggatt cagcttcgcg ctgcctgctg gctgggtgga gtctgacgcc     420
   gcccacttcg actacggttc agcactcctc agcaaaacca ccggggaccc gccatttccc     480
   ggacagccgc cgccggtggc caatgacacc cgtatcgtgc tcggccggct agaccaaaag     540
   ctttacgcca gcgccgaagc caccgactcc aaggccgcgg cccggttggg ctcggacatg     600
   ggtgagttct atatgcccta cccgggcacc cggatcaacc aggaaaccgt ctcgctcgac     660
   gccaacgggg tgtctggaag cgcgtcgtat tacgaagtca agttcagcga tccgagtaag     720
   ccgaacggcc agatctggac gggcgtaatc ggctcgcccg cggcgaacgc accggacgcc     780
   gggccccctc agcgctggtt tgtggtatgg ctcgggaccg ccaacaaccc ggtggacaag     840
   ggcgcggcca aggcgctggc cgaatcgatc cggcctttgg tcgccccgcc gccggcgccg     900
   gcaccggctc ctgcagagcc cgctccggcg ccggcgccgg ccggggaagt cgctcctacc     960
   ccgacgacac cgacaccgca gcggacctta ccggcctga                            999
<210> 53
   <211> 332
   <212> PRT
   <213> Mycobacterium tuberculosis
<400> 53
   Met His His His His His His Met His Gln Val Asp Pro Asn Leu Thr
   1               5                   10                  15
   Arg Arg Lys Gly Arg Leu Ala Ala Leu Ala Ile Ala Ala Met Ala Ser
               20                  25                  30
   Ala Ser Leu Val Thr Val Ala Val Pro Ala Thr Ala Asn Ala Asp Pro
           35                  40                  45
   Glu Pro Ala Pro Pro Val Pro Thr Thr Ala Ala Ser Pro Pro Ser Thr
       50                  55                  60
   Ala Ala Ala Pro Pro Ala Pro Ala Thr Pro Val Ala Pro Pro Pro Pro
   65                  70                  75                  80
   Ala Ala Ala Asn Thr Pro Asn Ala Gln Pro Gly Asp Pro Asn Ala Ala
                   85                  90                  95
   Pro Pro Pro Ala Asp Pro Asn Ala Pro Pro Pro Pro Val Ile Ala Pro
               100                 105                 110
   Asn Ala Pro Gln Pro Val Arg Ile Asp Asn Pro Val Gly Gly Phe Ser
           115                 120                 125
   Phe Ala Leu Pro Ala Gly Trp Val Glu Ser Asp Ala Ala His Phe Asp
       130                 135                 140
   Tyr Gly Ser Ala Leu Leu Ser Lys Thr Thr Gly Asp Pro Pro Phe Pro
   145                 150                 155                 160
   Gly Gln Pro Pro Pro Val Ala Asn Asp Thr Arg Ile Val Leu Gly Arg
                   165                 170                 175
   Leu Asp Gln Lys Leu Tyr Ala Ser Ala Glu Ala Thr Asp Ser Lys Ala
               180                 185                 190
   Ala Ala Arg Leu Gly Ser Asp Met Gly Glu Phe Tyr Met Pro Tyr Pro
           195                 200                 205
   Gly Thr Arg Ile Asn Gln Glu Thr Val Ser Leu Asp Ala Asn Gly Val
       210                 215                 220
   Ser Gly Ser Ala Ser Tyr Tyr Glu Val Lys Phe Ser Asp Pro Ser Lys
   225                 230                 235                 240
   Pro Asn Gly Gln Ile Trp Thr Gly Val Ile Gly Ser Pro Ala Ala Asn
                   245                 250                 255
   Ala Pro Asp Ala Gly Pro Pro Gln Arg Trp Phe Val Val Trp Leu Gly
               260                 265                 270
   Thr Ala Asn Asn Pro Val Asp Lys Gly Ala Ala Lys Ala Leu Ala Glu
           275                 280                 285
   Ser Ile Arg Pro Leu Val Ala Pro Pro Pro Ala Pro Ala Pro Ala Pro
       290                 295                 300
   Ala Glu Pro Ala Pro Ala Pro Ala Pro Ala Gly Glu Val Ala Pro Thr
   305 310   315 320
   Pro Thr Thr Pro Thr Pro Gln Arg Thr Leu Pro Ala
   325 330
<210> 54
   <211> 20
   <212> PRT
   <213> Mycobacterium tuberculosis
<220>
   <221> MISC_FEATURE
   <222> (11)..(11)
   <223> Unknown
<400> 54
   Asp Pro Val Asp Ala Val Ile Asn Thr Thr Xaa Asn Tyr Gly Gln Val
   1 5 10 15
   Val Ala Ala Leu
   20
<210> 55
   <211> 15
   <212> PRT
   <213> Mycobacterium tuberculosis
<400> 55
   Ala Val Glu Ser Gly Met Leu Ala Leu Gly Thr Pro Ala Pro Ser
   1 5 10 15
<210> 56
   <211> 19
   <212> PRT
   <213> Mycobacterium tuberculosis
<400> 56
   Ala Ala Met Lys Pro Arg Thr Gly Asp Gly Pro Leu Glu Ala Ala Lys
   1 5 10 15
   Glu Gly Arg
<210> 57
   <211> 15
   <212> PRT
   <213> Mycobacterium tuberculosis
<400> 57
   Tyr Tyr Trp Cys Pro Gly Gln Pro Phe Asp Pro Ala Trp Gly Pro
   1 5 10 15
<210> 58
   <211> 14
   <212> PRT
   <213> Mycobacterium tuberculosis
<220>
   <221> MISC_FEATURE
   <222> (12)..(12)
   <223> Unknown
<400> 58
   Asp Ile Gly Ser Glu Ser Thr Glu Asp Gln Gln Xaa Ala Val
   1 5 10
<210> 59
   <211> 13
   <212> PRT
   <213> Mycobacterium tuberculosis
<220>
   <221> MISC_FEATURE
   <222> (8)..(8)
   <223> Unknown
<220>
   <221> MISC_FEATURE
   <222> (10)..(10)
   <223> Unknown
<400> 59
   Ala Glu Glu Ser Ile Ser Thr Xaa Glu Xaa Ile Val Pro
   1 5 10
<210> 60
   <211> 17
   <212> PRT
   <213> Mycobacterium tuberculosis
<400> 60
   Asp Pro Glu Pro Ala Pro Pro Val Pro Thr Ala Ala Ala Ala Pro Pro
   1 5 10 15
   Ala
<210> 61
   <211> 15
   <212> PRT
   <213> Mycobacterium tuberculosis
<220>
   <221> MISC_FEATURE
   <222> (6)..(6)
   <223> Unknown
<400> 61
   Ala Pro Lys Thr Tyr Xaa Glu Glu Leu Lys Gly Thr Asp Thr Gly
   1               5                   10                  15
<210> 62
   <211> 30
   <212> PRT
   <213> Mycobacterium tuberculosis
<400> 62
   Asp Pro Ala Ser Ala Pro Asp Val Pro Thr Ala Ala Gln Gln Thr Ser
   1               5                   10                  15
   Leu Leu Asn Asn Leu Ala Asp Pro Asp Val Ser Phe Ala Asp
               20                  25                  30
<210> 63
   <211> 24
   <212> PRT
   <213> Mycobacterium tuberculosis
<400> 63
   Gly Cys Gly Asp Arg Ser Gly Gly Asn Leu Asp Gln Ile Arg Leu Arg
   1               5                   10                  15
   Arg Asp Arg Ser Gly Gly Asn Leu
               20
<210> 64
   <211> 187
   <212> PRT
   <213> Mycobacterium tuberculosis
<220>
   <221> MISC_FEATURE
   <222> (187)..(187)
   <223> Unknown
<400> 64
   Thr Gly Ser Leu Asn Gln Thr His Asn Arg Arg Ala Asn Glu Arg Lys
   1               5                   10                  15
   Asn Thr Thr Met Lys Met Val Lys Ser Ile Ala Ala Gly Leu Thr Ala
               20                  25                  30
   Ala Ala Ala Ile Gly Ala Ala Ala Ala Gly Val Thr Ser Ile Met Ala
           35                  40                  45
   Gly Gly Pro Val Val Tyr Gln Met Gln Pro Val Val Phe Gly Ala Pro
       50                  55                  60
   Leu Pro Leu Asp Pro Ala Ser Ala Pro Asp Val Pro Thr Ala Ala Gln
   65                  70                  75                  80
   Leu Thr Ser Leu Leu Asn Ser Leu Ala Asp Pro Asn Val Ser Phe Ala
                   85                  90                  95
   Asn Lys Gly Ser Leu Val Glu Gly Gly Ile Gly Gly Thr Glu Ala Arg
               100                 105                 110
   Ile Ala Asp His Lys Leu Lys Lys Ala Ala Glu His Gly Asp Leu Pro
           115                 120                 125
   Leu Ser Phe Ser Val Thr Asn Ile Gln Pro Ala Ala Ala Gly Ser Ala
       130                 135                 140
   Thr Ala Asp Val Ser Val Ser Gly Pro Lys Leu Ser Ser Pro Val Thr
   145                 150                 155                 160
   Gln Asn Val Thr Phe Val Asn Gln Gly Gly Trp Met Leu Ser Arg Ala
                   165                 170                 175
   Ser Ala Met Glu Leu Leu Gln Ala Ala Gly Xaa
               180                 185

<210> 65
   <211> 148
   <212> PRT
   <213> Mycobacterium tuberculosis
<400> 65
   Asp Glu Val Thr Val Glu Thr Thr Ser Val Phe Arg Ala Asp Phe Leu
   1 5 10 15
   Ser Glu Leu Asp Ala Pro Ala Gln Ala Gly Thr Glu Ser Ala Val Ser
   20 25 30
   Gly Val Glu Gly Leu Pro Pro Gly Ser Ala Leu Leu Val Val Lys Arg
   35 40 45
   Gly Pro Asn Ala Gly Ser Arg Phe Leu Leu Asp Gln Ala Ile Thr Ser
   50 55 60
   Ala Gly Arg His Pro Asp Ser Asp Ile Phe Leu Asp Asp Val Thr Val
   65 70 75 80
   Ser Arg Arg His Ala Glu Phe Arg Leu Glu Asn Asn Glu Phe Asn Val
   85 90 95
   Val Asp Val Gly Ser Leu Asn Gly Thr Tyr Val Asn Arg Glu Pro Val
   100 105 110
   Asp Ser Ala Val Leu Ala Asn Gly Asp Glu Val Gln Ile Gly Lys Leu
   115 120 125
   Arg Leu Val Phe Leu Thr Gly Pro Lys Gln Gly Glu Asp Asp Gly Ser
   130 135 140
   Thr Gly Gly Pro
   145
<210> 66
   <211> 230
   <212> PRT
   <213> Mycobacterium tuberculosis
<220>
   <221> MISC_FEATURE
   <222> (181)..(181)
   <223> Unknown
<400> 66
   Thr Ser Asn Arg Pro Ala Arg Arg Gly Arg Arg Ala Pro Arg Asp Thr
   1               5                   10                  15
   Gly Pro Asp Arg Ser Ala Ser Leu Ser Leu Val Arg His Arg Arg Gln
               20                  25                  30
   Gln Arg Asp Ala Leu Cys Leu Ser Ser Thr Gln Ile Ser Arg Gln Ser
           35                  40                  45
   Asn Leu Pro Pro Ala Ala Gly Gly Ala Ala Asn Tyr Ser Arg Arg Asn
       50                  55                  60
   Phe Asp Val Arg Ile Lys Ile Phe Met Leu Val Thr Ala Val Val Leu
   65                  70                  75                  80
   Leu Cys Cys Ser Gly Val Ala Thr Ala Ala Pro Lys Thr Tyr Cys Glu
                   85                  90                  95
   Glu Leu Lys Gly Thr Asp Thr Gly Gln Ala Cys Gln Ile Gln Met Ser
               100                 105                 110
   Asp Pro Ala Tyr Asn Ile Asn Ile Ser Leu Pro Ser Tyr Tyr Pro Asp
           115                 120                 125
   Gln Lys Ser Leu Glu Asn Tyr Ile Ala Gln Thr Arg Asp Lys Phe Leu
       130                 135                 140
   Ser Ala Ala Thr Ser Ser Thr Pro Arg Glu Ala Pro Tyr Glu Leu Asn
   145                 150                 155                 160
   Ile Thr Ser Ala Thr Tyr Gln Ser Ala Ile Pro Pro Arg Gly Thr Gln
                   165                 170                 175
   Ala Val Val Leu Xaa Val Tyr His Asn Ala Gly Gly Thr His Pro Thr
               180                 185                 190
   Thr Thr Tyr Lys Ala Phe Asp Trp Asp Gln Ala Tyr Arg Lys Pro Ile
           195                 200                 205
   Thr Tyr Asp Thr Leu Trp Gln Ala Asp Thr Asp Pro Leu Pro Val Val
       210                 215                 220
   Phe Pro Ile Val Ala Arg
   225                 230
<210> 67
   <211> 132
   <212> PRT
   <213> Mycobacterium tuberculosis
<400> 67
   Thr Ala Ala Ser Asp Asn Phe Gln Leu Ser Gln Gly Gly Gln Gly Phe
   1               5                   10                  15
   Ala Ile Pro Ile Gly Gln Ala Met Ala Ile Ala Gly Gln Ile Arg Ser
               20                  25                  30
   Gly Gly Gly Ser Pro Thr Val His Ile Gly Pro Thr Ala Phe Leu Gly
           35                  40                  45
   Leu Gly Val Val Asp Asn Asn Gly Asn Gly Ala Arg Val Gln Arg Val
       50                  55                  60
   Val Gly Ser Ala Pro Ala Ala Ser Leu Gly Ile Ser Thr Gly Asp Val
   65                  70                  75                  80
   Ile Thr Ala Val Asp Gly Ala Pro Ile Asn Ser Ala Thr Ala Met Ala
                   85                  90                  95
   Asp Ala Leu Asn Gly His His Pro Gly Asp Val Ile Ser Val Asn Trp
               100                 105                 110
   Gln Thr Lys Ser Gly Gly Thr Arg Thr Gly Asn Val Thr Leu Ala Glu
           115                 120                 125
   Gly Pro Pro Ala
       130
<210> 68
   <211> 100
   <212> PRT
   <213> Mycobacterium tuberculosis
<220>
   <221> MISC_FEATURE
   <222> (64)..(64)
   <223> Unknown
<220>
   <221> MISC_FEATURE
   <222> (75)..(75)
   <223> Unknown
<220>
   <221> MISC_FEATURE
   <222> (79)..(79)
   <223> Unknown
<220>
   <221> MISC_FEATURE
   <222> (85)..(85)
   <223> Unknown
<220>
   <221> MISC_FEATURE
   <222> (88)..(89)
   <223> Unknown
<400> 68
   Val Pro Leu Arg Ser Pro Ser Met Ser Pro Ser Lys Cys Leu Ala Ala
   1 5 10 15
   Ala Gln Arg Asn Pro Val Ile Arg Arg Arg Arg Leu Ser Asn Pro Pro
   20 25 30
   Pro Arg Lys Tyr Arg Ser Met Pro Ser Pro Ala Thr Ala Ser Ala Gly
   35 40 45
   Met Ala Arg Val Arg Arg Arg Ala Ile Trp Arg Gly Pro Ala Thr Xaa
   50 55 60
   Ser Ala Gly Met Ala Arg Val Arg Arg Trp Xaa Val Met Pro Xaa Val
   65 70 75 80
   Ile Gln Ser Thr Xaa Ile Arg Xaa Xaa Gly Pro Phe Asp Asn Arg Gly
   85 90 95
   Ser Glu Arg Lys
   100
<210> 69
   <211> 163
   <212> PRT
   <213> Mycobacterium tuberculosis
<220>
   <221> MISC_FEATURE
   <222> (39)..(39)
   <223> unknown
<220>
   <221> MISC_FEATURE
   <222> (41)..(41)
   <223> Unknown
<220>
   <221> MISC_FEATURE
   <222> (45)..(45)
   <223> Unknown
<220>
   <221> MISC_FEATURE
   <222> (147)..(147)
   <223> Unknown
<400> 69
   Met Thr Asp Asp Ile Leu Leu Ile Asp Thr Asp Glu Arg Val Arg Thr
   1               5                   10                  15
   Leu Thr Leu Asn Arg Pro Gln Ser Arg Asn Ala Leu Ser Ala Ala Leu
               20                  25                  30
   Arg Asp Arg Phe Phe Ala Xaa Leu Xaa Asp Ala Glu Xaa Asp Asp Asp
           35                  40                  45
   Ile Asp Val Val Ile Leu Thr Gly Ala Asp Pro Val Phe Cys Ala Gly
       50                  55                  60
   Leu Asp Leu Lys Val Ala Gly Arg Ala Asp Arg Ala Ala Gly His Leu
   65                  70                  75                  80
   Thr Ala Val Gly Gly His Asp Gln Ala Gly Asp Arg Arg Asp Gln Arg
                   85                  90                  95
   Arg Arg Gly His Arg Arg Ala Arg Thr Gly Ala Val Leu Arg His Pro
               100                 105                 110
   Asp Arg Leu Arg Ala Arg Pro Leu Arg Arg His Pro Arg Pro Gly Gly
           115                 120                 125
   Ala Ala Ala His Leu Gly Thr Gln Cys Val Leu Ala Ala Lys Gly Arg
       130                 135                 140
   His Arg Xaa Gly Pro Val Asp Glu Pro Asp Arg Arg Leu Pro Val Arg
   145                 150                 155                 160
   Asp Arg Arg
<210> 70
   <211> 344
   <212> PRT
   <213> Mycobacterium tuberculosis
<400> 70
   Met Lys Phe Val Asn His Ile Glu Pro Val Ala Pro Arg Arg Ala Gly
   1 5 10 15
   Gly Ala Val Ala Glu Val Tyr Ala Glu Ala Arg Arg Glu Phe Gly Arg
   20 25 30
   Leu Pro Glu Pro Leu Ala Met Leu Ser Pro Asp Glu Gly Leu Leu Thr
   35 40 45
   Ala Gly Trp Ala Thr Leu Arg Glu Thr Leu Leu Val Gly Gln Val Pro
   50 55 60
   Arg Gly Arg Lys Glu Ala Val Ala Ala Ala Val Ala Ala Ser Leu Arg
   65 70 75 80
   Cys Pro Trp Cys Val Asp Ala His Thr Thr Met Leu Tyr Ala Ala Gly
   85 90 95
   Gln Thr Asp Thr Ala Ala Ala Ile Leu Ala Gly Thr Ala Pro Ala Ala
   100 105 110
   Gly Asp Pro Asn Ala Pro Tyr Val Ala Trp Ala Ala Gly Thr Gly Thr
   115 120 125
   Pro Ala Gly Pro Pro Ala Pro Phe Gly Pro Asp Val Ala Ala Glu Tyr
   130 135 140
   Leu Gly Thr Ala Val Gln Phe His Phe Ile Ala Arg Leu Val Leu Val
   145 150 155 160
   Leu Leu Asp Glu Thr Phe Leu Pro Gly Gly Pro Arg Ala Gln Gln Leu
   165 170 175
   Met Arg Arg Ala Gly Gly Leu Val Phe Ala Arg Lys Val Arg Ala Glu
   180 185 190
   His Arg Pro Gly Arg Ser Thr Arg Arg Leu Glu Pro Arg Thr Leu Pro
   195 200 205
   Asp Asp Leu Ala Trp Ala Thr Pro Ser Glu Pro Ile Ala Thr Ala Phe
   210 215 220
   Ala Ala Leu Ser His His Leu Asp Thr Ala Pro His Leu Pro Pro Pro
   225 230 235 240
   Thr Arg Gln Val Val Arg Arg Val Val Gly Ser Trp His Gly Glu Pro
   245 250 255
   Met Pro Met Ser Ser Arg Trp Thr Asn Glu His Thr Ala Glu Leu Pro
   260 265 270
   Ala Asp Leu His Ala Pro Thr Arg Leu Ala Leu Leu Thr Gly Leu Ala
   275 280 285
   Pro His Gln Val Thr Asp Asp Asp Val Ala Ala Ala Arg Ser Leu Leu
   290 295 300
   Asp Thr Asp Ala Ala Leu Val Gly Ala Leu Ala Trp Ala Ala Phe Thr
   305 310 315 320
   Ala Ala Arg Arg Ile Gly Thr Trp Ile Gly Ala Ala Ala Glu Gly Gln
   325 330 335
   Val Ser Arg Gln Asn Pro Thr Gly
   340
<210> 71
   <211> 485
   <212> PRT
   <213> Mycobacterium tuberculosis <400> 71

<210> 72
   <211> 267
   <212> PRT
   <213> Mycobacterium tuberculosis
<400> 72
   Gly Val Ile Val Leu Asp Leu Glu Pro Arg Gly Pro Leu Pro Thr Glu
   1               5                   10                  15
   Ile Tyr Trp Arg Arg Arg Gly Leu Ala Leu Gly Ile Ala Val Val Val
               20                  25                  30
   Val Gly Ile Ala Val Ala Ile Val Ile Ala Phe Val Asp Ser Ser Ala
           35                  40                  45
   Gly Ala Lys Pro Val Ser Ala Asp Lys Pro Ala Ser Ala Gln Ser His
       50                  55                  60
   Pro Gly Ser Pro Ala Pro Gln Ala Pro Gln Pro Ala Gly Gln Thr Glu
   65                  70                  75                  80
   Gly Asn Ala Ala Ala Ala Pro Pro Gln Gly Gln Asn Pro Glu Thr Pro
                   85                  90                  95
   Thr Pro Thr Ala Ala Val Gln Pro Pro Pro Val Leu Lys Glu Gly Asp
               100                 105                 110
   Asp Cys Pro Asp Ser Thr Leu Ala Val Lys Gly Leu Thr Asn Ala Pro
           115                 120                 125
   Gln Tyr Tyr Val Gly Asp Gln Pro Lys Phe Thr Met Val Val Thr Asn
       130                 135                 140
   Ile Gly Leu Val Ser Cys Lys Arg Asp Val Gly Ala Ala Val Leu Ala
   145                 150                 155                 160
   Ala Tyr Val Tyr Ser Leu Asp Asn Lys Arg Leu Trp Ser Asn Leu Asp
                   165                 170                 175
   Cys Ala Pro Ser Asn Glu Thr Leu Val Lys Thr Phe Ser Pro Gly Glu
               180                 185                 190
   Gln Val Thr Thr Ala Val Thr Trp Thr Gly Met Gly Ser Ala Pro Arg
           195                 200                 205
   Cys Pro Leu Pro Arg Pro Ala Ile Gly Pro Gly Thr Tyr Asn Leu Val
       210                 215                 220
   Val Gln Leu Gly Asn Leu Arg Ser Leu Pro Val Pro Phe Ile Leu Asn
   225                 230                 235                 240
   Gln Pro Pro Pro Pro Pro Gly Pro Val Pro Ala Pro Gly Pro Ala Gln
                   245                 250                 255
   Ala Pro Pro Pro Glu Ser Pro Ala Gln Gly Gly
               260                 265
<210> 73
   <211> 97
   <212> PRT
   <213> Mycobacterium tuberculosis
<400> 73
   Leu Ile Ser Thr Gly Lys Ala Ser His Ala Ser Leu Gly Val Gln Val
   1               5                   10                  15
   Thr Asn Asp Lys Asp Thr Pro Gly Ala Lys Ile Val Glu Val Val Ala
               20                  25                  30
   Gly Gly Ala Ala Ala Asn Ala Gly Val Pro Lys Gly Val Val Val Thr
           35                  40                  45
   Lys Val Asp Asp Arg Pro Ile Asn Ser Ala Asp Ala Leu Val Ala Ala
       50                  55                  60
   Val Arg Ser Lys Ala Pro Gly Ala Thr Val Ala Leu Thr Phe Gln Asp
   65                  70                  75                  80
   Pro Ser Gly Gly Ser Arg Thr Val Gln Val Thr Leu Gly Lys Ala Glu
                   85                  90                  95
   Gln
<210> 74
   <211> 364
   <212> PRT
   <213> Mycobacterium tuberculosis
<400> 74
   Gly Ala Ala Val Ser Leu Leu Ala Ala Gly Thr Leu Val Leu Thr Ala
   1               5                   10                  15
   Cys Gly Gly Gly Thr Asn Ser Ser Ser Ser Gly Ala Gly Gly Thr Ser
               20                  25                  30
   Gly Ser Val His Cys Gly Gly Lys Lys Glu Leu His Ser Ser Gly Ser
           35                  40                  45
   Thr Ala Gln Glu Asn Ala Met Glu Gln Phe Val Tyr Ala Tyr Val Arg
       50                  55                  60
   Ser Cys Pro Gly Tyr Thr Leu Asp Tyr Asn Ala Asn Gly Ser Gly Ala
   65                  70                  75                  80
   Gly Val Thr Gln Phe Leu Asn Asn Glu Thr Asp Phe Ala Gly Ser Asp
                   85                  90                  95
   Val Pro Leu Asn Pro Ser Thr Gly Gln Pro Asp Arg Ser Ala Glu Arg
               100                 105                 110
   Cys Gly Ser Pro Ala Trp Asp Leu Pro Thr Val Phe Gly Pro Ile Ala
           115                 120                 125
   Ile Thr Tyr Asn Ile Lys Gly Val Ser Thr Leu Asn Leu Asp Gly Pro
   130 135 140
   Thr Thr Ala Lys Ile Phe Asn Gly Thr Ile Thr Val Trp Asn Asp Pro
   145 150 155 160
   Gln Ile Gln Ala Leu Asn Ser Gly Thr Asp Leu Pro Pro Thr Pro Ile
   165 170 175
   Ser Val Ile Phe Arg Ser Asp Lys Ser Gly Thr Ser Asp Asn Phe Gln
   180 185 190
   Lys Tyr Leu Asp Gly Val Ser Asn Gly Ala Trp Gly Lys Gly Ala Ser
   195 200 205
   Glu Thr Phe Ser Gly Gly Val Gly Val Gly Ala Ser Gly Asn Asn Gly
   210 215 220
   Thr Ser Ala Leu Leu Gln Thr Thr Asp Gly Ser Ile Thr Tyr Asn Glu
   225 230 235 240
   Trp Ser Phe Ala Val Gly Lys Gln Leu Asn Met Ala Gln Ile Ile Thr
   245 250 255
   Ser Ala Gly Pro Asp Pro Val Ala Ile Thr Thr Glu Ser Val Gly Lys
   260 265 270
   Thr Ile Ala Gly Ala Lys Ile Met Gly Gln Gly Asn Asp Leu Val Leu
   275 280 285
   Asp Thr Ser Ser Phe Tyr Arg Pro Thr Gln Pro Gly Ser Tyr Pro Ile
   290 295 300
   Val Leu Ala Thr Tyr Glu Ile Val Cys Ser Lys Tyr Pro Asp Ala Thr
   305 310 315 320
   Thr Gly Thr Ala Val Arg Ala Phe Met Gln Ala Ala Ile Gly Pro Gly
   325 330 335
   Gln Glu Gly Leu Asp Gln Tyr Gly Ser Ile Pro Leu Pro Lys Ser Phe
   340 345 350
   Gln Ala Lys Leu Ala Ala Ala Val Asn Ala Ile Ser
   355 360
<210> 75
   <211> 309
   <212> PRT
   <213> Mycobacterium tuberculosis
<400> 75
   Gln Ala Ala Ala Gly Arg Ala Val Arg Arg Thr Gly His Ala Glu Asp
   1 5 10 15
   Gln Thr His Gln Asp Arg Leu His His Gly Cys Arg Arg Ala Ala Val
   20 25 30
   Val Val Arg Gln Asp Arg Ala Ser Val Ser Ala Thr Ser Ala Arg Pro
   35 40 45
   Pro Arg Arg His Pro Ala Gln Gly His Arg Arg Arg Val Ala Pro Ser
   50 55 60
   Gly Gly Arg Arg Arg Pro His Pro His His Val Gln Pro Asp Asp Arg
   65 70 75 80
   Arg Asp Arg Pro Ala Leu Leu Asp Arg Thr Gln Pro Ala Glu His Pro
   85 90 95
   Asp Pro His Arg Arg Gly Pro Ala Asp Pro Gly Arg Val Arg Gly Arg
   100 105 110
   Gly Arg Leu Arg Arg Val Asp Asp Gly Arg Leu Gln Pro Asp Arg Asp
   115 120 125
   Ala Asp His Gly Ala Pro Val Arg Gly Arg Gly Pro His Arg Gly Val
   130 135 140
   Gln His Arg Gly Gly Pro Val Phe Val Arg Arg Val Pro Gly Val Arg
   145 150 155 160
   Cys Ala His Arg Arg Gly His Arg Arg Val Ala Ala Pro Gly Gln Gly
   165 170 175
   Asp Val Leu Arg Ala Gly Leu Arg Val Glu Arg Leu Arg Pro Val Ala
   180 185 190
   Ala Val Glu Asn Leu His Arg Gly Ser Gln Arg Ala Asp Gly Arg Val
   195 200 205
   Phe Arg Pro Ile Arg Arg Gly Ala Arg Leu Pro Ala Arg Arg Ser Arg
   210 215 220
   Ala Gly Pro Gln Gly Arg Leu His Leu Asp Gly Ala Gly Pro Ser Pro
   225 230 235 240
   Leu Pro Ala Arg Ala Gly Gln Gln Gln Pro Ser Ser Ala Gly Gly Arg
   245 250 255
   Arg Ala Gly Gly Ala Glu Arg Ala Asp Pro Gly Gln Arg Gly Arg His
   260 265 270
   His Gln Gly Gly His Asp Pro Gly Arg Gln Gly Ala Gln Arg Gly Thr
   275 280 285
   Ala Gly Val Ala His Ala Ala Ala Gly Pro Arg Arg Ala Ala Val Arg
   290 295 300
   Asn Arg Pro Arg Arg
   305
<210> 76
   <211> 580
   <212> PRT
   <213> Mycobacterium tuberculosis
<400> 76
   Ser Ala Val Trp Cys Leu Asn Gly Phe Thr Gly Arg His Arg His Gly
   1               5                   10                  15
   Arg Cys Arg Val Arg Ala Ser Gly Trp Arg Ser Ser Asn Arg Trp Cys
               20                  25                  30
   Ser Thr Thr Ala Asp Cys Cys Ala Ser Lys Thr Pro Thr Gln Ala Ala
           35                  40                  45
   Ser Pro Leu Glu Arg Arg Phe Thr Cys Cys Ser Pro Ala Val Gly Cys
       50                  55                  60
   Arg Phe Arg Ser Phe Pro Val Arg Arg Leu Ala Leu Gly Ala Arg Thr
   65                  70                  75                  80
   Ser Arg Thr Leu Gly Val Arg Arg Thr Leu Ser Gln Trp Asn Leu Ser
                   85                  90                  95
   Pro Arg Ala Gln Pro Ser Cys Ala Val Thr Val Glu Ser His Thr His
               100                 105                 110
   Ala Ser Pro Arg Met Ala Lys Leu Ala Arg Val Val Gly Leu Val Gln
           115                 120                 125
   Glu Glu Gln Pro Ser Asp Met Thr Asn His Pro Arg Tyr Ser Pro Pro
       130                 135                 140
   Pro Gln Gln Pro Gly Thr Pro Gly Tyr Ala Gln Gly Gln Gln Gln Thr
   145                 150                 155                 160
   Tyr Ser Gln Gln Phe Asp Trp Arg Tyr Pro Pro Ser Pro Pro Pro Gln
                   165                 170                 175
   Pro Thr Gln Tyr Arg Gln Pro Tyr Glu Ala Leu Gly Gly Thr Arg Pro
               180                 185                 190
   Gly Leu Ile Pro Gly Val Ile Pro Thr Met Thr Pro Pro Pro Gly Met
           195                 200                 205
   Val Arg Gln Arg Pro Arg Ala Gly Met Leu Ala Ile Gly Ala Val Thr
   210 215 220
   Ile Ala Val Val Ser Ala Gly Ile Gly Gly Ala Ala Ala Ser Leu Val
   225 230 235 240
   Gly Phe Asn Arg Ala Pro Ala Gly Pro Ser Gly Gly Pro Val Ala Ala
   245 250 255
   Ser Ala Ala Pro Ser Ile Pro Ala Ala Asn Met Pro Pro Gly Ser Val
   260 265 270
   Glu Gln Val Ala Ala Lys Val Val Pro Ser Val Val Met Leu Glu Thr
   275 280 285
   Asp Leu Gly Arg Gln Ser Glu Glu Gly Ser Gly Ile Ile Leu Ser Ala
   290 295 300
   Glu Gly Leu Ile Leu Thr Asn Asn His Val Ile Ala Ala Ala Ala Lys
   305 310 315 320
   Pro Pro Leu Gly Ser Pro Pro Pro Lys Thr Thr Val Thr Phe Ser Asp
   325 330 335
   Gly Arg Thr Ala Pro Phe Thr Val Val Gly Ala Asp Pro Thr Ser Asp
   340 345 350
   Ile Ala Val Val Arg Val Gln Gly Val Ser Gly Leu Thr Pro Ile Ser
   355 360 365
   Leu Gly Ser Ser Ser Asp Leu Arg Val Gly Gln Pro Val Leu Ala Ile
   370 375 380
   Gly Ser Pro Leu Gly Leu Glu Gly Thr Val Thr Thr Gly Ile Val Ser
   385 390 395 400
   Ala Leu Asn Arg Pro Val Ser Thr Thr Gly Glu Ala Gly Asn Gln Asn
   405 410 415
   Thr Val Leu Asp Ala Ile Gln Thr Asp Ala Ala Ile Asn Pro Gly Asn
   420 425 430
   Ser Gly Gly Ala Leu Val Asn Met Asn Ala Gln Leu Val Gly Val Asn
   435 440 445
   Ser Ala Ile Ala Thr Leu Gly Ala Asp Ser Ala Asp Ala Gln Ser Gly
   450 455 460
   Ser Ile Gly Leu Gly Phe Ala Ile Pro Val Asp Gln Ala Lys Arg Ile
   465 470 475 480
   Ala Asp Glu Leu Ile Ser Thr Gly Lys Ala Ser His Ala Ser Leu Gly
   485 490 495
   Val Gln Val Thr Asn Asp Lys Asp Thr Pro Gly Ala Lys Ile Val Glu
   500 505 510
   Val Val Ala Gly Gly Ala Ala Ala Asn Ala Gly Val Pro Lys Gly Val
   515 520 525
   Val Val Thr Lys Val Asp Asp Arg Pro Ile Asn Ser Ala Asp Ala Leu
   530 535 540
   Val Ala Ala Val Arg Ser Lys Ala Pro Gly Ala Thr Val Ala Leu Thr
   545 550 555 560
   Phe Gln Asp Pro Ser Gly Gly Ser Arg Thr Val Gln Val Thr Leu Gly
   565 570 575
   Lys Ala Glu Gln
   580
<210> 77
   <211> 233
   <212> PRT
   <213> Mycobacterium tuberculosis
<400> 77
   Met Asn Asp Gly Lys Arg Ala Val Thr Ser Ala Val Leu Val Val Leu
   1               5                   10                  15
   Gly Ala Cys Leu Ala Leu Trp Leu Ser Gly Cys Ser Ser Pro Lys Pro
               20                  25                  30
   Asp Ala Glu Glu Gln Gly Val Pro Val Ser Pro Thr Ala Ser Asp Pro
           35                  40                  45
   Ala Leu Leu Ala Glu Ile Arg Gln Ser Leu Asp Ala Thr Lys Gly Leu
       50                  55                  60
   Thr Ser Val His Val Ala Val Arg Thr Thr Gly Lys Val Asp Ser Leu
   65                  70                  75                  80
   Leu Gly Ile Thr Ser Ala Asp Val Asp Val Arg Ala Asn Pro Leu Ala
                   85                  90                  95
   Ala Lys Gly Val Cys Thr Tyr Asn Asp Glu Gln Gly Val Pro Phe Arg
               100                 105                 110
   Val Gln Gly Asp Asn Ile Ser Val Lys Leu Phe Asp Asp Trp Ser Asn
           115                 120                 125
   Leu Gly Ser Ile Ser Glu Leu Ser Thr Ser Arg Val Leu Asp Pro Ala
       130                 135                 140
   Ala Gly Val Thr Gln Leu Leu Ser Gly Val Thr Asn Leu Gln Ala Gln
   145                 150                 155                 160
   Gly Thr Glu Val Ile Asp Gly Ile Ser Thr Thr Lys Ile Thr Gly Thr
                   165                 170                 175
   Ile Pro Ala Ser Ser Val Lys Met Leu Asp Pro Gly Ala Lys Ser Ala
               180                 185                 190
   Arg Pro Ala Thr Val Trp Ile Ala Gln Asp Gly Ser His His Leu Val
           195                 200                 205
   Arg Ala Ser Ile Asp Leu Gly Ser Gly Ser Ile Gln Leu Thr Gln Ser
       210                 215                 220
   Lys Trp Asn Glu Pro Val Asn Val Asp
   225                 230
<210> 78
   <211> 66
   <212> PRT
   <213> Mycobacterium tuberculosis
<400> 78
   Val Ile Asp Ile Ile Gly Thr Ser Pro Thr Ser Trp Glu Gln Ala Ala
   1 5 10 15
   Ala Glu Ala Val Gln Arg Ala Arg Asp Ser Val Asp Asp Ile Arg Val
   20 25 30
   Ala Arg Val Ile Glu Gln Asp Met Ala Val Asp Ser Ala Gly Lys Ile
   35 40 45
   Thr Tyr Arg Ile Lys Leu Glu Val Ser Phe Lys Met Arg Pro Ala Gln
   50 55 60
   Pro Arg
   65
<210> 79
   <211> 69
   <212> PRT
   <213> Mycobacterium tuberculosis
<400> 79
   Val Pro Pro Ala Pro Pro Leu Pro Pro Leu Pro Pro Ser Pro Ile Ser
   1 5 10 15
   Cys Ala Ser Pro Pro Ser Pro Pro Leu Pro Pro Ala Pro Pro Val Ala
   20 25 30
   Pro Gly Pro Pro Met Pro Pro Leu Asp Pro Trp Pro Pro Ala Pro Pro
   35 40 45
   Leu Pro Tyr Ser Thr Pro Pro Gly Ala Pro Leu Pro Pro Ser Pro Pro
   50 55 60
   Ser Pro Pro Leu Pro
   65
<210> 80
   <211> 355
   <212> PRT
   <213> Mycobacterium tuberculosis
<400> 80
   Met Ser Asn Ser Arg Arg Arg Ser Leu Arg Trp Ser Trp Leu Leu Ser
   1               5                   10                  15
   Val Leu Ala Ala Val Gly Leu Gly Leu Ala Thr Ala Pro Ala Gln Ala
               20                  25                  30
   Ala Pro Pro Ala Leu Ser Gln Asp Arg Phe Ala Asp Phe Pro Ala Leu
           35                  40                  45
   Pro Leu Asp Pro Ser Ala Met Val Ala Gln Val Ala Pro Gln Val Val
       50                  55                  60
   Asn Ile Asn Thr Lys Leu Gly Tyr Asn Asn Ala Val Gly Ala Gly Thr
   65                  70                  75                  80
   Gly Ile Val Ile Asp Pro Asn Gly Val Val Leu Thr Asn Asn His Val
                   85                  90                  95
   Ile Ala Gly Ala Thr Asp Ile Asn Ala Phe Ser Val Gly Ser Gly Gln
               100                 105                 110
   Thr Tyr Gly Val Asp Val Val Gly Tyr Asp Arg Thr Gln Asp Val Ala
           115                 120                 125
   Val Leu Gln Leu Arg Gly Ala Gly Gly Leu Pro Ser Ala Ala Ile Gly
       130                 135                 140
   Gly Gly Val Ala Val Gly Glu Pro Val Val Ala Met Gly Asn Ser Gly
   145                 150                 155                 160
   Gly Gln Gly Gly Thr Pro Arg Ala Val Pro Gly Arg Val Val Ala Leu
                   165                 170                 175
   Gly Gln Thr Val Gln Ala Ser Asp Ser Leu Thr Gly Ala Glu Glu Thr
               180                 185                 190
   Leu Asn Gly Leu Ile Gln Phe Asp Ala Ala Ile Gln Pro Gly Asp Ser
           195                 200                 205
   Gly Gly Pro Val Val Asn Gly Leu Gly Gln Val Val Gly Met Asn Thr
       210                 215                 220
   Ala Ala Ser Asp Asn Phe Gln Leu Ser Gln Gly Gly Gln Gly Phe Ala
   225                 230                 235                 240
   Ile Pro Ile Gly Gln Ala Met Ala Ile Ala Gly Gln Ile Arg Ser Gly
                   245                 250                 255
   Gly Gly Ser Pro Thr Val His Ile Gly Pro Thr Ala Phe Leu Gly Leu
               260                 265                 270
   Gly Val Val Asp Asn Asn Gly Asn Gly Ala Arg Val Gln Arg Val Val
           275                 280                 285
   Gly Ser Ala Pro Ala Ala Ser Leu Gly Ile Ser Thr Gly Asp Val Ile
       290                 295                 300
   Thr Ala Val Asp Gly Ala Pro Ile Asn Ser Ala Thr Ala Met Ala Asp
   305                 310                 315                 320
   Ala Leu Asn Gly His His Pro Gly Asp Val Ile Ser Val Asn Trp Gln
                   325                 330                 335
   Thr Lys Ser Gly Gly Thr Arg Thr Gly Asn Val Thr Leu Ala Glu Gly
               340                 345                 350
   Pro Pro Ala
           355
<210> 81
   <211> 205
   <212> PRT
   <213> Mycobacterium tuberculosis
<400> 81
   Ser Pro Lys Pro Asp Ala Glu Glu Gln Gly Val Pro Val Ser Pro Thr
   1               5                   10                  15
   Ala Ser Asp Pro Ala Leu Leu Ala Glu Ile Arg Gln Ser Leu Asp Ala
               20                  25                  30
   Thr Lys Gly Leu Thr Ser Val His Val Ala Val Arg Thr Thr Gly Lys
           35                  40                  45
   Val Asp Ser Leu Leu Gly Ile Thr Ser Ala Asp Val Asp Val Arg Ala
       50                  55                  60
   Asn Pro Leu Ala Ala Lys Gly Val Cys Thr Tyr Asn Asp Glu Gln Gly
   65                  70                  75                  80
   Val Pro Phe Arg Val Gln Gly Asp Asn Ile Ser Val Lys Leu Phe Asp
                   85                  90                  95
   Asp Trp Ser Asn Leu Gly Ser Ile Ser Glu Leu Ser Thr Ser Arg Val
               100                 105                 110
   Leu Asp Pro Ala Ala Gly Val Thr Gln Leu Leu Ser Gly Val Thr Asn
           115                 120                 125
   Leu Gln Ala Gln Gly Thr Glu Val Ile Asp Gly Ile Ser Thr Thr Lys
       130                 135                 140
   Ile Thr Gly Thr Ile Pro Ala Ser Ser Val Lys Met Leu Asp Pro Gly
   145                 150                 155                 160
   Ala Lys Ser Ala Arg Pro Ala Thr Val Trp Ile Ala Gln Asp Gly Ser
                   165                 170                 175
   His His Leu Val Arg Ala Ser Ile Asp Leu Gly Ser Gly Ser Ile Gln
               180                 185                 190
   Leu Thr Gln Ser Lys Trp Asn Glu Pro Val Asn Val Asp
           195                 200                 205
<210> 82
   <211> 286
   <212> PRT
   <213> Mycobacterium tuberculosis
<220>
   <221> MISC_FEATURE
   <222> (63)..(63)
   <223> Unknown
<220>
   <221> MISC_FEATURE
   <222> (121)..(121)
   <223> Unknown
<220>
   <221> MISC_FEATURE
   <222> (285)..(285)
   <223> Unknown
<400> 82
   Gly Asp Ser Phe Trp Ala Ala Ala Asp Gln Met Ala Arg Gly Phe Val
   1               5                   10                  15
   Leu Gly Ala Thr Ala Gly Arg Thr Thr Leu Thr Gly Glu Gly Leu Gln
               20                  25                  30
   His Ala Asp Gly His Ser Leu Leu Leu Asp Ala Thr Asn Pro Ala Val
           35                  40                  45
   Val Ala Tyr Asp Pro Ala Phe Ala Tyr Glu Ile Gly Tyr Ile Xaa Glu
       50                  55                  60
   Ser Gly Leu Ala Arg Met Cys Gly Glu Asn Pro Glu Asn Ile Phe Phe
   65 70 75 80
   Tyr Ile Thr Val Tyr Asn Glu Pro Tyr Val Gln Pro Pro Glu Pro Glu
   85 90 95
   Asn Phe Asp Pro Glu Gly Val Leu Gly Gly Ile Tyr Arg Tyr His Ala
   100 105 110
   Ala Thr Glu Gln Arg Thr Asn Lys Xaa Gln Ile Leu Ala Ser Gly Val
   115 120 125
   Ala Met Pro Ala Ala Leu Arg Ala Ala Gln Met Leu Ala Ala Glu Trp
   130 135 140
   Asp Val Ala Ala Asp Val Trp Ser Val Thr Ser Trp Gly Glu Leu Asn
   145 150 155 160
   Arg Asp Gly Val Val Ile Glu Thr Glu Lys Leu Arg His Pro Asp Arg
   165 170 175
   Pro Ala Gly Val Pro Tyr Val Thr Arg Ala Leu Glu Asn Ala Arg Gly
   180 185 190
   Pro Val Ile Ala Val Ser Asp Trp Met Arg Ala Val Pro Glu Gln Ile
   195 200 205
   Arg Pro Trp Val Pro Gly Thr Tyr Leu Thr Leu Gly Thr Asp Gly Phe
   210 215 220
   Gly Phe Ser Asp Thr Arg Pro Ala Gly Arg Arg Tyr Phe Asn Thr Asp
   225 230 235 240
   Ala Glu Ser Gln Val Gly Arg Gly Phe Gly Arg Gly Trp Pro Gly Arg
   245 250 255
   Arg Val Asn Ile Asp Pro Phe Gly Ala Gly Arg Gly Pro Pro Ala Gln
   260 265 270
   Leu Pro Gly Phe Asp Glu Gly Gly Gly Leu Arg Pro Xaa Lys
   275 280 285
<210> 83
   <211> 173
   <212> PRT
   <213> Mycobacterium tuberculosis
<400> 83
   Thr Lys Phe His Ala Leu Met Gln Glu Gln Ile His Asn Glu Phe Thr
   1               5                   10                  15
   Ala Ala Gln Gln Tyr Val Ala Ile Ala Val Tyr Phe Asp Ser Glu Asp
               20                  25                  30
   Leu Pro Gln Leu Ala Lys His Phe Tyr Ser Gln Ala Val Glu Glu Arg
           35                  40                  45
   Asn His Ala Met Met Leu Val Gln His Leu Leu Asp Arg Asp Leu Arg
       50                  55                  60
   Val Glu Ile Pro Gly Val Asp Thr Val Arg Asn Gln Phe Asp Arg Pro
   65                  70                  75                  80
   Arg Glu Ala Leu Ala Leu Ala Leu Asp Gln Glu Arg Thr Val Thr Asp
                   85                  90                  95
   Gln Val Gly Arg Leu Thr Ala Val Ala Arg Asp Glu Gly Asp Phe Leu
               100                 105                 110
   Gly Glu Gln Phe Met Gln Trp Phe Leu Gln Glu Gln Ile Glu Glu Val
           115                 120                 125
   Ala Leu Met Ala Thr Leu Val Arg Val Ala Asp Arg Ala Gly Ala Asn
       130                 135                 140
   Leu Phe Glu Leu Glu Asn Phe Val Ala Arg Glu Val Asp Val Ala Pro
   145                 150                 155                 160
   Ala Ala Ser Gly Ala Pro His Ala Ala Gly Gly Arg Leu
                   165                 170

<210> 84
   <211> 107
   <212> PRT
   <213> Mycobacterium tuberculosis
<220>
   <221> MISC_FEATURE
   <222> (60)..(60)
   <223> Unknown
<220>
   <221> MISC_FEATURE
   <222> (64)..(64)
   <223> Unknown
<220> <221> MISC_FEATURE
   <222> (73)..(73)

   <223> unknown
<220>
   <221> MISC_FEATURE
   <222> (77)..(77)
   <223> Unknown
<220>
   <221> MISC_FEATURE
   <222> (79)..(79)
   <223> Unknown
<220>
   <221> MISC_FEATURE
   <222> (86)..(86)
   <223> unknown
<220>
   <221> MISC_FEATURE
   <222> (99)..(99)
   <223> unknown
<220>
   <221> MISC_FEATURE
   <222> (102)..(103)
   <223> Unknown
<220>
   <221> MISC_FEATURE
   <222> (106)..(106)
   <223> unknown
<400> 84
   Arg Ala Asp Glu Arg Lys Asn Thr Thr Met Lys Met Val Lys Ser Ile
   1               5                   10                  15
   Ala Ala Gly Leu Thr Ala Ala Ala Ala Ile Gly Ala Ala Ala Ala Gly
               20                  25                  30
   Val Thr Ser Ile Met Ala Gly Gly Pro Val Val Tyr Gln Met Gln Pro
           35                  40                  45
   Val Val Phe Gly Ala Pro Leu Pro Leu Asp Pro Xaa Ser Ala Pro Xaa
       50                  55                  60
   Val Pro Thr Ala Ala Gln Trp Thr Xaa Leu Leu Asn Xaa Leu Xaa Asp
   65                  70                  75                  80
   Pro Asn Val Ser Phe Xaa Asn Lys Gly Ser Leu Val Glu Gly Gly Ile
                   85                  90                  95
   Gly Gly Xaa Glu Gly Xaa Xaa Arg Arg Xaa Gln
               100                 105
<210> 85
   <211> 125
   <212> PRT
   <213> Mycobacterium tuberculosis
<220>
   <221> MISC_FEATURE
   <222> (12)..(12)
   <223> unknown
<220>
   <221> MISC_FEATURE
   <222> (45)..(45)
   <223> Unknown
<220>
   <221> MISC_FEATURE
   <222> (54)..(54)
   <223> Unknown
<400> 85
   Val Leu Ser Val Pro Val Gly Asp Gly Phe Trp Xaa Arg Val Val Asn
   1               5                   10                  15
   Pro Leu Gly Gln Pro Ile Asp Gly Arg Gly Asp Val Asp Ser Asp Thr
               20                  25                  30
   Arg Arg Ala Leu Glu Leu Gln Ala Pro Ser Val Val Xaa Arg Gln Gly
           35                  40                  45
   Val Lys Glu Pro Leu Xaa Thr Gly Ile Lys Ala Ile Asp Ala Met Thr
       50                  55                  60
   Pro Ile Gly Arg Gly Gln Arg Gln Leu Ile Ile Gly Asp Arg Lys Thr
   65                  70                  75                  80
   Gly Lys Asn Arg Arg Leu Cys Arg Thr Pro Ser Ser Asn Gln Arg Glu
                   85                  90                  95
   Glu Leu Gly Val Arg Trp Ile Pro Arg Ser Arg Cys Ala Cys Val Tyr
               100                 105                 110
   Val Gly His Arg Ala Arg Arg Gly Thr Tyr His Arg Arg
           115                 120                 125
<210> 86
   <211> 117
   <212> PRT
   <213> Mycobacterium tuberculosis
<220>
   <221> MISC_FEATURE
   <222> (99)..(100)
   <223> Unknown
<220>
   <221> MISC_FEATURE
   <222> (104)..(104)
   <223> Unknown
<220>
   <221> MISC_FEATURE
   <222> (108)..(108)
   <223> Unknown
<220>
   <221> MISC_FEATURE
   <222> (112)..(112)
   <223> Unknown
<220>
   <221> MISC_FEATURE
   <222> (116)..(116)
   <223> unknown
<400> 86
   Cys Asp Ala Val Met Gly Phe Leu Gly Gly Ala Gly Pro Leu Ala Val
   1               5                   10                  15
   Val Asp Gln Gln Leu Val Thr Arg Val Pro Gln Gly Trp Ser Phe Ala
               20                  25                  30
   Gln Ala Ala Ala Val Pro Val Val Phe Leu Thr Ala Trp Tyr Gly Leu
           35                  40                  45
   Ala Asp Leu Ala Glu Ile Lys Ala Gly Glu Ser Val Leu Ile His Ala
       50                  55                  60
   Gly Thr Gly Gly Val Gly Met Ala Ala Val Gln Leu Ala Arg Gln Trp
   65                  70                  75                  80
   Gly Val Glu Val Phe Val Thr Ala Ser Arg Gly Lys Trp Asp Thr Leu
                   85                  90                  95
   Arg Ala Xaa Xaa Phe Asp Asp Xaa Pro Tyr Arg Xaa Phe Pro His Xaa
               100                 105                 110
   Arg Ser Ser Xaa Gly
           115
<210> 87
   <211> 103
   <212> PRT
   <213> Mycobacterium tuberculosis
<400> 87
   Met Tyr Arg Phe Ala Cys Arg Thr Leu Met Leu Ala Ala Cys Ile Leu
   1               5                   10                  15
   Ala Thr Gly Val Ala Gly Leu Gly Val Gly Ala Gln Ser Ala Ala Gln
               20                  25                  30
   Thr Ala Pro Val Pro Asp Tyr Tyr Trp Cys Pro Gly Gln Pro Phe Asp
           35                  40                  45
   Pro Ala Trp Gly Pro Asn Trp Asp Pro Tyr Thr Cys His Asp Asp Phe
       50                  55                  60
   His Arg Asp Ser Asp Gly Pro Asp His Ser Arg Asp Tyr Pro Gly Pro
   65                  70                  75                  80
   Ile Leu Glu Gly Pro Val Leu Asp Asp Pro Gly Ala Ala Pro Pro Pro
                   85                  90                  95
   Pro Ala Ala Gly Gly Gly Ala
               100
<210> 88
   <211> 88
   <212> PRT
   <213> Mycobacterium tuberculosis
<400> 88
   Val Gln Cys Arg Val Trp Leu Glu Ile Gln Trp Arg Gly Met Leu Gly
   1               5                   10                  15
   Ala Asp Gln Ala Arg Ala Gly Gly Pro Ala Arg Ile Trp Arg Glu His
               20                  25                  30
   Ser Met Ala Ala Met Lys Pro Arg Thr Gly Asp Gly Pro Leu Glu Ala
           35                  40                  45
   Thr Lys Glu Gly Arg Gly Ile Val Met Arg Val Pro Leu Glu Gly Gly
       50                  55                  60
   Gly Arg Leu Val Val Glu Leu Thr Pro Asp Glu Ala Ala Ala Leu Gly
   65                  70                  75                  80
   Asp Glu Leu Lys Gly Val Thr Ser
                   85
<210> 89
   <211> 95
   <212> PRT
   <213> Mycobacterium tuberculosis
<400> 89
   Thr Asp Ala Ala Thr Leu Ala Gln Glu Ala Gly Asn Phe Glu Arg Ile
   1               5                   10                  15
   Ser Gly Asp Leu Lys Thr Gln Ile Asp Gln Val Glu Ser Thr Ala Gly
               20                  25                  30
   Ser Leu Gln Gly Gln Trp Arg Gly Ala Ala Gly Thr Ala Ala Gln Ala
           35                  40                  45
   Ala Val Val Arg Phe Gln Glu Ala Ala Asn Lys Gln Lys Gln Glu Leu
       50                  55                  60
   Asp Glu Ile Ser Thr Asn Ile Arg Gln Ala Gly Val Gln Tyr Ser Arg
   65                  70                  75                  80
   Ala Asp Glu Glu Gln Gln Gln Ala Leu Ser Ser Gln Met Gly Phe
                   85                  90                  95
<210> 90
   <211> 166
   <212> PRT
   <213> Mycobacterium tuberculosis
<220>
   <221> MISC_FEATURE
   <222> (41)..(42)
   <223> unknown
<220>
   <221> MISC_FEATURE
   <222> (49)..(49)
   <223> Unknown
<220>
   <221> MISC_FEATURE
   <222> (80)..(80)
   <223> Unknown
<220>
   <221> MISC_FEATURE
   <222> (152)..(152)
   <223> unknown
<220>
   <221> MISC_FEATURE
   <222> (159)..(159)
   <223> unknown
<400> 90
   Met Thr Gln Ser Gln Thr Val Thr Val Asp Gln Gln Glu Ile Leu Asn
   1               5                   10                  15
   Arg Ala Asn Glu Val Glu Ala Pro Met Ala Asp Pro Pro Thr Asp Val
               20                  25                  30
   Pro Ile Thr Pro Cys Glu Leu Thr Xaa Xaa Lys Asn Ala Ala Gln Gln
           35                  40                  45
   Xaa Val Leu Ser Ala Asp Asn Met Arg Glu Tyr Leu Ala Ala Gly Ala
       50                  55                  60
   Lys Glu Arg Gln Arg Leu Ala Thr Ser Leu Arg Asn Ala Ala Lys Xaa
   65                  70                  75                  80
   Tyr Gly Glu Val Asp Glu Glu Ala Ala Thr Ala Leu Asp Asn Asp Gly
                   85                  90                  95
   Glu Gly Thr Val Gln Ala Glu Ser Ala Gly Ala Val Gly Gly Asp Ser
               100                 105                 110
   Ser Ala Glu Leu Thr Asp Thr Pro Arg Val Ala Thr Ala Gly Glu Pro
           115                 120                 125
   Asn Phe Met Asp Leu Lys Glu Ala Ala Arg Lys Leu Glu Thr Gly Asp
       130                 135                 140
   Gln Gly Ala Ser Leu Ala His Xaa Gly Asp Gly Trp Asn Thr Xaa Thr
   145                 150                 155                 160
   Leu Thr Leu Gln Gly Asp
                   165
<210> 91
   <211> 5
   <212> PRT
   <213> Mycobacterium tuberculosis
<400> 91
   Arg Ala Glu Arg Met
   1               5
<210> 92
   <211> 263
   <212> PRT
   <213> Mycobacterium tuberculosis
<220>
   <221> MISC_FEATURE
   <222> (254)..(254)
   <223> unknown
<400> 92
   Val Ala Trp Met Ser Val Thr Ala Gly Gln Ala Glu Leu Thr Ala Ala
   1 5 10 15
   Gln Val Arg Val Ala Ala Ala Ala Tyr Glu Thr Ala Tyr Gly Leu Thr
   20 25 30
   Val Pro Pro Pro Val Ile Ala Glu Asn Arg Ala Glu Leu Met Ile Leu
   35 40 45
   Ile Ala Thr Asn Leu Leu Gly Gln Asn Thr Pro Ala Ile Ala Val Asn
   50 55 60
   Glu Ala Glu Tyr Gly Glu Met Trp Ala Gln Asp Ala Ala Ala Met Phe
   65 70 75 80
   Gly Tyr Ala Ala Ala Thr Ala Thr Ala Thr Ala Thr Leu Leu Pro Phe
   85 90 95
   Glu Glu Ala Pro Glu Met Thr Ser Ala Gly Gly Leu Leu Glu Gln Ala
   100 105 110
   Ala Ala Val Glu Glu Ala Ser Asp Thr Ala Ala Ala Asn Gln Leu Met
   115 120 125
   Asn Asn Val Pro Gln Ala Leu Lys Gln Leu Ala Gln Pro Thr Gln Gly
   130 135 140
   Thr Thr Pro Ser Ser Lys Leu Gly Gly Leu Trp Lys Thr Val Ser Pro
   145 150 155 160
   His Arg Ser Pro Ile Ser Asn Met Val Ser Met Ala Asn Asn His Met
   165 170 175
   Ser Met Thr Asn Ser Gly Val Ser Met Thr Asn Thr Leu Ser Ser Met
   180 185 190
   Leu Lys Gly Phe Ala Pro Ala Ala Ala Ala Gln Ala Val Gln Thr Ala
           195                 200                 205
   Ala Gln Asn Gly Val Arg Ala Met Ser Ser Leu Gly Ser Ser Leu Gly
       210                 215                 220
   Ser Ser Gly Leu Gly Gly Gly Val Ala Ala Asn Leu Gly Arg Ala Ala
   225                 230                 235                 240
   Ser Val Arg Tyr Gly His Arg Asp Gly Gly Lys Tyr Ala Xaa Ser Gly
                   245                 250                 255
   Arg Arg Asn Gly Gly Pro Ala
               260
<210> 93
   <211> 303
   <212> PRT
   <213> Mycobacterium tuberculosis
<400> 93
   Met Thr Tyr Ser Pro Gly Asn Pro Gly Tyr Pro Gln Ala Gln Pro Ala
   1               5                   10                  15
   Gly Ser Tyr Gly Gly Val Thr Pro Ser Phe Ala His Ala Asp Glu Gly
               20                  25                  30
   Ala Ser Lys Leu Pro Met Tyr Leu Asn Ile Ala Val Ala Val Leu Gly
           35                  40                  45
   Leu Ala Ala Tyr Phe Ala Ser Phe Gly Pro Met Phe Thr Leu Ser Thr
       50                  55                  60
   Glu Leu Gly Gly Gly Asp Gly Ala Val Ser Gly Asp Thr Gly Leu Pro
   65                  70                  75                  80
   Val Gly Val Ala Leu Leu Ala Ala Leu Leu Ala Gly Val Val Leu Val
                   85                  90                  95
   Pro Lys Ala Lys Ser His Val Thr Val Val Ala Val Leu Gly Val Leu
               100                 105                 110
   Gly Val Phe Leu Met Val Ser Ala Thr Phe Asn Lys Pro Ser Ala Tyr
           115                 120                 125
   Ser Thr Gly Trp Ala Leu Trp Val Val Leu Ala Phe Ile Val Phe Gln
       130                 135                 140
   Ala Val Ala Ala Val Leu Ala Leu Leu Val Glu Thr Gly Ala Ile Thr
   145                 150                 155                 160
   Ala Pro Ala Pro Arg Pro Lys Phe Asp Pro Tyr Gly Gln Tyr Gly Arg
                   165                 170                 175
   Tyr Gly Gln Tyr Gly Gln Tyr Gly Val Gln Pro Gly Gly Tyr Tyr Gly
               180                 185                 190
   Gln Gln Gly Ala Gln Gln Ala Ala Gly Leu Gln Ser Pro Gly Pro Gln
           195                 200                 205
   Gln Ser Pro Gln Pro Pro Gly Tyr Gly Ser Gln Tyr Gly Gly Tyr Ser
       210                 215                 220
   Ser Ser Pro Ser Gln Ser Gly Ser Gly Tyr Thr Ala Gln Pro Pro Ala
   225                 230                 235                 240
   Gln Pro Pro Ala Gln Ser Gly Ser Gln Gln Ser His Gln Gly Pro Ser
                   245                 250                 255
   Thr Pro Pro Thr Gly Phe Pro Ser Phe Ser Pro Pro Pro Pro Val Ser
               260                 265                 270
   Ala Gly Thr Gly Ser Gln Ala Gly Ser Ala Pro Val Asn Tyr Ser Asn
           275                 280                 285
   Pro Ser Gly Gly Glu Gln Ser Ser Ser Pro Gly Gly Ala Pro Val
       290                 295                 300
<210> 94
   <211> 168
   <212> PRT
   <213> Mycobacterium tuberculosis
<400> 94
   Met Lys Met Val Lys Ser Ile Ala Ala Gly Leu Thr Ala Ala Ala Ala
   1               5                   10                  15
   Ile Gly Ala Ala Ala Ala Gly Val Thr Ser Ile Met Ala Gly Gly Pro
               20                  25                  30
   Val Val Tyr Gln Met Gln Pro Val Val Phe Gly Ala Pro Leu Pro Leu
           35                  40                  45
   Asp Pro Ala Ser Ala Pro Asp Val Pro Thr Ala Ala Gln Leu Thr Ser
       50                  55                  60
   Leu Leu Asn Ser Leu Ala Asp Pro Asn Val Ser Phe Ala Asn Lys Gly
   65                  70                  75                  80
   Ser Leu Val Glu Gly Gly Ile Gly Gly Thr Glu Ala Arg Ile Ala Asp
                   85                  90                  95
   His Lys Leu Lys Lys Ala Ala Glu His Gly Asp Leu Pro Leu Ser Phe
               100                 105                 110
   Ser Val Thr Asn Ile Gln Pro Ala Ala Ala Gly Ser Ala Thr Ala Asp
           115                 120                 125
   Val Ser Val Ser Gly Pro Lys Leu Ser Ser Pro Val Thr Gln Asn Val
       130                 135                 140
   Thr Phe Val Asn Gln Gly Gly Trp Met Leu Ser Arg Ala Ser Ala Met
   145                 150                 155                 160
   Glu Leu Leu Gln Ala Ala Gly Asn
                   165
<210> 95
   <211> 332
   <212> PRT
   <213> Mycobacterium tuberculosis
<400> 95
   Met His His His His His His Met His Gln Val Asp Pro Asn Leu Thr
   1               5                   10                  15
   Arg Arg Lys Gly Arg Leu Ala Ala Leu Ala Ile Ala Ala Met Ala Ser
               20                  25                  30
   Ala Ser Leu Val Thr Val Ala Val Pro Ala Thr Ala Asn Ala Asp Pro
           35                  40                  45
   Glu Pro Ala Pro Pro Val Pro Thr Thr Ala Ala Ser Pro Pro Ser Thr
       50                  55                  60
   Ala Ala Ala Pro Pro Ala Pro Ala Thr Pro Val Ala Pro Pro Pro Pro
   65                  70                  75                  80
   Ala Ala Ala Asn Thr Pro Asn Ala Gln Pro Gly Asp Pro Asn Ala Ala
                   85                  90                  95
   Pro Pro Pro Ala Asp Pro Asn Ala Pro Pro Pro Pro Val Ile Ala Pro
               100                 105                 110
   Asn Ala Pro Gln Pro Val Arg Ile Asp Asn Pro Val Gly Gly Phe Ser
           115                 120                 125
   Phe Ala Leu Pro Ala Gly Trp Val Glu Ser Asp Ala Ala His Phe Asp
       130                 135                 140
   Tyr Gly Ser Ala Leu Leu Ser Lys Thr Thr Gly Asp Pro Pro Phe Pro
   145                 150                 155                 160
   Gly Gln Pro Pro Pro Val Ala Asn Asp Thr Arg Ile Val Leu Gly Arg
                   165                 170                 175
   Leu Asp Gln Lys Leu Tyr Ala Ser Ala Glu Ala Thr Asp Ser Lys Ala
   180 185 190
   Ala Ala Arg Leu Gly Ser Asp Met Gly Glu Phe Tyr Met Pro Tyr Pro
   195 200 205
   Gly Thr Arg Ile Asn Gln Glu Thr Val Ser Leu Asp Ala Asn Gly Val
   210 215 220
   Ser Gly Ser Ala Ser Tyr Tyr Glu Val Lys Phe Ser Asp Pro Ser Lys
   225 230 235 240
   Pro Asn Gly Gln Ile Trp Thr Gly Val Ile Gly Ser Pro Ala Ala Asn
   245 250 255
   Ala Pro Asp Ala Gly Pro Pro Gln Arg Trp Phe Val Val Trp Leu Gly
   260 265 270
   Thr Ala Asn Asn Pro Val Asp Lys Gly Ala Ala Lys Ala Leu Ala Glu
   275 280 285
   Ser Ile Arg Pro Leu Val Ala Pro Pro Pro Ala Pro Ala Pro Ala Pro
   290 295 300
   Ala Glu Pro Ala Pro Ala Pro Ala Pro Ala Gly Glu Val Ala Pro Thr
   305 310 315 320
   Pro Thr Thr Pro Thr Pro Gln Arg Thr Leu Pro Ala
   325 330
<210> 96
   <211> 500
   <212> DNA
   <213> Mycobacterium tuberculosis
<400> 96
   cgtggcaatg tcgttgaccg tcggggccgg ggtcgcctcc gcagatcccg tggacgcggt 60
   cattaacacc acctgcaatt acgggcaggt agtagctgcg ctcaacgcga cggatccggg 120
   ggctgccgca cagttcaacg cctcaccggt ggcgcagtcc tatttgcgca atttcctcgc 180
   cgcaccgcca cctcagcgcg ctgccatggc cgcgcaattg caagctgtgc cgggggcggc 240
   acagtacatc ggccttgtcg agtcggttgc cggctcctgc aacaactatt aagcccatgc 300
   gggccccatc ccgcgacccg gcatcgtcgc cggggctagg ccagattgcc ccgctcctca 360
   acgggccgca tcccgcgacc cggcatcgtc gccggggcta ggccagattg ccccgctcct 420
   caacgggccg catctcgtgc cgaattcctg cagcccgggg gatccactag ttctagagcg 480
   gccgccaccg cggtggagct 500
<210> 97
   <211> 96
   <212> PRT
   <213> Mycobacterium tuberculosis
<400> 97
   Val Ala Met Ser Leu Thr Val Gly Ala Gly Val Ala Ser Ala Asp Pro
   1               5                   10                  15
   Val Asp Ala Val Ile Asn Thr Thr Cys Asn Tyr Gly Gln Val Val Ala
               20                  25                  30
   Ala Leu Asn Ala Thr Asp Pro Gly Ala Ala Ala Gln Phe Asn Ala Ser
           35                  40                  45
   Pro Val Ala Gln Ser Tyr Leu Arg Asn Phe Leu Ala Ala Pro Pro Pro
       50                  55                  60
   Gln Arg Ala Ala Met Ala Ala Gln Leu Gln Ala Val Pro Gly Ala Ala
   65                  70                  75                  80
   Gln Tyr Ile Gly Leu Val Glu Ser Val Ala Gly Ser Cys Asn Asn Tyr
                   85                  90                  95
<210> 98
   <211> 154
   <212> DNA
   <213> Mycobacterium tuberculosis
<400> 98
   atgacagagc agcagtggaa tttcgcgggt atcgaggccg cggcaagcgc aatccaggga       60
   aatgtcacgt ccattcattc cctccttgac gaggggaagc agtccctgac caagctcgca      120
   gcggcctggg gcggtagcgg ttcggaagcg tacc                                  154
<210> 99
   <211> 51
   <212> PRT
   <213> Mycobacterium tuberculosis
<400> 99
   Met Thr Glu Gln Gln Trp Asn Phe Ala Gly Ile Glu Ala Ala Ala Ser
   1               5                   10                  15
   Ala Ile Gln Gly Asn Val Thr Ser Ile His Ser Leu Leu Asp Glu Gly
               20                  25                  30
   Lys Gln Ser Leu Thr Lys Leu Ala Ala Ala Trp Gly Gly Ser Gly Ser
           35                  40                  45
   Glu Ala Tyr
       50
<210> 100
   <211> 282
   <212> DNA
   <213> Mycobacterium tuberculosis
<220>
   <221> misc_feature
   <222> (41)..(41)
   <223> Unknown
<220>
   <221> misc_feature
   <222> (154)..(154)
   <223> Unknown
<220>
   <221> misc_feature
   <222> (165)..(165)
   <223> Unknown
<220>
   <221> misc_feature
   <222> (190)..(190)
   <223> unknown
<220>
   <221> misc_feature
   <222> (226)..(226)
   <223> unknown
<220>
   <221> misc feature
   <222> (229)..(229)
   <223> unknown
<220>
   <221> misc_feature
   <222> (255)..(255)
   <223> unknown
<400> 100
   cggtcgcgca cttccaggtg actatgaaag tcggcttccg nctggaggat tcctgaacct 60
   tcaagcgcgg ccgataactg aggtgcatca ttaagcgact tttccagaac atcctgacgc 120
   gctcgaaacg cggcacagcc gacggtggct ccgncgaggc gctgnctcca aaatccctga 180
   gacaattcgn cgggggcgcc tacaaggaag tcggtgctga attcgncgng tatctggtcg 240
   acctgtgtgg tctgnagccg gacgaagcgg tgctcgacgt cg 282
<210> 101
   <211> 1565
   <212> DNA
   <213> Mycobacterium tuberculosis
<400> 101
   gtatgcggcc actgaagtcg ccaatgcggc ggcggccagc taagccagga acagtcggca 60
   cgagaaacca cgagaaatag ggacacgtaa tggtggattt cggggcgtta ccaccggaga 120
   tcaactccgc gaggatgtac gccggcccgg gttcggcctc gctggtggcc gcggctcaga 180
   tgtgggacag cgtggcgagt gacctgtttt cggccgcgtc ggcgtttcag tcggtggtct 240
   ggggtctgac ggtggggtcg tggataggtt cgtcggcggg tctgatggtg gcggcggcct 300
   cgccgtatgt ggcgtggatg agcgtcaccg cggggcaggc cgagctgacc gccgcccagg 360
   tccgggttgc tgcggcggcc tacgagacgg cgtatgggct gacggtgccc ccgccggtga 420
   tcgccgagaa ccgtgctgaa ctgatgattc tgatagcgac caacctcttg gggcaaaaca 480
   ccccggcgat cgcggtcaac gaggccgaat acggcgagat gtgggcccaa gacgccgccg 540
   cgatgtttgg ctacgccgcg gcgacggcga cggcgacggc gacgttgctg ccgttcgagg 600
   aggcgccgga gatgaccagc gcgggtgggc tcctcgagca ggccgccgcg gtcgaggagg 660
   cctccgacac cgccgcggcg aaccagttga tgaacaatgt gccccaggcg ctgcaacagc 720
   tggcccagcc cacgcagggc accacgcctt cttccaagct gggtggcctg tggaagacgg 780
   tctcgccgca tcggtcgccg atcagcaaca tggtgtcaat ggccaacaac cacatgtcaa 840
   tgaccaactc gggtgtgtca atgaccaaca ccttgagctc gatgttgaag ggctttgctc 900
   cggcggcggc cgcccaggcc gtgcaaaccg cggcgcaaaa cggggtccgg gcgatgagct 960
   cgctgggcag ctcgctgggt tcttcgggtc tgggcggtgg ggtggccgcc aacttgggtc 1020
   gggcggcctc ggtcggttcg ttgtcggtgc cgcaggcctg ggccgcggcc aaccaggcag 1080
   tcaccccggc ggcgcgggcg ctgccgctga ccagcctgac cagcgccgcg gaaagagggc 1140
   ccgggcagat gctgggcggg ctgccggtgg ggcagatggg cgccagggcc ggtggtgggc 1200
   tcagtggtgt gctgcgtgtt ccgccgcgac cctatgtgat gccgcattct ccggcggccg 1260
   gctaggagag ggggcgcaga ctgtcgttat ttgaccagtg atcggcggtc tcggtgtttc 1320
   cgcggccggc tatgacaaca gtcaatgtgc atgacaagtt acaggtatta ggtccaggtt 1380
   caacaaggag acaggcaaca tggcctcacg ttttatgacg gatccgcacg cgatgcggga 1440
   catggcgggc cgttttgaag tgcacgccca gacggtggag gacgaggctc gccggatgtg 1500
   ggcgtccgcg caaaacattt ccggtgcggg ctggagtggc atggccgagg cgacctcgct 1560
   agaca 1565
<210> 102
   <211> 391
   <212> PRT
   <213> Mycobacterium tuberculosis
<400> 102
   Met Val Asp Phe Gly Ala Leu Pro Pro Glu Ile Asn Ser Ala Arg Met
   1               5                   10                  15
   Tyr Ala Gly Pro Gly Ser Ala Ser Leu Val Ala Ala Ala Gln Met Trp
               20                  25                  30
   Asp Ser Val Ala Ser Asp Leu Phe Ser Ala Ala Ser Ala Phe Gln Ser
           35                  40                  45
   Val Val Trp Gly Leu Thr Val Gly Ser Trp Ile Gly Ser Ser Ala Gly
       50                  55                  60
   Leu Met Val Ala Ala Ala Ser Pro Tyr Val Ala Trp Met Ser Val Thr
   65                  70                  75                  80
   Ala Gly Gln Ala Glu Leu Thr Ala Ala Gln Val Arg Val Ala Ala Ala
                   85                  90                  95
   Ala Tyr Glu Thr Ala Tyr Gly Leu Thr Val Pro Pro Pro Val Ile Ala
   100 105 110
   Glu Asn Arg Ala Glu Leu Met Ile Leu Ile Ala Thr Asn Leu Leu Gly
   115 120 125
   Gln Asn Thr Pro Ala Ile Ala Val Asn Glu Ala Glu Tyr Gly Glu Met
   130 135 140
   Trp Ala Gln Asp Ala Ala Ala Met Phe Gly Tyr Ala Ala Ala Thr Ala
   145 150 155 160
   Thr Ala Thr Ala Thr Leu Leu Pro Phe Glu Glu Ala Pro Glu Met Thr
   165 170 175
   Ser Ala Gly Gly Leu Leu Glu Gln Ala Ala Ala Val Glu Glu Ala Ser
   180 185 190
   Asp Thr Ala Ala Ala Asn Gln Leu Met Asn Asn Val Pro Gln Ala Leu
   195 200 205
   Gln Gln Leu Ala Gln Pro Thr Gln Gly Thr Thr Pro Ser Ser Lys Leu
   210 215 220
   Gly Gly Leu Trp Lys Thr Val Ser Pro His Arg Ser Pro Ile Ser Asn
   225 230 235 240
   Met Val Ser Met Ala Asn Asn His Met Ser Met Thr Asn Ser Gly Val
   245 250 255
   Ser Met Thr Asn Thr Leu Ser Ser Met Leu Lys Gly Phe Ala Pro Ala
   260 265 270
   Ala Ala Ala Gln Ala Val Gln Thr Ala Ala Gln Asn Gly Val Arg Ala
   275 280 285
   Met Ser Ser Leu Gly Ser Ser Leu Gly Ser Ser Gly Leu Gly Gly Gly
   290 295 300
   Val Ala Ala Asn Leu Gly Arg Ala Ala Ser Val Gly Ser Leu Ser Val
   305 310 315 320
   Pro Gln Ala Trp Ala Ala Ala Asn Gln Ala Val Thr Pro Ala Ala Arg
   325 330 335
   Ala Leu Pro Leu Thr Ser Leu Thr Ser Ala Ala Glu Arg Gly Pro Gly
   340 345 350
   Gln Met Leu Gly Gly Leu Pro Val Gly Gln Met Gly Ala Arg Ala Gly
   355 360 365
   Gly Gly Leu Ser Gly Val Leu Arg Val Pro Pro Arg Pro Tyr Val Met
   370 375 380
   Pro His Ser Pro Ala Ala Gly
   385 390
<210> 103
   <211> 259
   <212> DNA
   <213> Mycobacterium tuberculosis
<220>
   <221> misc_feature
   <222> (18)..(18)
   <223> Unknown
<400> 103
   accaacacct tgcactcnat gttgaagggc ttagctccgg cggcggctca ggccgtggaa 60
   accgcggcgg aaaacggggt ctgggcaatg agctcgctgg gcagccagct gggttcgtcg 120
   ctgggttctt cgggtctggg cgctggggtg gccgccaact tgggtcgggc ggcctcggtc 180
   ggttcgttgt cggtgccgcc agcatgggcc gcggccaacc aggcggtcac cccggcggcg 240
   cgggcgctgc cgctgacca 259
<210> 104
   <211> 86
   <212> PRT
   <213> Mycobacterium tuberculosis
<400> 104
   Thr Asn Thr Leu His Ser Met Leu Lys Gly Leu Ala Pro Ala Ala Ala
   1 5 10 15
   Gln Ala Val Glu Thr Ala Ala Glu Asn Gly Val Trp Ala Met Ser Ser
   20 25 30
   Leu Gly Ser Gln Leu Gly Ser Ser Leu Gly Ser Ser Gly Leu Gly Ala
   35 40 45
   Gly Val Ala Ala Asn Leu Gly Arg Ala Ala Ser Val Gly Ser Leu Ser
   50 55 60
   Val Pro Pro Ala Trp Ala Ala Ala Asn Gln Ala Val Thr Pro Ala Ala
   65 70 75 80
   Arg Ala Leu Pro Leu Thr
   85
<210> 105
   <211> 1109
   <212> DNA
   <213> Mycobacterium tuberculosis
<400> 105
   tacttgagag aatttgacct gttgccgacg ttgtttgctg tccatcattg gtgctagtta 60
   tggccgagcg gaaggattat cgaagtggtg gacttcgggg cgttaccacc ggagatcaac 120
   tccgcgagga tgtacgccgg cccgggttcg gcctcgctgg tggccgccgc gaagatgtgg 180
   gacagcgtgg cgagtgacct gttttcggcc gcgtcggcgt ttcagtcggt ggtctggggt 240
   ctgacgacgg gatcgtggat aggttcgtcg gcgggtctga tggtggcggc ggcctcgccg 300
   tatgtggcgt ggatgagcgt caccgcgggg caggccgagc tgaccgccgc ccaggtccgg 360
   gttgctgcgg cggcctacga gacggcgtat gggctgacgg tgcccccgcc ggtgatcgcc 420
   gagaaccgtg ctgaactgat gattctgata gcgaccaacc tcttggggca aaacaccccg 480
   gcgatcgcgg tcaacgaggc cgaatacggg gagatgtggg cccaagacgc cgccgcgatg 540
   tttggctacg ccgccacggc ggcgacggcg accgaggcgt tgctgccgtt cgaggacgcc 600
   ccactgatca ccaaccccgg cgggctcctt gagcaggccg tcgcggtcga ggaggccatc 660
   gacaccgccg cggcgaacca gttgatgaac aatgtgcccc aagcgctgca acaactggcc 720
   cagcccacga aaagcatctg gccgttcgac caactgagtg aactctggaa agccatctcg 780
   ccgcatctgt cgccgctcag caacatcgtg tcgatgctca acaaccacgt gtcgatgacc 840
   aactcgggtg tgtcaatggc cagcaccttg cactcaatgt tgaagggctt tgctccggcg 900
   gcggctcagg ccgtggaaac cgcggcgcaa aacggggtcc aggcgatgag ctcgctgggc 960
   agccagctgg gttcgtcgct gggttcttcg ggtctgggcg ctggggtggc cgccaacttg 1020
   ggtcgggcgg cctcggtcgg ttcgttgtcg gtgccgcagg cctgggccgc ggccaaccag 1080
   gcggtcaccc cggcggcgcg ggcgctgcc 1109
<210> 106
   <211> 341
   <212> PRT
   <213> Mycobacterium tuberculosis
<400> 106
   Val Val Asp Phe Gly Ala Leu Pro Pro Glu Ile Asn Ser Ala Arg Met
   1               5                   10                  15
   Tyr Ala Gly Pro Gly Ser Ala Ser Leu Val Ala Ala Ala Lys Met Trp
               20                  25                  30
   Asp Ser Val Ala Ser Asp Leu Phe Ser Ala Ala Ser Ala Phe Gln Ser
           35                  40                  45
   Val Val Trp Gly Leu Thr Thr Gly Ser Trp Ile Gly Ser Ser Ala Gly
       50                  55                  60
   Leu Met Val Ala Ala Ala Ser Pro Tyr Val Ala Trp Met Ser Val Thr
   65                  70                  75                  80
   Ala Gly Gln Ala Glu Leu Thr Ala Ala Gln Val Arg Val Ala Ala Ala
                   85                  90                  95
   Ala Tyr Glu Thr Ala Tyr Gly Leu Thr Val Pro Pro Pro Val Ile Ala
               100                 105                 110
   Glu Asn Arg Ala Glu Leu Met Ile Leu Ile Ala Thr Asn Leu Leu Gly
           115                 120                 125
   Gln Asn Thr Pro Ala Ile Ala Val Asn Glu Ala Glu Tyr Gly Glu Met
       130                 135                 140
   Trp Ala Gln Asp Ala Ala Ala Met Phe Gly Tyr Ala Ala Thr Ala Ala
   145                 150                 155                 160
   Thr Ala Thr Glu Ala Leu Leu Pro Phe Glu Asp Ala Pro Leu Ile Thr
                   165                 170                 175
   Asn Pro Gly Gly Leu Leu Glu Gln Ala Val Ala Val Glu Glu Ala Ile
               180                 185                 190
   Asp Thr Ala Ala Ala Asn Gln Leu Met Asn Asn Val Pro Gln Ala Leu
           195                 200                 205
   Gln Gln Leu Ala Gln Pro Thr Lys Ser Ile Trp Pro Phe Asp Gln Leu
       210                 215                 220
   Ser Glu Leu Trp Lys Ala Ile Ser Pro His Leu Ser Pro Leu Ser Asn
   225                 230                 235                 240
   Ile Val Ser Met Leu Asn Asn His Val Ser Met Thr Asn Ser Gly Val
                   245                 250                 255
   Ser Met Ala Ser Thr Leu His Ser Met Leu Lys Gly Phe Ala Pro Ala
               260                 265                 270
   Ala Ala Gln Ala Val Glu Thr Ala Ala Gln Asn Gly Val Gln Ala Met
           275                 280                 285
   Ser Ser Leu Gly Ser Gln Leu Gly Ser Ser Leu Gly Ser Ser Gly Leu
       290                 295                 300
   Gly Ala Gly Val Ala Ala Asn Leu Gly Arg Ala Ala Ser Val Gly Ser
   305                 310                 315                 320
   Leu Ser Val Pro Gln Ala Trp Ala Ala Ala Asn Gln Ala Val Thr Pro
                   325                 330                 335
   Ala Ala Arg Ala Leu
               340
<210> 107
   <211> 1256
   <212> DNA
   <213> Mycobacterium tuberculosis
<220>
   <221> misc_feature
   <222> (48)..(48)
   <223> Unknown
<400> 107
   catcggaggg agtgatcacc atgctgtggc acgcaatgcc accggagnta aataccgcac      60
   ggctgatggc cggcgcgggt ccggctccaa tgcttgcggc ggccgcggga tggcagacgc     120
   tttcggcggc tctggacgct caggccgtcg agttgaccgc gcgcctgaac tctctgggag     180
   aagcctggac tggaggtggc agcgacaagg cgcttgcggc tgcaacgccg atggtggtct     240
   ggctacaaac cgcgtcaaca caggccaaga cccgtgcgat gcaggcgacg gcgcaagccg     300
   cggcatacac ccaggccatg gccacgacgc cgtcgctgcc ggagatcgcc gccaaccaca     360
   tcacccaggc cgtccttacg gccaccaact tcttcggtat caacacgatc ccgatcgcgt     420
   tgaccgagat ggattatttc atccgtatgt ggaaccaggc agccctggca atggaggtct     480
   accaggccga gaccgcggtt aacacgcttt tcgagaagct cgagccgatg gcgtcgatcc     540
   ttgatcccgg cgcgagccag agcacgacga acccgatctt cggaatgccc tcccctggca     600
   gctcaacacc ggttggccag ttgccgccgg cggctaccca gaccctcggc caactgggtg     660
   agatgagcgg cccgatgcag cagctgaccc agccgctgca gcaggtgacg tcgttgttca     720
   gccaggtggg cggcaccggc ggcggcaacc cagccgacga ggaagccgcg cagatgggcc     780
   tgctcggcac cagtccgctg tcgaaccatc cgctggctgg tggatcaggc cccagcgcgg     840
   gcgcgggcct gctgcgcgcg gagtcgctac ctggcgcagg tgggtcgttg acccgcacgc     900
   cgctgatgtc tcagctgatc gaaaagccgg ttgccccctc ggtgatgccg gcggctgctg     960
   ccggatcgtc ggcgacgggt ggcgccgctc cggtgggtgc gggagcgatg ggccagggtg    1020
   cgcaatccgg cggctccacc aggccgggtc tggtcgcgcc ggcaccgctc gcgcaggagc    1080
   gtgaagaaga cgacgaggac gactgggacg aagaggacga ctggtgagct cccgtaatga    1140
   caacagactt cccggccacc cgggccggaa gacttgccaa cattttggcg aggaaggtaa    1200
   agagagaaag tagtccagca tggcagagat gaagaccgat gccgctaccc tcgcgc        1256
<210> 108
   <211> 432
   <212> DNA
   <213> Mycobacterium tuberculosis
<400> 108
   ctagtggatg ggaccatggc cattttctgc agtctcactg ccttctgtgt tgacattttg 60
   gcacgccggc ggaaacgaag cactggggtc gaagaacggc tgcgctgcca tatcgtccgg 120
   agcttccata ccttcgtgcg gccggaagag cttgtcgtag tcggccgcca tgacaacctc 180
   tcagagtgcg ctcaaacgta taaacacgag aaagggcgag accgacggaa ggtcgaactc 240
   gcccgatccc gtgtttcgct attctacgcg aactcggcgt tgccctatgc gaacatccca 300
   gtgacgttgc cttcggtcga agccattgcc tgaccggctt cgctgatcgt ccgcgccagg 360
   ttctgcagcg cgttgttcag ctcggtagcc gtggcgtccc atttttgctg gacaccctgg 420
   tacgcctccg aa 432
<210> 109
   <211> 368
   <212> PRT
   <213> Mycobacterium tuberculosis
<220>
   <221> MISC_FEATURE
   <222> (10)..(10)
   <223> Unknown
<400> 109
   Met Leu Trp His Ala Met Pro Pro Glu Xaa Asn Thr Ala Arg Leu Met
   1               5                   10                  15
   Ala Gly Ala Gly Pro Ala Pro Met Leu Ala Ala Ala Ala Gly Trp Gln
               20                  25                  30
   Thr Leu Ser Ala Ala Leu Asp Ala Gln Ala Val Glu Leu Thr Ala Arg
           35                  40                  45
   Leu Asn Ser Leu Gly Glu Ala Trp Thr Gly Gly Gly Ser Asp Lys Ala
       50                  55                  60
   Leu Ala Ala Ala Thr Pro Met Val Val Trp Leu Gln Thr Ala Ser Thr
   65                  70                  75                  80
   Gln Ala Lys Thr Arg Ala Met Gln Ala Thr Ala Gln Ala Ala Ala Tyr
                   85                  90                  95
   Thr Gln Ala Met Ala Thr Thr Pro Ser Leu Pro Glu Ile Ala Ala Asn
               100                 105                 110
   His Ile Thr Gln Ala Val Leu Thr Ala Thr Asn Phe Phe Gly Ile Asn
           115                 120                 125
   Thr Ile Pro Ile Ala Leu Thr Glu Met Asp Tyr Phe Ile Arg Met Trp
       130                 135                 140
   Asn Gln Ala Ala Leu Ala Met Glu Val Tyr Gln Ala Glu Thr Ala Val
   145                 150                 155                 160
   Asn Thr Leu Phe Glu Lys Leu Glu Pro Met Ala Ser Ile Leu Asp Pro
                   165                 170                 175
   Gly Ala Ser Gln Ser Thr Thr Asn Pro Ile Phe Gly Met Pro Ser Pro
               180                 185                 190
   Gly Ser Ser Thr Pro Val Gly Gln Leu Pro Pro Ala Ala Thr Gln Thr
           195                 200                 205
   Leu Gly Gln Leu Gly Glu Met Ser Gly Pro Met Gln Gln Leu Thr Gln
       210                 215                 220
   Pro Leu Gln Gln Val Thr Ser Leu Phe Ser Gln Val Gly Gly Thr Gly
   225                 230                 235                 240
   Gly Gly Asn Pro Ala Asp Glu Glu Ala Ala Gln Met Gly Leu Leu Gly
                   245                 250                 255
   Thr Ser Pro Leu Ser Asn His Pro Leu Ala Gly Gly Ser Gly Pro Ser
               260                 265                 270
   Ala Gly Ala Gly Leu Leu Arg Ala Glu Ser Leu Pro Gly Ala Gly Gly
           275                 280                 285
   Ser Leu Thr Arg Thr Pro Leu Met Ser Gln Leu Ile Glu Lys Pro Val
       290                 295                 300
   Ala Pro Ser Val Met Pro Ala Ala Ala Ala Gly Ser Ser Ala Thr Gly
   305                 310                 315                 320
   Gly Ala Ala Pro Val Gly Ala Gly Ala Met Gly Gln Gly Ala Gln Ser
                   325                 330                 335
   Gly Gly Ser Thr Arg Pro Gly Leu Val Ala Pro Ala Pro Leu Ala Gln
               340                 345                 350
   Glu Arg Glu Glu Asp Asp Glu Asp Asp Trp Asp Glu Glu Asp Asp Trp
           355                 360                 365

<210> 110
   <211> 12
   <212> PRT
   <213> Mycobacterium tuberculosis
<400> 110
   Met Ala Glu Met Lys Thr Asp Ala Ala Thr Leu Ala
   1               5                   10
<210> 111
   <211> 396
   <212> DNA
   <213> Mycobacterium tuberculosis
<400> 111
   gatctccggc gacctgaaaa cccagatcga ccaggtggag tcgacggcag gttcgttgca 60
   gggccagtgg cgcggcgcgg cggggacggc cgcccaggcc gcggtggtgc gcttccaaga 120
   agcagccaat aagcagaagc aggaactcga cgagatctcg acgaatattc gtcaggccgg 180
   cgtccaatac tcgagggccg acgaggagca gcagcaggcg ctgtcctcgc aaatgggctt 240
   ctgacccgct aatacgaaaa gaaacggagc aaaaacatga cagagcagca gtggaatttc 300
   gcgggtatcg aggccgcggc aagcgcaatc cagggaaatg tcacgtccat tcattccctc 360
   cttgacgagg ggaagcagtc cctgaccaag ctcgca 396
<210> 112
   <211> 80
   <212> PRT
   <213> Mycobacterium tuberculosis
<400> 112
   Ile Ser Gly Asp Leu Lys Thr Gln Ile Asp Gln Val Glu Ser Thr Ala
   1 5 10 15
   Gly Ser Leu Gln Gly Gln Trp Arg Gly Ala Ala Gly Thr Ala Ala Gln
   20 25 30
   Ala Ala Val Val Arg Phe Gln Glu Ala Ala Asn Lys Gln Lys Gln Glu
   35 40 45
   Leu Asp Glu Ile Ser Thr Asn Ile Arg Gln Ala Gly Val Gln Tyr Ser
   50 55 60
   Arg Ala Asp Glu Glu Gln Gln Gln Ala Leu Ser Ser Gln Met Gly Phe
   65 70 75 80
<210> 113
   <211> 387
   <212> DNA
   <213> Mycobacterium tuberculosis
<400> 113
   gtggatcccg atcccgtgtt tcgctattct acgcgaactc ggcgttgccc tatgcgaaca      60
   tcccagtgac gttgccttcg gtcgaagcca ttgcctgacc ggcttcgctg atcgtccgcg     120
   ccaggttctg cagcgcgttg ttcagctcgg tagccgtggc gtcccatttt tgctggacac     180
   cctggtacgc ctccgaaccg ctaccgcccc aggccgctgc gagcttggtc agggactgct     240
   tcccctcgtc aaggagggaa tgaatggacg tgacatttcc ctggattgcg cttgccgcgg     300
   cctcgatacc cgcgaaattc cactgctgct ctgtcatgtt tttgctccgt ttcttttcgt     360
   attagcgggt cagaagccca tttgcga                                         387
<210> 114
   <211> 272
   <212> DNA
   <213> Mycobacterium tuberculosis
<400> 114
   cggcacgagg atctcggttg gcccaacggc gctggcgagg gctccgttcc gggggcgagc      60
   tgcgcgccgg atgcttcctc tgcccgcagc cgcgcctgga tggatggacc agttgctacc     120
   ttcccgacgt ttcgttcggt gtctgtgcga tagcggtgac cccggcgcgc acgtcgggag     180
   tgttgggggg caggccgggt cggtggttcg gccggggacg cagacggtct ggacggaacg     240
   ggcgggggtt cgccgattgg catctttgcc ca                                   272
<210> 115
   <211> 20
   <212> PRT
   <213> Mycobacterium tuberculosis
<400> 115
   Asp Pro Val Asp Ala Val Ile Asn Thr Thr Cys Asn Tyr Gly Gln Val
   1               5                   10                  15
   Val Ala Ala Leu
               20
<210> 116
   <211> 15
   <212> PRT
   <213> Mycobacterium tuberculosis
<400> 116
   Ala Val Glu Ser Gly Met Leu Ala Leu Gly Thr Pro Ala Pro Ser
   1               5                   10                  15
<210> 117
   <211> 19
   <212> PRT
   <213> Mycobacterium tuberculosis
<400> 117
   Ala Ala Met Lys Pro Arg Thr Gly Asp Gly Pro Leu Glu Ala Ala Lys
   1               5                   10                  15
   Glu Gly Arg
<210> 118
   <211> 15
   <212> PRT
   <213> Mycobacterium tuberculosis
<400> 118
   Tyr Tyr Trp Cys Pro Gly Gln Pro Phe Asp Pro Ala Trp Gly Pro
   1               5                   10                  15
<210> 119
   <211> 14
   <212> PRT
   <213> Mycobacterium tuberculosis
<220>
   <221> MISC_FEATURE
   <222> (12)..(12)
   <223> Unknown
<400> 119
   Asp Ile Gly Ser Glu Ser Thr Glu Asp Gln Gln Xaa Ala Val
   1               5                   10
<210> 120
   <211> 13
   <212> PRT
   <213> Mycobacterium tuberculosis
<220>
   <221> MISC_FEATURE
   <222> (8)..(8)
   <223> unknown
<220>
   <221> MISC_FEATURE
   <222> (10)..(10)
   <223> Unknown
<400> 120
   Ala Glu Glu Ser Ile Ser Thr Xaa Glu Xaa Ile Val Pro
   1 5 10
<210> 121
   <211> 17
   <212> PRT
   <213> Mycobacterium tuberculosis
<400> 121
   Asp Pro Glu Pro Ala Pro Pro Val Pro Thr Thr Ala Ala Ser Pro Pro
   1 5 10 15
   Ser
<210> 122
   <211> 15
   <212> PRT
   <213> Mycobacterium tuberculosis
<220>
   <221> MISC_FEATURE
   <222> (6)..(6)
   <223> Unknown
<400> 122
   Ala Pro Lys Thr Tyr Xaa Glu Glu Leu Lys Gly Thr Asp Thr Gly
   1 5 10 15
<210> 123
   <211> 30
   <212> PRT
   <213> Mycobacterium tuberculosis
<400> 123
   Asp Pro Ala Ser Ala Pro Asp Val Pro Thr Ala Ala Gln Leu Thr Ser
   1 5 10 15
   Leu Leu Asn Ser Leu Ala Asp Pro Asn Val Ser Phe Ala Asn
   20 25 30
<210> 124
   <211> 22
   <212> PRT
   <213> Mycobacterium tuberculosis
<220>
   <221> MISC_FEATURE
   <222> (8)..(8)
   <223> Unknown
<220>
   <221> MISC_FEATURE
   <222> (21)..(21)
   <223> unknown
<400> 124
   Asp Pro Pro Asp Pro His Gln Xaa Asp Met Thr Lys Gly Tyr Tyr Pro
   1               5                   10                  15
   Gly Gly Arg Arg Xaa Phe
               20
<210> 125
   <211> 7
   <212> PRT
   <213> Mycobacterium tuberculosis
<400> 125
   Asp Pro Gly Tyr Thr Pro Gly
   1               5
<210> 126
   <211> 10
   <212> PRT
   <213> Mycobacterium tuberculosis
<220>
   <221> MISC_FEATURE
   <222> (1)..(1)
   <223> Unknown
<220>
   <221> MISC_FEATURE
   <222> (2)..(2)
   <223> Pro or Thr
<400> 126
   Xaa Xaa Gly Phe Thr Gly Pro Gln Phe Tyr
   1               5                   10
<210> 127
   <211> 9
   <212> PRT
   <213> Mycobacterium tuberculosis
<220>
   <221> MISC_FEATURE
   <222> (1)..(1)
   <223> Unknown
<220>
   <221> MISC_FEATURE
   <222> (3)..(3)
   <223> Gln or Leu
<400> 127
   Xaa Pro Xaa Val Thr Ala Tyr Ala Gly
   1               5
<210> 128
   <211> 9
   <212> PRT
   <213> Mycobacterium tuberculosis
<220>
   <221> MISC_FEATURE
   <222> (1)..(3)
   <223> Unknown
<400> 128
   Xaa Xaa Xaa Glu Lys Pro Phe Leu Arg
   1               5
<210> 129
   <211> 15
   <212> PRT
   <213> Mycobacterium tuberculosis
<220>
   <221> MISC_FEATURE
   <222> (1)..(1)
   <223> Unknown
<400> 129
   Xaa Asp Ser Glu Lys Ser Ala Thr Ile Lys Val Thr Asp Ala Ser
   1               5                   10                  15
<210> 130
   <211> 15
   <212> PRT
   <213> Mycobacterium tuberculosis
<220>
   <221> MISC_FEATURE
   <222> (5)..(5)
   <223> unknown
<400> 130
   Ala Gly Asp Thr Xaa Ile Tyr Ile Val Gly Asn Leu Thr Ala Asp
   1               5                   10                  15
<210> 131
   <211> 15
   <212> PRT
   <213> Mycobacterium tuberculosis
<400> 131
   Ala Pro Glu Ser Gly Ala Gly Leu Gly Gly Thr Val Gln Ala Gly
   1               5                   10                  15
<210> 132
   <211> 21
   <212> PRT
   <213> Mycobacterium tuberculosis
<220>
   <221> MISC_FEATURE
   <222> (1)..(1)
   <223> Unknown
<220>
   <221> MISC_FEATURE
   <222> (6)..(6)
   <223> Unknown
<400> 132
   Xaa Tyr Ile Ala Tyr Xaa Thr Thr Ala Gly Ile Val Pro Gly Lys Ile
   1               5                   10                  15
   Asn Val His Leu Val
               20